(19) Europäisches Patentamt
European Patent Office
Office européen des brevets

(11) Veröffentlichungsnummer: **0 054 278 B1**

(12) **EUROPÄISCHE PATENTSCHRIFT**

(45) Veröffentlichungstag der Patentschrift: **01.04.87**

(51) Int. Cl.⁴: **A 01 N 25/32,** C 07 D 263/04

(21) Anmeldenummer: **81110371.2**

(22) Anmeldetag: **11.12.81**

(54) **Pflanzenschutzmittel zur Verwendung bei der Unkrautbekämpfung sowie 2-(Dichlormethyl)-3-phenyl-1,3-oxazolidin und 2-(Dichlormethyl)-3-allyl-1,3-oxazolidin und Verfahren zur Herstellung der in den ersteren als Antidota enthaltenen Verbindungen beziehungsweise der letztgenannten Verbindungen.**

(30) Priorität: **11.12.80 HU 296780**

(43) Veröffentlichungstag der Anmeldung: **23.06.82 Patentblatt 82/25**

(45) Bekanntmachung des Hinweises auf die Patenterteilung: **01.04.87 Patentblatt 87/14**

(84) Benannte Vertragsstaaten: **AT BE CH DE FR GB IT LI LU NL SE**

(56) Entgegenhaltungen:
**DE - A - 2 134 499**
**DE - A - 2 341 469**
**FR - A - 2 448 857**
**US - A - 3 976 469**
**US - A - 3 989 503**
**US - A - 4 021 228**
**US - A - 4 197 110**

**HOUBEN-WEYL; METHODEN DER ORGANISCHEN CHEMIE, 4. Auflage, Band VI/3, 1965, Georg-Thieme Verlag STUTTGART (DE), Herstellung von Acetalen. Kodensation von Aldehyden oder Ketonen mit Alkoholen, Seiten 204-220, 250-252**

**Die Akte enthält technische Angaben, die nach dem Eingang der Anmeldung eingereicht wurden und die nicht in dieser Patentschrift enthalten sind.**

(73) Patentinhaber: **MAGYAR TUDOMANYOS AKADEMIA KÖZP KEMIAI KUTATO INTEZET, Pusztaszeri ut 59-67 Postfach 17, H-1025 Budapest II (HU)**
Patentinhaber: **NITROKEMIA IPARTELEPEK, Pf. 45, H-8184 Füzfögyártelep (HU)**

(72) Erfinder: **Dutka, Ferenc, Dipl.-Chem., Határor u. 22, H-1122 Budapest (HU)**
Erfinder: **Komives, Tamás, Dipl.-Chem., Attila u. 1, H-1028 Budapest (HU)**
Erfinder: **Fodor, Katalin, geb. Csorba, Dipl.-Chem., Népköztarsaság u. 3, H-1061 Budapest (HU)**
Erfinder: **Márton, Attila, Dipl.-Chem., Igaz u. 2, H-1181 Budapest (HU)**
Erfinder: **Csikós, Anikó, geb. Glück, Reáltanoda u. 16, H-1053 Budapest (HU)**
Erfinder: **Osztheimer, Eva, Csermák A. u. 84, H-1038 Budapest (HU)**
Erfinder: **Henger, Károly, Dipl.-Chem., Gagarin u. 28, H-8175 Balatonfüzfo (HU)**
Erfinder: **Laborczy, Róbert, Dipl.-Chem., Gagarin u. 12, H-8175 Balatonfüzfo (HU)**
Erfinder: **Réti, Zsuzsanna, geb. Bosnyák, Dipl.-Chem., Haszkovo u. 35, H-8200 Veszprém (HU)**
Erfinder: **Sebok, Dezso, Dipl.-Chem., Halle u. 5/e, H-8200 Veszprém (HU)**
Erfinder: **Szabolcs, József, Dipl.-Chem., Táncsics u. 63, H-8220 Balatonalmádi (HU)**
Erfinder: **Tömördi, Elemér, Dipl.-Chem., H-8182 Peremarton, 91. sz. (HU)**

(74) Vertreter: **Beszédes, Stephan G. Dr., Münchener Strasse 80a Postfach 1168, D-8060 Dachau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Erzeugnisse, enthaltend mindestens 1 Antidotum und 1 oder mehr Thiocarbamat-, Carbamat-, Säureamid-, Harnstoffderivat- und/oder Thioharnstoffderivatherbizidwirkstoff(e) als Kombinationspräparat zur gleichzeitigen, getrennten oder zeitlich abgestuften Anwendung im Pflanzenschutz zur Unkrautbekämpfung sowie
2-(Dichlormethyl)-3-phenyl-1,3-oxazolidin und
2-(Dichlormethyl)-3-allyl-1,3-oxazolidin
und ein Verfahren zur Herstellung der letztgenannten Verbindungen.

Zur wirkungsvolleren Anwendung der Herbizide ist die Erhöhung ihrer Selektivität erforderlich. Die im Handel erhältlichen zahlreichen Herbizidwirkstoffe sind allein oder in Kombination mit anderen Herbizidwirkstoffen gegenüber sehr unterschiedlichen Unkrautarten in von der Widerstandsfähigkeit des Unkrauts abhängigen Konzentrationen direkt toxisch. Ihre wirtschaftliche Anwendung wird jedoch dadurch beschränkt beziehungsweise behindert, dass bei ihrer Anwendung in solchen Konzentrationen auch die Nutzpflanzen schwer geschädigt werden. Die Schädigung der Nutzpflanzen und ihre abnorme Entwicklung können eine bedeutende Senkung des Ernteertrages herbeiführen. Diese schädliche Wirkung kann durch Antidota (Gegengifte) für Herbizide gemindert und eventuell beseitigt werden. Solche Antidota und ihre Anwendung sind in den ungarischen Patentschriften 165 736, 173 775 und 174 487 beschrieben. Diese bekannten Antidota haben jedoch den Nachteil, dass sie nur für eine beschränkte Zahl von Kombinationen von Herbiziden und Nutzpflanzen eine positive Wirkung haben.

Ferner sind aus der US-Patentschrift 3 976 469 Antidota der allgemeinen Formel

$$R_1-\overset{\overset{O}{\|}}{C}-R_2 \qquad Oa,$$

worin

$R_1$ für einen Halogencycloalkylrest, einen niederen Alkylrest, einen niederen Halogenalkylrest, einen Phenylrest, einen Benzylrest oder einen p-Tolylrest steht und

$R_2$ einen niederen Halogenalkylrest, einen α-Halogenbenzylrest, einen Halogenacetonylrest oder einen Halogenalkylencarboalkoxyrest bedeutet,

zur Verringerung der Schädigung von Maispflanzen durch Herbizide, insbesondere Thiocarbamatherbizide, bekannt. Sie haben jedoch den Nachteil, dass ihre Schutzwirkung insbesondere bei Verwendung von hohen Aufwandmengen der Herbizide nicht ausreichend ist.

Weiterhin sind in der US-Patentschrift 4 021 228 Antidota der allgemeinen Formel

$$R_1-S-R_2 \qquad Ob,$$

worin

$R_1$ für einen Di-p-chlorphenylmethyl-, Phthalimidomethyl-, Pentachlorphenyl-, Alkenyl-, Halogenalkyl-, Chloralkenyl-, Aminoalkyl-, Hydroxyäthyl-, Carboxymethyl-, N-Alkylcarbamoylmethyl-, Monochlorbenzamidoäthyl-, Dichlorbenzamidoäthyl-, Monobrombenzamidoäthyl-, β-S-Äthylthiocarboxyaminoäthyl- oder Dichloracetamidoäthylrest steht und

$R_2$ einen p-Chlorphenyl-, Alkyl-, Halogenalkyl-, α-Hydroxytrichloräthyl-, Alkenyl-, Chloralkenyl-, Aminoalkyl-, Cyanalkyl-, Cyanchloralkyl-, Monochlorbenzamidoäthyl-, Dichlorbenzamidoäthyl-, Monobrombenzamidoäthyl-, β-S-Äthylthiocarboxyaminoäthyl- oder Dichloracetamidoäthylrest bedeutet,

zum Schutz von Kulturpflanzen vor der Schädigung durch Herbizide, insbesondere Thiocarbamatherbizide, beschrieben. Auch die Wirkung dieser Antidota ist jedoch insbesondere bei Verwendung von hohen Herbizidaufwandmengen unbefriedigend.

Ausserdem sind aus HOUBEN-WEYL, METHODEN DER ORGANISCHEN CHEMIE, VIERTE AUFLAGE, BAND VI/3, 1965, Seiten 204 bis 220 und 250 bis 252 Verfahren zur Herstellung von Acetalen oder Ketalen durch Umsetzen von Aldehyden beziehungsweise Ketonen mit Alkoholen in Gegenwart von sauren Katalysatoren oder durch Umacetalisieren von Acetalen in Gegenwart von sauren Katalysatoren bekannt. In dieser Druckschrift ist aber nicht im geringsten von Pflanzenschutzmitteln oder gar Antidota die Rede.

Ferner sind in der US-Patentschrift 4 197 110 Oxazolidin-, Thiazolidin- und Imidazolidinderivate und die entsprechenden heterocyclischen Derivate mit 6 oder 7 Ringgliedern sowie die entsprechenden Derivate mit ankondensiertem Benzolring, bei welchen in der 2-Stellung ein Monohalogen- oder Polyhalogenmethylrest ist und am Stickstoffatom beziehungsweise an mindestens 1 Stickstoffatom (3-Stellung) zwingend ein Acylrest sich befindet, und ihre Verwendung als Antidota bekannt. In der US-Patentschrift 4 197 110 sind auch als Zwischenprodukte Oxazolidin-, Thiazolidin- und Imidazolidinderivate und die entsprechenden heterocyclischen Derivate mit 6 oder 7 Ringgliedern sowie die entsprechenden Derivate mit ankondensiertem Benzolring, bei welchen in der 2-Stellung ein Monohalogen- oder Polyhalogenmethylrest ist und am Stickstoffatom (in der 3-Stellung) sich Wasserstoff befindet, beschrieben, wie es aus den Beispielen und der Formel IV in Spalte 3, Zeile 5 dieser Druckschrift hervorgeht.

Weiterhin sind aus der US-Patentschrift 3 989 503 1,3-Oxazolidin- und 1,3-Thiazolidinderivate, bei welchen am Stickstoffatom (3-Stellung) zwingend ein Acyl- oder Alkylthiocarbonylrest hängt und welche in den 2-, 4- und/oder 5-Stellungen als Substituenten Alkyl-, Alkoxyalkyl- und/oder Alkylolreste aufweisen können, bekannt. Ferner sind aus der US-Patentschrift 3 989 503 auch 1,3-Oxazolidin- und 1,3-Thiazolidinderivate, bei welchen am Stickstoffatom ein Wasserstoffatom ist, als Ausgangsstoffe gebracht.

Ausserdem sind aus der französischen Offenlegungsschrift 2 448 857 1,3-Oxazolidin- und 1,3-Thiazolidinderivate, bei welchen das Stickstoffatom (3-Stellung) zwingend durch einen Dichloracetylrest substituiert ist und das Kohlenstoffatom in der 2-Stellung durch einen, gegebenenfalls halogensubstituierten, Alkyl- oder Phenylrest substituiert sein kann und die entsprechenden Tetrahydrooxazinderivate und Tetrahydrooxathiazinderivate, sowie ihre Verwendung als Antidota bekannt. Es handelt sich also auch in der französischen Offenlegungsschrift 2 448 857 um Acyloxazolidine beziehungsweise -thiazolidine wie in den US-Patentschriften 3 989 503 und 4 197 110, jedoch mit der Einschränkung, dass die Acylgruppe im erstgenannten Fall nur eine Dichloracetylgruppe sein kann. Die überwiegende Mehrzahl der Verbindungen der französischen Offenlegungsschrift 2 448 857 sind Spiroderivate mit einer komplizierten chemischen Struktur.

Ferner sind aus der deutschen Offenlegungsschrift 2 134 499 1,2-Methylendioxybenzolderivate, bei welchen der Benzolring durch einen, gegebenenfalls halogensubstituierten, Alkyl- oder Alkenylrest substituiert ist, als Pflanzenwachstumsregler bekannt.

Schliesslich sind aus der deutschen Offenlegungsschrift 2 341 469 Verbindungen, welche eine Methylendioxyphenylgruppe aufweisen, als Mittel zum Schutz von Pflanzen gegen oxydative Schädigung durch Luft bekannt. In den einzelnen erwähnten Verbindungen ist der Benzolring stets substituiert.

Der Erfindung liegt die Aufgabe zugrunde, unter Behebung der Nachteile des Standes der Technik Erzeugnisse, enthaltend mindestens 1 Antidotum, durch welche die schädliche Wirkung von mehr Herbiziden auf verschiedene Kulturpflanzen wesentlich gesenkt werden kann, welche also eine breitere Anwendung haben, sowie neue 1,3-Oxazolidinderivate mit solcher Wirkung und ein Verfahren zur Herstellung der neuen 1,3-Oxazolidinderivate zu schaffen.

Das Obige wurde überraschenderweise durch die Erfindung erreicht.

Es wurde nämlich überraschenderweise festgestellt, dass durch die im folgenden festgelegten Antidota, deren chemische Struktur sich von der der bekannten Antidota grundlegend unterscheidet, im Gegensatz zu den letzteren eine wesentliche Senkung beziehungsweise Verhinderung der schädlichen Wirkung der Herbizide auf verschiedene Kulturpflanzen in einem breiteren Anwendungsbereich hinsichtlich der Herbizide und Kulturpflanzen erreicht werden kann, wenn die Samen der Kulturpflanzen und/oder der Boden, in welchem sie wachsen, vor dem Pflanzen und/oder Säen mit 1 oder mehr der im folgenden festgelegten Antidota in Mischung mit dem Herbizid beziehungsweise den Herbiziden und/oder vor und/oder nach der Behandlung mit dem letzteren beziehungsweise den letzteren behandelt werden beziehungsweise wird.

Gegenstand der Erfindung sind daher Erzeugnisse, enthaltend mindestens 1 Antidotum der allgemeinen Formel

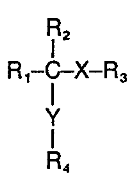

I ,

worin

X und Y unabhängig voneinander für Sauerstoff, Schwefel, einen Rest der allgemeinen Formel

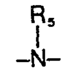

II ,

in welch letzterer

$R_5$ Wasserstoff, einen, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierten, Phenylrest, einen Alkylrest mit 1 bis 4 Kohlenstoffatom(en) oder einen Alkenylrest mit 3 oder 4 Kohlenstoffatomen bedeutet,

beziehungsweise einen Rest der allgemeinen Formel

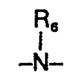

III ,

in welch letzterer

$R_6$ Wasserstoff, einen, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierten, Phenylrest, einen Alkylrest mit 1 bis 4 Kohlenstoffatom(en) oder einen Alkenylrest mit 3 oder 4 Kohlenstoffatomen bedeutet,

stehen,

$R_1$ und $R_2$ unabhängig voneinander Wasserstoff, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierte, Phenylrest(e), Furylrest(e), gegebenenfalls durch 1 oder mehr Halogenatom(e) substituierte, Phenylalkylreste mit 1 bis 4 Kohlenstoffatom(en) im Alkylteil, geradkettige oder verzweigte Alkyl-, Mono- beziehungsweise Polyhalogenalkyl- beziehungsweise Mono- beziehungsweise Polycyanalkylreste mit 1 bis 4 Kohlenstoffatom(en) beziehungsweise geradkettige oder verzweigte Alkenylreste mit 2 bis 4 Kohlenstoffatomen bedeuten und von diesen Symbolen wahlweise,

falls X und/oder Y für einen Rest beziehungsweise Reste der allgemeinen Formel(n) II und/oder III steht beziehungsweise stehen,

$R_1$ auch eine das Kohlenstoffatom, an welches es gebunden ist, mit X oder Y verbindende zweite Bindung darstellen kann, und

$R_3$ und $R_4$ unabhängig voneinander für Wasserstoff, geradkettige oder verzweigte Alkylreste mit 1 bis 18 Kohlenstoffatom(en), Mono- beziehungsweise Polyhalogenalkyl- beziehungsweise Mono- beziehungsweise Polycyanalkylreste jeweils mit 1 bis 4 Kohlenstoffatom(en), geradkettige oder verzweigte Alkoxyalkyl- beziehungsweise Alkoxyalkoxyalkylreste mit 1 bis 4 Kohlenstoffatom(en) im Alkylteil und 1 bis 4 Kohlenstoffatom(en) im Alkoxyteil beziehungsweise in den Alkoxyteilen, Cycloalkylreste mit 5 oder 6 Kohlenstoffatomen, Phenylalkylreste mit 1 oder 2 Kohlenstoffatom(en) im Alkylteil, Alkenylreste mit 3 oder 4 Kohlenstoffatomen, Alkinylreste mit 3 oder 4 Kohlenstoffatomen, Phenylalkenylreste mit 3 oder 4 Kohlenstoffatomen im Alkenylteil, Dialkylaminoalkylreste mit 1 bis 4 Kohlenstoffatom(en) in jedem Alkylteil, Hydroxyalkylreste mit 1 bis 4 Kohlenstoffatom(en), Furfurylreste beziehungsweise Tetrahydrofurfurylreste stehen oder

$R_3$ und $R_4$ zusammen einen X und Y unter Bildung eines 5- bis 7-gliedrigen Ringes verbindenden, gegebenenfalls durch 1 oder mehr Hydroxygruppe(n), Methoxyrest(e), Halogenatom(e) und/oder Acetoxyrest(e) substituierten, Alkylenrest mit 2 bis 4 Kohlenstoffatom(en), Alkenylenrest mit 2 bis 4 Kohlenstoffatomen oder Phenylenrest oder zusammen einen X und Y verbindenden Glucofuranosylenrest darstellen,

mit den weiteren Massgaben, dass

a) im Falle dass mindestens 1 von $R_1$ und $R_2$ für einen Methyl-, Monochlormethyl- oder Monochlorphenylrest steht, mindestens 1 von $R_3$ und $R_4$ von einem nicht substituierten Alkylrest mit 2 bis 18 Kohlenstoffatomen verschieden ist, und

b) im Falle dass $R_2$ für Wasserstoff steht und $R_1$ einen nicht substituierten Phenylrest bedeutet, mindestens 1 von $R_3$ und $R_4$ von einem Methylrest verschieden ist,

und 1 oder mehr Thiocarbamat-, Carbamat-, Säureamid-; Harnstoffderivat- und/oder Thioharnstoffderivatherbizidwirkstoff(e) als Kombinationspräparat zur gleichzeitigen, getrennten oder zeitlich abgestuften Anwendung im Pflanzenschutz zur Unkrautbekämpfung, zweckmässig zusammen mit 1 oder mehr üblichen Träger- und/oder Hilfsstoff(en).

Die Antidota der erfindungsgemässen Erzeugnisse unterscheiden sich von denen der US-Patentschrift 3 976 469 grundlegend insbesondere darin, dass sie keine Ketone sind und sie daher nicht die Formel Oa haben, wobei sie überhaupt keine Ketogruppe aufweisen und daher auch nicht mit Oxoreagenzien reagieren.

Die Antidota der erfindungsgemässen Erzeugnisse unterscheiden sich von denen der US-Patentschrift 4 021 228 grundlegend insbesondere darin, dass sie keine Sulfide sind und daher nicht die allgemeine Formel Ob haben, wobei sie, sofern sie überhaupt Schwefel enthalten, offenkettige oder ringförmige Thioacetale, Thioketale beziehungsweise heterocyclische Verbindungen sind.

Ferner ist kein einziges der Antidota der erfindungsgemässen Erzeugnisse in der Druckschrift HOUBEN-WEYL, METHODEN DER ORGANISCHEN CHEMIE, VIERTE AUFLAGE, BAND VI/3, 1965 enthalten, geschweige denn von ihrer Verwendung in Pflanzenschutzmitteln oder gar als Antidota die Rede.

Von den Verbindungen der US-Patentschrift 4 197 110, für welche in der genannten Entgegenhaltung eine Antidotumwirkung angegeben ist, unterscheiden sich die Antidota der erfindungsgemässen Erzeugnisse darin, dass sie am Stickstoffatom beziehungsweise im Falle des Vorliegens von 2 Stickstoffatomen an beiden Stickstoffatomen keinen Acylrest aufweisen können, denn gemäss der obigen Definition von $R_5$ und $R_6$ sowie $R_3$ und $R_4$ können an diesen keine Acylreste hängen. In Bezug auf die in der US-Patentschrift 4 197 110 nur als Zwischenprodukte genannten Oxazolidin-, Thiazolidin- und Imidazolidinderivate ist darauf hinzuweisen, dass aus dieser Druckschrift zwar alle Antidotaverbindungen der erfindungsgemässen Erzeugnisse, bei welchen X für Sauerstoff, Schwefel oder einen Rest der allgemeinen Formel II mit $R_5$ = Wasserstoff, Arylrest, Alkenylrest oder Alkylrest steht, Y einen Rest der allgemeinen Formel III mit $R_6$ = Wasserstoff bedeutet, $R_3$ und $R_4$ zusammen einen X und Y unter Bildung eines 5- bis 7-gliedrigen Ringes mit Sauerstoff, Stickstoff oder Schwefel in der 1-Stellung und Stickstoff in der 3-Stellung verbindenden Alkylenrest darstellen, $R_1$ für einen Monohalogen- oder Polyhalogenmethylrest steht und $R_2$ Wasserstoff bedeutet, an sich bekannt sind, für diese Verbindungen aber keine biologische Wirkung, geschweige denn eine Antidotumwirkung angegeben ist. In diesem Zusammenhang ist darauf hinzuweisen, dass nach dem Fachschrifttum nicht die Aminverbindungen, sondern nur ihre acylierten, insbesondere dichloracetylierten, Derivate eine Antidotumwirkungen haben (siehe beispielsweise Weed Research, 1982, Volume 22, Seiten 27 bis 32, insbesondere Seite 32, linke Spalte, letzte Zeile bis rechte Spalte, Zeile 2, J. Agric. Food Chem., Vol. 26, No. 1, 1978, Seiten 137 bis 140, insbesondere Seite 139, linke Spalte, Zeilen 26 von unten bis 20 von unten und Seite 139, rechte Spalte, Table III, und AGRICULTURAL AND FOOD CHEMISTRY, Vol. 27, No. 3, May/June 1979, Seiten 543 bis 547, insbesondere Seite 544, rechte Spalte, Table II). Sehr anschaulich zeigt die zweitgenannte von diesen Druckschriften, dass selbst wenn die Dichloracetylgruppe durch eine andere Säuregruppe ersetzt wurde, also nicht einmal die Acylgruppe ganz weggelassen wurde, eine schlechtere Antidotumwirksamkeit sich ergab. Daraus konnte der Fachmann nur folgern, dass, wenn die Dichloracetylgruppe ganz weggelassen, also durch Wasserstoff ersetzt wird, erst recht keine befriedigende Antidotumwirkung zu erzielen ist. Die gegenteilige erfindungsgemässe Feststellung ist daher überraschend.

Von den in der US-Patentschrift 3 989 503 als Antidota beschriebenen Verbindungen unterscheiden sich alle Antidota der erfindungsgemässen Erzeugnisse darin, dass sie am Stickstoffatom (3-Stellung) keinen Acylrest aufweisen können. Ausserdem sind die (von Wasserstoff verschiedenen) Substituenten, welche $R_1$ und $R_2$ in der vorliegenden Patentanmeldung bedeuten können, und die für die gegebenenfalls vorliegende Substitution des Alkylenrestes, den $R_3$ und $R_4$ zusammen darstellen können, in der vorliegenden Patentanmeldung andere als in der US-Patentschrift 3 989 503. In Bezug auf die in der US-Patentschrift 3 989 503 nur als Ausgangsstoffe verwendeten 1,3-Oxazolidin- und 1,3-Thiazolidinderivate, bei welchen am Stickstoffatom ein Wasserstoffatom ist, ist darauf hinzuweisen, dass damit zwar die Antidotaverbindungen der erfindungsgemässen Erzeugnisse, welche 1,3-Oxazolidin- beziehungsweise 1,3-Thiazolidinderivate mit $R_1$ = Wasserstoff, $R_2$ = Wasserstoff, X = Sauerstoff, Y = Rest

der Formel II, $R_3 + R_4$ = Alkylenrest ohne Substitution und $R_5$ = Wasserstoff sind, an sich bekannt sind, eine Antidotawirkung für diese Verbindungen jedoch in der US-Patentschrift 3 989 503 nicht angegeben ist. Auch gehen aus der US-Patentschrift 3 989 503 keine Antidotaverbindungen der erfindungsgemässen Erzeugnisse, bei welchen $R_1$, $R_2$ und/oder $R_5$ von Wasserstoff verschieden ist beziehungsweise sind und/oder am Alkylenrest, welchen $R_3$ und $R_4$ zusammen darstellen, [einen] Substituenten aufweisen, hervor, und zwar nicht einmal an sich. Daher gelten im vorstehenden Zusammenhang die bezüglich der US-Patentschrift 4 197 110 gemachten Ausführungen sinngemäss.

Von den Antidota der französischen Offenlegungsschrift 2 448 857 unterscheiden sich alle Antidota der erfindungsgemässen Erzeugnisse darin, dass sie am Stickstoffatom (3-Stellung) zwingend keinen Dichloracetylrest aufweisen können. Überdies haben in einer Reihe von in erfindungsgemässen Erzeugnissen als Antidota verwendeten 1,3-Oxazolidin- und 1,3-Thiazolidinderivaten $R_1$ und/oder $R_2$ andere Bedeutungen als die Substituenten am Kohlenstoffatom in der 2-Stellung der Verbindungen der französischen Offenlegungsschrift 2 448 857; ausserdem können in einer Reihe von Antidotaverbindungen der erfindungsgemässen Erzeugnisse am Alkylenrest, den $R_3$ und $R_4$ zusammen darstellen können, Substituenten vorliegen, was für die Antidota der französischen Offenlegungsschrift 2 448 857 nicht vorgesehen ist.

Alle Antidota der erfindungsgemässen Erzeugnisse sind von den in der deutschen Offenlegungsschrift 2 134 499 enthaltenen Verbindungen verschieden, denn, wenn $R_3$ und $R_4$ zusammen einen Phenylenrest darstellen, dann ist dieser zwingend nicht substituiert und ausserdem können dann $R_1$ und $R_2$ nicht Wasserstoffatome bedeuten.

Von den in der deutschen Offenlegungsschrift 2 341 469 im einzelnen erwähnten Verbindungen unterscheiden sich alle Antidota der erfindungsgemässen Erzeugnisse darin, dass, soweit überhaupt $R_3$ und $R_4$ zusammen einen Phenylenrest darstellen, dieser nicht substituiert ist.

Die Alkylreste, auch soweit sie substituiert sind oder Teile von Resten darstellen, können geradkettig oder verzweigt sein. Die Halogenatome der Mono- beziehungsweise Polyhalogenalkylreste können vorteilhaft Chlor, Brom und/oder Fluor sein. Ihre Zahl beträgt vorteilhaft 1 bis 4.

Bevorzugte Ausführungsformen der erfindungsgemässen Erzeugnisse sind Gegenstände der Patentansprüche 2 bis 22.

Es ist besonders bevorzugt, dass der/die Alkylrest(e), für den/die $R_1$ und/oder $R_2$ stehen kann/können, [ein] Methylrest(e) ist/sind.

Weiterhin ist es besonders bevorzugt, dass der Mono- oder Polyhalogenalkylrest, für den $R_1$ stehen kann, ein 1,2-Dichloräthyl-, 1,2-Dibromäthyl- oder 1,2-Dibrom-n-propylrest ist.

Vorteilhaft ist der Phenylalkylrest, für den $R_1$ stehen kann, soweit er halogensubstituiert ist, in seinem Alkylteil halogensubstituiert, es ist aber statt dessen oder zusätzlich dazu auch eine Substitution in seinem Phenylteil möglich.

Ferner ist es ganz besonders bevorzugt, dass der/die Alkylrest(e), für den/die $R_3$ und/oder $R_4$ stehen kann/können, ein solcher/solche mit 1 bis 5 Kohlenstoffatom(en) ist/sind.

Weiterhin ist es besonders bevorzugt, dass der/die Monohalogenalkylrest(e), für den/die $R_3$ und/oder $R_4$ stehen kann/können, ein solcher/solche mit 1 oder 2 Kohlenstoffatom(en) ist/sind.

Vorzugsweise ist/sind der/die Alkoxyalkyl- und/oder Alkoxyalkoxyalkylrest(e), für den/die $R_3$ und/oder $R_4$ stehen kann/können, ein solcher/solche mit 1 oder 2 Kohlenstoffatom(en) im Alkylteil.

Besonders bevorzugt ist/sind der/die Phenylalkylrest(e), für den/die $R_3$ und/oder $R_4$ stehen kann/können, ein solcher/solche mit 1 Kohlenstoffatom im Alkylteil.

Besonders bevorzugt ist/sind der/die Dialkylaminoalkylrest(e), für den/die $R_3$ und/oder $R_4$ stehen kann/können, [ein] Dimethylaminoäthyl- und/oder Diäthylaminoäthylrest(e).

Gegenstand der Erfindung sind auch die unter die allgemeine Formel I fallenden neuen Verbindungen 2-(Dichlormethyl)-3-phenyl-1,3-oxazolidin und 2-(Dichlormethyl)-3-allyl-1,3-oxazolidin.

Besonders bevorzugte Gruppen der Antidotaverbindungen der allgemeinen Formel I der erfindungsgemässen Erzeugnisse sind wie folgt:

A) Verbindungen, bei welchen $R_1$ für einen geradkettigen oder verzweigten Aklyl-, Mono- oder Polyhalogenalkyl- oder Monocyanalkylrest jeweils mit 1 bis 4 Kohlenstoffatom(en) oder einen geradkettigen Alkenylrest mit 2 bis 4 Kohlenstoffatomen steht, $R_2$ Wasserstoff bedeutet, X Sauerstoff darstellt und Y für Sauerstoff oder Schwefel steht.

B) Verbindungen, bei welchen X und Y für Sauerstoff stehen, $R_1$ für einen geradkettigen oder verzweigten Alkyl-, Mono- oder Polyhalogenalkyl- oder Monocyanalkylrest jeweils mit 1 bis 4 Kohlenstoffatom(en) oder einen geradkettigen Alkenylrest mit 2 bis 4 Kohlenstoffatomen steht und $R_2$ einen Alkylrest mit 1 bis 4 Kohlenstoffatom(en) oder einen Phenylrest bedeutet.

C) Verbindungen, bei welchen $R_1$, $R_2$, X und Y wie unter A) festgelegt sind und $R_3$ und $R_4$ zusammen einen Alkylenrest mit 2 bis 4 Kohlenstoffatomen oder einen Alkenylenrest mit 2 bis 4 Kohlenstoffatomen darstellen.

D) Verbindungen, bei welchen $R_1$ für einen Dichlormethyl- oder Trichlormethylrest steht, $R_2$ Wasserstoff bedeutet, X und Y jeweils Sauerstoff bedeuten und $R_3$ und $R_4$ zusammen einen Glucofuranosylenrest darstellen.

E) Verbindungen, bei welchen $R_3$ und $R_4$ zusammen einen Alkylenrest mit 2 bis 4 Kohlenstoffatomen oder einen Alkenylenrest mit 2 bis 4 Kohlenstoffatomen darstellen, X für Sauerstoff steht und Y einen Rest der allgemeinen Formel III, deren $R_6$ für einen Alkylrest mit 1 bis 4 Kohlenstoffatom(en), einen Alkenylrest mit 3 oder 4 Kohlenstoffatomen oder einen Phenylrest steht, bedeutet.

F) Verbindungen, bei welchen $R_3$ und $R_4$ zusammen einen Alkylenrest mit 2 bis 4 Kohlenstoffatomen darstellen und X einen Rest der allgemeinen Formel II und Y einen Rest der allgemeinen Formel III, deren $R_5$ und $R_6$ unabhängig voneinander für einen Alkylrest mit 1 bis 4 Kohlenstoffatom(en), einen Alkenylrest mit 3 oder 4 Kohlenstoffatomen oder einen Phenylrest stehen, bedeuten.

G) Verbindungen, bei welchen $R_1$ für einen Brommethylrest steht, $R_2$ Wasserstoff bedeutet und X und Y wie unter A) festgelegt sind.

H) Verbindungen, bei welchen $R_1$ für einen 1,2-Dibromäthylrest steht, $R_2$ Wasserstoff bedeutet und X und Y wie unter G) festgelegt sind.

I) Verbindungen, bei welchen $R_1$ für einen Chlormethylrest steht, $R_2$ Wasserstoff bedeutet und X und Y wie unter G) festgelegt sind.

K) Verbindungen, bei welchen $R_1$ für einen Dichlormethylrest steht, $R_2$ Wasserstoff bedeutet und X und Y wie unter G) festgelegt sind.

L) Verbindungen, bei welchen $R_1$ für einen 1,2-Dichloräthylrest steht, $R_2$ Wasserstoff bedeutet und X und Y wie unter G) festgelegt sind.

M) Verbindungen, bei welchen $R_1$ für einen Dichlormethylrest steht, $R_2$ Wasserstoff bedeutet und $R_3$ und $R_4$ unabhängig voneinander geradkettige beziehungsweise verzweigte Alkylreste mit 1 bis 4 Kohlenstoffatom(en) darstellen und X und Y jeweils Sauerstoff bedeuten.

N) Verbindungen, bei welchen $R_1$ für einen Dichlormethylrest steht, $R_2$ Wasserstoff bedeutet, $R_3$ und $R_4$ zusamen einen But-1,2-ylenrest darstellen und X und Y jeweils Sauerstoff bedeuten.

O) Verbindungen, bei welchen $R_1$ für einen Dichlormethylrest steht, $R_2$ Wasserstoff bedeutet, $R_3$ und $R_4$ zusammen einen Äthylenrest darstellen, X für Sauerstoff steht und Y einen Rest der allgemeinen Formel III, deren $R_6$ für einen Allyl- oder Phenylrest steht, bedeutet.

P) Verbindungen, bei welchen $R_1$ für einen Brommethylrest steht und $R_2$, $R_3$, $R_4$, X und Y wie unter M) festgelegt sind.

Q) Verbindungen, bei welchen $R_2$, $R_3$, $R_4$, X und Y wie unter P) festgelegt sind und $R_1$ für einen 1,2-Dibrompropylrest steht.

Vorteilhaft enthalten die erfindungsgemässen Erzeugnisse das Antidotum beziehungsweise die Antidota der allgemeinen Formel I in einer Menge von 0,01 bis 15 Gew.-%, bezogen auf das Gewicht des Herbizidwirkstoffes beziehungsweise der Herbizidwirkstoffe, und vorteilhaft beträgt die Gesamtmenge des Antidotums beziehungsweise der Antidota und des Herbizidwirkstoffes beziehungsweise der Herbizidwirkstoffe, bezogen auf die Gesamtmenge des Erzeugnisses, 0,1 bis 95 Gew.-%, während der Rest 1 oder mehr feste[r] und/oder flüssige[r] neutrale[r] Träger und/oder anionenaktive[r], kationenaktive[r] und/oder nicht-ionogene[r] oberflächenaktive[r] Stoff(e) ist beziehungsweise sind.

Vorteilhaft können die erfindungsgemässen Erzeugnisse, in welchen das Antidotum beziehungsweise die Antidota der allgemeinen Formel I enthalten ist beziehungsweise sind, als Thiocarbamat(e)

S-Äthyl-N,N-di-n-propylthiocarbamat [EPTC],
S-Äthyl-N,N-diisobutylthiocarbamat [Butylat],
S-n-Propyl-N,N-di-n-propylthiocarbamat [Vernolat],
S-n-Propyl-N-n-butyl-N-äthylthiocarbamat [Pebulat],
S-Äthyl-N,N-hexamethylenthiocarbamat [Molinat],
S-Äthyl-N-äthyl-N-cyclohexylthiocarbamat [Cycloat] und/oder
S-(2,3-Dichlorallyl)-N,N-diisopropylthiocarbamat [Diallat]

enthalten.

Ferner können die erfindungsgemässen Erzeugnisse, in welchen das Antidotum beziehungsweise die Antidota der allgemeinen Formel I enthalten ist beziehungsweise sind, als Carbamat vorteilhaft O-{4-(Chlor)-n-but-2-inyl}-N-(3′-chlorphenyl)-carbamat [Barban] enthalten.

Weiterhin können die erfindungsgemässen Erzeugnisse, in welchen das Antidotum beziehungsweise die Antidota der allgemeinen Formel I enthalten ist beziehungsweise sind, als Säureamid(e), vor allem Chloracetanilid(e), vorteilhaft

2′-Äthyl-6′-methyl-N-(äthoxymethyl)-2-chloracetanilid [Acetochlor],
2′,6′-Diäthyl-N-(methoxymethyl)-2-chloracetanilid [Alachlor] und/oder
6′-Äthyl-2′-methyl-N-(2″-methoxy-1″-methyläthyl)-2-chloracetanilid [Metolachlor]

enthalten.

Auch können die erfindungsgemässen Erzeugnisse, in welchen das Antidotum beziehungsweise die Antidota der allgemeinen Formel I enthalten ist beziehungsweise sind, vorteilhaft als Harnstoffderivat 3-(3′,4′-Dichlorphenyl)-1-methoxy-1-methylharnstoff [Linuron] enthalten.

Die Antidota der allgemeinen Formel I der erfindungsgemässen Erzeugnisse beziehungsweise erfindungsgemässen Verbindungen beziehungsweise Erzeugnisse können in jeder für praktische Zwecke üblichen Form vorliegen und angewandt werden. So können sie emulgierbare Flüssigkeiten, emulgierbare Konzentrate, benetzbare Pulver oder körnige Präparate sein.

Als feste beziehungsweise flüssige Trägerstoffe können in den Erzeugnissen, in welchen das Antidotum beziehungsweise die Antidota der allgemeinen Formel I enthalten ist beziehungsweise sind, beispielsweise Kaolin, Talk, Wasser und/oder organische Lösungs- beziehungsweise Verdünnungsmittel, enthalten sein. Als oberflächenaktive Stoffe können in ihnen Netz- und/oder Emulgiermittel vorliegen. Auch können sie sonstige übliche Zusätze enthalten.

Als Herbizidwirkstoff(e) ist beziehungsweise sind 1 oder mehr Thiocarbamat(e) am meisten bevorzugt.

Die den beziehungsweise die Herbizidwirkstoff(e), das Antidotum beziehungsweise die Antidota der allgemeinen Formel I und den beziehungsweise die Träger- und/oder Hilfsstoff(e) enthaltenden Pflanzenschutzmittelpräparate können vor der Aussaat oder danach in den Boden eingebracht werden oder es können aber auch die auszustreuenden Samen mit ihnen behandelt werden. Es kann auch in der Weise vorgegangen

werden, dass einerseits der beziehungsweise die Herbizidwirkstoff(e) und andererseits das Antidotum beziehungsweise die Antidota der allgemeinen Formel I der erfindungsgemässen Erzeugnisse beziehungsweise die erfindungsgemässe(n) Verbindung(en) jeweils gesondert mit 1 oder mehr entsprechenden flüssigen oder festen Träger- und/oder Hilfsstoff(en) zu einem für die Behandlung von Boden, Samen beziehungsweise Pflanzen geeigneten Pflanzenschutzmittelpräparat zubereitet und diese nacheinander in den Boden eingebracht oder auf die zu behandelnden Samenkörner oder Pflanzen aufgebracht werden. Nach einer Abwandlung dieser Verfahrensweise kann beziehungsweise können das Antidotum beziehungsweise die Antidota der erfindungsgemässen Erzeugnisse beziehungsweise die erfindungsgemässe(n) Verbindung(en) auf die Samen aufgebracht und der beziehungsweise die Herbizidwirkstoff(e) vor oder nach der Aussaat in den Boden eingebracht beziehungsweise auf ihn aufgebracht werden.

Jedes der Antidota der allgemeinen Formel I der erfindungsgemässen Erzeugnisse beziehungsweise jede der erfindungsgemässen Verbindungen kann zusammen mit solchen Herbizidwirkstoffen, welche die herbiziden Thiocarbamate, Carbamate, Säureamide, Harnstoff- und/oder Thioharnstoffderivat(e) allein oder in irgendeiner Kombination enthalten, eingesetzt werden. Bei einzelnen Herbizidwirkstoffen beziehungsweise deren Kombinationen kann sich die herbizide Wirksamkeit ändern, wobei sie bis zu einem bestimmten Grad von den Pflanzenarten, für welche sie angewandt werden, abhängen kann. Mit Einsatz eines der Antidota der allgemeinen Formel I der erfindungsgemässen Erzeugnisse beziehungsweise einer der erfindungsgemässen Verbindung(en) kann in jedem dieser Fälle der für Nutzpflanzen schädlichen Wirkung des gewählten Herbizidwirkstoffes beziehungsweise der gewählten Herbizidwirkstoffkombination vorgebeugt werden.

Unabhängig von jeglichem konkreten Wirkungsmechanismus besteht der Vorteil der die Verbindungen der allgemeinen Formel I als Antidota enthaltenden erfindungsgemässen Erzeugnisse beziehungsweise der unter die allgemeine Formel I fallenden erfindungsgemässen 1,3-Oxazolidinderivate darin, dass mit ihnen die schädliche Wirkung der Herbizide auf Nutzpflanzen ohne Senkung der unkrautvernichtenden Wirkung vermindert oder beseitigt ist.

Der Schädigung von Nutzpflanzenarten durch Herbizide oder Herbizidkombinationen kann also mit den Antidota der erfindungsgemässen Erzeugnisse vorgebeugt werden. Besonders vorteilhaft können durch die Antidota der allgemeinen Formel I enthaltenden erfindungsgemässen Erzeugnisse Mais und Kornhirse geschützt werden. Der Kreis der Nutzpflanzen, die gegen die Schädigung durch Herbizidwirkstoffe geschützt werden können, beschränkt sich dabei aber nicht auf diese Kulturpflanzen.

Die in den erfindungsgemässen Erzeugnissen mit den Antidota der allgemeinen Formel I in Kombination einsetzbaren Herbizidwirkstoffe sind gegen sehr viele Arten von Pflanzen wirksam. Gegebenenfalls hängt die für die gewünschte Wirkung notwendige Menge auch von der Art der erwarteten Wirkung und den örtlichen Bedingungen ab.

Gegenstand der Erfindung ist auch ein Verfahren zur Herstellung der obigen erfindungsgemässen Verbindungen, welches dadurch gekennzeichnet ist, dass in an sich bekannter Weise Dichloracetaldehyd mit N-(Phenyl)-äthanolamin oder N-(Allyl)-äthanolamin in aromatischen Kohlenwasserstoffen, insbesondere Benzol, in etwa stöchiometrischen Mengenverhältnissen vermischt und mehrere, zweckmässig 4 bis 11, Stunden unter Rückfluss zum Sieden erhitzt werden.

Analog können auch die anderen Antidota der allgemeinen Formel I der erfindungsgemässen Erzeugnisse hergestellt werden.

Im erfindungsgemässen Verfahren wird zweckmässig das entstehende Wasser laufend aus dem System (beispielsweise mit einem Dean-Stark-Gerät) entfernt.

Das Reaktionsprodukt kann je nach seiner Art durch Filtrieren oder Destillieren abgetrennt werden.

Die Verbindungen der allgemeinen Formel I, bei welchen X und Y beide Sauerstoff bedeuten oder X für Sauerstoff steht und Y Schwefel bedeutet, können durch Umsetzen von Dialkylacetalen der allgemeinen Formel

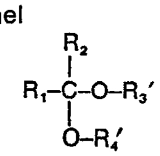

V ,

worin $R_1$ und $R_2$ wie weiter oben festgelegt sind und $R_3'$ und $R_4'$ für gleiche oder verschiedene Alkylreste mit 1 bis 4 Kohlenstoffatom(en) stehen, mit passenden 1- oder 2-wertigen Alkoholen oder Thioalkoholen in Gegenwart von Katalysatoren unter Erhitzen zum Sieden hergestellt werden. Als Katalysator wird vorzugsweise 4-Methylbenzolsulfonsäure verwendet. Aber auch andere Säuren, wie Essigsäure, Toluolsulfonsäuren oder Schwefelsäure, und Säureester, wie Orthoameisensäuretriäthylester, sind verwendbar.

Die im erfindungsgemässen Verfahren als Ausgangsstoffe verwendeten Verbindungen der allgemeinen Formel IV beziehungsweise einige der Verbindungen der allgemeinen Formel I, insbesondere solche, bei welchen $R_3$ und $R_4$ gleiche oder verschiedene Alkylreste mit 1 bis 4 Kohlenstoffatom(en) bedeuten und X und Y beide Sauerstoff oder eines von beiden Sauerstoff und das andere Schwefel darstellen, können beziehungsweise können auch nach verschiedenen speziellen und allgemeinen Verfahren, die im Fachschrifttum, beispielsweise in

Houben-Weyl, Methoden der organischen Chemie, IV. Auflage, Georg-Thieme-Verlag, Stuttgart, Band V/3 [1962], Seiten 611 bis 612, 616 und 619, Band V/4 [1960], Seiten 163 bis 170, 173 und 175 bis 182, Band VI/3 [1965], Seiten 250 bis 252, 256 bis 259, 275 bis 278 und 286,

Ullmanns Encyklopädie der technischen Chemie, Urban und Schwarzenberg, München-Berlin, III. Auflage [1954], Band 5, Seite 319 sowie

D.P. Wyman und P.R. Kaufman, J. Org. Chem. 29 [1964], Seiten 1 956 bis 1 960

beschrieben sind, hergestellt werden.

Die Herstellung von Antidota der allgemeinen Formel I von erfindungsgemässen Erzeugnissen und der erfindungsgemässen Verbindungen wird an Hand der folgenden Beispiele näher erläutert.

Beispiel 1

Dichloracetaldehyddiäthylacetal

Es wurde in auf 25 °C gehaltenes 95%-iges Äthanol solange Chlorgas eingeführt, bis 2 Phasen entstanden. Die organische Phase wurde über Calciumcarbonat getrocknet und dann destilliert. So wurde Dichloracetaldehyddiäthylacetal mit einem Siedepunkt von 107 bis 108 °C/38 mm Hg erhalten.

Beispiel 2

2-(Trichlormethyl)-N-(phenyl)-oxazolidin

Es wurden 7 g Chloralhydrat in einem Gemisch aus 5 $cm^3$ Essigsäure, 50 $cm^3$ Benzol und 7 g N-(Phenyl)-äthanolamin gelöst. Das Gemisch wurde 5 Stunden lang erwärmt und das sich bildende Wasser wurde abgetrennt. Dann wurden das Benzol und die Essigsäure abdestilliert und das Produkt wurde unter Vakuum destilliert. So wurde 2-(Trichlormethyl)-N-(phenyl)-oxazolidin mit einem Siedepunkt von 140 bis 141 °C/5 mm Hg erhalten.

Beispiel 3

2,3-Dibrompropionaldehyddiäthylacetal

Es wurden 7 g 1,2-Dibrompropionaldehyd einem Gemisch von 5 g Orthoameisensäuretriäthylester und 10 $cm^3$ Äthanol zugemischt. Die Lösung wurde 2 Stunden lang unter Rückfluss zum Sieden erhitzt, über Nacht stehengelassen und dann unter Vakuum destilliert. So wurde 2,3-Dibrompropionaldehyddiäthylacetal mit einem Siedepunkt von 107 bis 108 °C/11 mm Hg erhalten.

Beispiel 4

Trichloracetaldehyddi-n-pentylacetal

Es wurde ein Gemisch aus 5 g Chloralhydrat, 10 $cm^3$ Amylalkohol, 0,1 g 4-Methylbenzolsulfonsäure und 50 $cm^3$ Benzol auf der Rückflusstemperatur gehalten und das sich bildende Wasser wurde mit Hilfe eines Dean-Stark-Gerätes abgetrennt. Nach einer Reaktionsdauer von 9 Stunden wurde die benzolische Lösung mit einer Natriumbicarbonatlösung und Wasser gewaschen und über Magnesiumsulfat getrocknet und dann wurde das Benzol unter Vakuum abdestilliert. So wurden Trichloracetaldehyddi-n-pentylacetal mit einem $n_D^{20}$-Wert von 1,4549 erhalten.

Beispiel 5

Dichloracetaldehydmethylacetal

Es wurde ein Gemisch von 8 g Dichloracetaldehyddiäthylacetal, 30 $cm^3$ Methanol und 0,1 g 4-Methylbenzolsulfonsäure 5 Stunden lang auf der Rückflusstemperatur gehalten und dann destilliert. So wurden Dichloracetaldehyddimethylacetal mit einem Siedepunkt von 167 bis 168 °C erhalten.

Beispiel 6

2-(Trichlormethyl)-oxazolidin

Es wurden 6 g Äthanolamin mit 25 $cm^3$ Essigsäure vermischt und unter Kühlen wurden 14,5 g Chloral bei einer Temperatur unter 20 °C zugegeben. Die Essigsäure wurde abdestilliert und das Produkt wurde unter Vakuum destilliert. Siedepunkt: 111 bis 112 °C/19 mm Hg. Beim Stehen wurde der Stoff fest. So wurde 2-(Trichlormethyl)-oxazolidin mit einem Schmelzpunkt von 74 bis 75 °C erhalten.

Beispiel 7

Trichloracetaldehyddi-n-heptylacetal

Es wurde ein Gemisch von 5 g Chloralhydrat, 12 g n-Heptanol, 0,1 g 4-Methylbenzolsulfonsäure und 50 $cm^3$ Benzol auf der Rückflusstemperatur gehalten und das entstehende Wasser wurde mit Hilfe eines Dean-Stark-Gerätes abgetrennt. Nach einer Reaktionsdauer von 9 Stunden wurde die benzolische Lösung mit einer Natriumbicarbonatlösung und Wasser gewaschen und über Magnesiumsulfat getrocknet und dann wurde das Benzol unter Vakuum abdestilliert. So wurde Trichloracetaldehyddi-n-heptylacetal mit einem $n_D^{20}$-Wert von 1,4573 erhalten.

Beispiel 8

2-(Trichlormethyl)-1,3-dioxan

Es wurden 21,6 g Chloralhydrat mit 9,6 g n-Propan-1,3-diol [Propylenglykol] vermischt und dann wurden 16,8 $cm^3$ konzentrierte Schwefelsäure zugegeben. Das Gemisch wurde 2 Stunden lang auf 60 bis 70 °C erwärmt und dann nach dem Verdünnen mit 60 $cm^3$ Wasser 2-mal mit je 60 $cm^3$ Chloroform extrahiert. Die Chloroformphase wurde mit Wasser und einer Natriumbicarbonatlösung gewaschen und über Magnesiumsulfat getrocknet und dann wurde das Chloroform abdestilliert und das Produkt unter Vakuum destilliert. So wurde 2-(Trichlormethyl)-1,3-dioxan mit einem Siedepunkt von 98 °C/9 mm Hg erhalten.

Beispiel 9

Trichloracetaldehyddiisopentylacetal

Es wurde ein Gemisch von 5 g Chloralhydrat, 10 g Isoamylalkohol, 0,1 g 4-Methylbenzolsulfonsäure und 50 $cm^3$ Benzol auf der Rückflusstemperatur gehalten und das entstehende Wasser wurde mit Hilfe eines Dean-Stark-Gerätes abgetrennt. Nach einer Reaktionsdauer von 9 Stunden wurde die benzolische Lösung mit einer Natriumbicarbonatlösung und Wasser gewaschen und über Magnesiumsulfat getrocknet und das Benzol wurde unter Vakuum abdestilliert. So wurde Trichloracetaldehyddiisopentylacetal mit einem $n_D^{20}$-Wert von 1,4519 erhalten.

Beispiel 10

Trichloracetaldehyddimethylacetal

Es wurden 5 g Chloralhydrat, 30 $cm^3$ absolutes Methanol und 2,5 $cm^3$ konzentrierte Schwefelsäure 10 Stunden lang auf der Rückflusstemperatur gehalten und dann wurde das Reaktionsgemisch destilliert. So wurde Trichloracetaldehyddimethylacetal mit einem Siedepunkt von 175 bis 176 °C erhalten.

Beispiel 11

Trichloracetaldehyddi-n-butylacetal

Es wurden in eine mit einem Dean-Stark-Wasserabscheider versehene Reaktionsvorrichtung 7 g Chloralhydrat, 10 g n-Butylalkohol, 0,1 g 4-Methylbenzolsulfonsäure und 50 $cm^3$ Benzol eingewogen. Die Wasserentfernung wurde unter Erwärmen auf einem Wasserbad 10 Stunden lang fortgeführt. Die benzolische Lösung wurde mit Wasser und einer Natriumbicarbonatlösung gewaschen und das Benzol wurde abdestilliert. Das Produkt wurde unter Vakuum destilliert. So wurde Trichloracetaldehyddi-n-butylacetal mit einem Siedepunkt von 104 bis 105 °C/3 mm Hg erhalten.

Beispiel 12

2-(Dichlormethyl)-1,3-dioxan

Es wurden 9 g Dichloracetaldehyddiäthylacetal und 5 g Propan-1,3-diol [Propylenglykol] in Gegenwart von 0,1 g 4-Toluolsulfonsäure 4 Stunden lang auf einem Wasserbad erwärmt. Das Produkt wurde in Gegenwart von 0,3 g Kaliumcarbonat durch Vakuumdestillation gereinigt. So wurde 2-(Dichlormethyl)-1,3-dioxan mit einem Siedepunkt von 78 bis 79 °C/15 mm Hg erhalten.

Beispiel 13

2-(Trichlormethyl)-4-(hydroxymethyl)-1,3-dioxolan

Es wurde ein Gemisch von 16 g Chloral, 5 g Glycerin und 5 $cm^3$ konzentrierter Schwefelsäure 10 Stunden lang auf einem Wasserbad erwärmt und dann wurde das Reaktionsgemisch unter Vakuum destilliert. So wurde 2-(Trichlormethyl)-4-(hydroxymethyl)-dioxolan mit einem Siedepunkt von 162 bis 164 °C/25 mm Hg erhalten.

Beispiel 14

Trichloracetaldehyd-(methyl)-(n-butyl)-acetal

Es wurden zu 10 g 1-Methoxy-2,2,2-trichloräthanol 4 g n-Butylalkohol, 0,1 g 4-Methylbenzolsulfonsäure und 50 $cm^3$ Benzol zugegeben. Das Wasserabscheiden wurde in einem Dean-Stark-Gerät 10 Stunden lang unter Erwärmen auf einem Wasserbad fortgeführt. Die benzolische Lösung wurde mit Wasser und einer wässrigen Natriumbicarbonatlösung gewaschen und über Magnesiumsulfat getrocknet und das Benzol wurde abdestilliert. Das Produkt wurde durch Vakuumdestillation gereinigt. So wurde Trichloracetaldehyd-(methyl)-(n-butyl)-acetal mit einem Siedepunkt von 77 bis 78 °C/4 mm Hg erhalten.

Beispiel 15

Bromacetaldehyddiäthylacetal

Es wurde zu einem Gemisch von 10 g Vinylacetat und 20 $cm^3$ Tetrachlorkohlenstoff unter Kühlen (Temperatur $< 10$ °C) eine Lösung von 10 $cm^3$ Tetrachlorkohlenstoff und 18,5 g Brom zugegeben. Dem Gemisch wurden 60 $cm^3$ Äthanol zugesetzt und es wurde 2 Tage lang stehengelassen. Die organische Phase wurde 2-mal mit je 50 $cm^3$ Wasser gewaschen und dann unter Vakuum destilliert. So wurde Bromacetaldehyddiäthylacetal mit einem Siedepunkt von 59 bis 61 °C/19 mm Hg erhalten.

Beispiel 16

Trichloracetaldehyddi-n-hexylacetal

Es wurde ein Gemisch von 5 g Chloralhydrat, 11 g n-Hexanol, 0,1 g 4-Methylbenzolsulfonsäure und 50 $cm^3$ Benzol auf der Rückflusstemperatur gehalten und das entstehende Wasser wurde mit Hilfe eines Dean-Stark-Gerätes abgetrennt. Nach einer Reaktionsdauer von 9 Stunden wurde die benzolische Lösung mit einer Natriumbicarbonatlösung und Wasser gewaschen und über Magnesiumsulfat getrocknet und dann wurde das Benzol unter Vakuum abdestilliert. So wurde Trichloracetaldehyddi-n-hexylacetal mit einem $n_D^{20}$-Wert von 1,4562 erhalten.

Beispiel 17

Cyanacetaldehyddiäthylacetal

Es wurde ein Gemisch aus 8 g Bromacetaldehyddiäthylacetal, 3 g Natriumcyanid und 20 $cm^3$ Dimethylsulfoxyd 5 Stunden lang bei einer Temperatur von 80 °C gerührt beziehungsweise geschüttelt. Das Reaktionsgemisch wurde in 80 $cm^3$ Wasser eingegossen und dann 2-mal mit je 50 $cm^3$ Chloroform extrahiert. Der chloroformhaltige Auszug wurde 2-mal mit je 30 $cm^3$ Wasser gewaschen, über Magnesiumsulfat getrocknet und dann unter Vakuum destilliert. So wurde Cyanacetaldehyddiäthylacetal mit einem Siedepunkt von 97 bis 98 °C/10 mm Hg erhalten.

Beispiel 18

Dichloracetaldehyddi-n-propylacetal

Es wurde ein Gemisch von 5 g Dichloracetaldehyddiäthylacetal, 30 $cm^3$ n-Propanol und 0,1 g 4-Methylbenzolsulfonsäure 5 Stunden lang auf einem Wasserbad erwärmt und dann destilliert. So wurde Dichloracetaldehyddi-n-propylacetal mit einem Siedepunkt von 210 bis 211 °C erhalten.

Beispiel 19

Trichloracetaldehyddiäthylacetal

Es wurden 5 g Chloralhydrat, 40 $cm^3$ absolutes Äthanol und 2,5 $cm^3$ konzentrierte Schwefelsäure 10 Stunden lang auf der Rückflusstemperatur gehalten und dann wurde das Reaktionsgemisch destilliert. So wurde Trichloracetaldehyddiäthylacetal mit einem Siedepunkt von 190 bis 192 °C beziehungsweise 84 bis 85 °C/10 mm Hg erhalten.

Beispiel 20

2-(Dichlormethyl)-1,3-dioxolan

Es wurden 10 g Dichloracetaldehyddiäthylacetal und 3 g Äthylenglykol in Gegenwart von 0,1 g 4-Methylbenzolsulfonsäure 5 Stunden lang auf einem Wasserbad erwärmt und dann wurde das Produkt unter Vakuum destilliert. So wurde 2-(Dichlormethyl)-1,3-dioxolan mit einem Siedepunkt von 187 °C beziehungsweise 69 bis 72 °C/9 mm Hg erhalten.

Beispiel 21

2-(Trichlormethyl)-1,3-oxathiolan

Es wurden 14 g Chloral und 7,5 g 2-Mercaptoäthanol in 50 $cm^3$ Benzol miteinander vermischt, 5 Stunden lang auf 50 °C gehalten und dann kühlen gelassen. Das abgeschiedene feste Produkt wurde filtriert. So wurde 2-(Trichlormethyl)-1,3-oxathiolan mit einem Schmelzpunkt von 67 bis 68 °C erhalten.

Beispiel 22

2-(Trichlormethyl)-1,3-diphenylimidazolidin

Es wurden zu 7 g N,N'-(Diphenyl)-äthylendiamin 40 $cm^3$ Essigsäure und dann 30 g Chloral zugegeben. Das Gemisch wurde 72 Stunden lang stehengelassen und dann wurde das abgeschiedene feste Produkt abfiltriert. So wurde 2-(Trichlormethyl)-1,3-diphenylimidazolidin mit einem Schmelzpunkt von 136 bis 137 °C erhalten.

Beispiel 23

Dichloracetaldehyddi-n-dodecylacetal

Es wurde ein Gemisch von 5 g Dichloracetaldehyddiäthylacetal, 30 g n-Dodecanol und 0,1 g 4-Methylbenzolsulfonsäure 5 Stunden lang auf einem Wasserbad erwärmt und dann destilliert. So wurde Dichloracetaldehyddi-n-dodecylacetal mit einem $n_D^{20}$-Wert von 1,4783 erhalten.

Beispiel 24

[Methyl]-[(1-methoxy-2,2,2-trichlor)-äthyl]-sulfid

Es wurden 9 g 2,2,2-Trichlor-1-(methylmercapto)-äthanol bei einer Temperatur von −15 °C einer Lösung von 2 g Diazomethan in 30 $cm^3$ Äthanol zugesetzt. Das Gemisch wurde über Nacht bei Raumtemperatur stehengelassen und dann destilliert. So wurde [Methyl]-[(1-methoxy-2,2,2-trichlor)-äthyl]-sulfid mit einem Siedepunkt von 130 bis 132 °C/12 mm Hg erhalten.

Beispiel 25

Dichloracetaldehyddi-n-octylacetal

Es wurde ein Gemisch von 5 g Dichloracetaldehyddiäthylacetal, 25 g n-Octanol und 0,1 g 4-Methylbenzolsulfonsäure 5 Stunden lang auf einem Wasserbad erwärmt und dann destilliert. So wurde Dichloracetaldehyddi-n-octylacetal mit einem $n_D^{20}$-Wert von 1,4627 erhalten.

Beispiel 26

2,2,2-Trichlor-1-(n-butylthio)-äthylalkohol

Es wurde ein Gemisch von 7 g Chloral, 4 g n-Butylmercaptan und 15 $cm^3$ Benzol über Nacht bei Raumtemperatur stehengelassen und dann destilliert. So wurde 2,2,2-Trichlor-1-(n-butylthio)-äthylalkohol mit einem Schmelzpunkt von 56 bis 57 °C erhalten.

Beispiel 27

2-(Dichlormethyl)-4-(hydroxymethyl)-1,3-dioxolan

Es wurde zu einem Gemisch von 5,5 g 2-(Trichlormethyl)-4-(hydroxymethyl)-dioxolan und 30 $cm^3$ Essigsäure unter Rühren beziehungsweise Schütteln während 30 Minuten 1,6 g Zinkpulver zugegeben. Nach noch 15 Minuten langem Rühren beziehungsweise Schütteln wurde das Reaktionsgemisch destilliert. So wurde 2-(Dichlormethyl)-4-(hydroxymethyl)-dioxolan mit einem Siedepunkt von 156 bis 157 °C/19 mm Hg erhalten.

Beispiel 28

2-(Trichlormethyl)-4-(methoxymethyl)-1,3-dioxolan

Es wurde ein Gemisch von 5,5 g 2-(Trichlormethyl)-4-(hydroxymethyl)-dioxolan, 3 g Dimethylsulfat, 3 g Kaliumcarbonat und 30 $cm^3$ Wasser 4 Stunden lang bei Raumtemperatur gerührt beziehungsweise geschüttelt. Das Gemisch wurde 2-mal mit je 30 $cm^3$ Chloroform extrahiert und der Auszug wurde mit 50 $cm^3$ Wasser gewaschen und dann destilliert. So wurde 2-(Trichlormethyl)-4-(methoxymethyl)-dioxolan mit einem Siedepunkt von 153 bis 155 °C/18 mm Hg erhalten.

Beispiel 29

Dichloracetaldehydäthylhemiacetal

Es wurde ein Gemisch von 6,1 g Dichloracetaldehyd, 2,3 g Äthanol und 15 $cm^3$ Benzol über Nacht bei Raumtemperatur stehengelassen und dann destilliert. So wurde Dichloracetaldehydäthylhemiacetal mit einem Siedepunkt von 109 bis 110 °C erhalten.

Beispiel 30

2,2,2-Trichlor-1-[2'-(äthoxy)-äthoxy]-äthanol

Es wurde ein Gemisch von 7 g Chloral, 4,8 g Äthylenglykolmonoäthyläther und 15 $cm^3$ Benzol über Nacht bei Raumtemperatur stehengelassen und dann destilliert. So wurde 2,2,2-Trichlor-1-[2'-(äthoxy)-äthoxy]-äthanol mit einem $n_D^{20}$-Wert von 1,4697 erhalten.

Beispiel 31

2,2,2-Trichlor-1-(allyloxy)-äthanol

Es wurde ein Gemisch von 7 g Chloral, 2,9 g Allylalkohol und 15 $cm^3$ Benzol über Nacht bei Raumtemperatur stehengelassen und dann destilliert. So wurde 2,2,2-Trichlor-1-(allyloxy)-äthanol mit einem Schmelzpunkt von 116 °C erhalten.

Beispiel 32

Chloracetaldehyddimethylacetal

Es wurde in ein mit Aceton und Trockeneis gekühltes Gemisch von 5 g Vinylacetat und 20 $cm^3$ Methanol 5 g trockenes Chlorgas eingeleitet und dann wurde das Gemisch über Nacht bei Raumtemperatur stehengelassen. Das Reaktionsgemisch wurde dann in 50 g Eiswasser eingegossen. Das Produkt wurde mit 30 $cm^3$ Chloroform extrahiert und der Auszug wurde mit einer Natriumbicarbonatlösung und Wasser gewaschen und dann über Magnesiumsulfat getrocknet und destilliert. So wurde Chloracetaldehyddimethylacetal mit einem Siedepunkt von 53 bis 54 °C/16 mm Hg erhalten.

Beispiel 33
α-Chloralose

Es wurde ein Gemisch von 4,5 g Glucose, 19 g Chloral, 20 $cm^3$ Chloroform und 0,05 g 4-Methylbenzolsulfonsäure 7 Stunden lang auf der Rückflusstemperatur gehalten und das entstehende Wasser wurde mit einem Dean-Stark-Gerät abgetrennt. Das Lösungsmittel wurde abdestilliert und der Rückstand wurde 20 Minuten lang auf einer Temperatur von 60 °C gehalten. Der abgeschiedene Stoff wurde abfiltriert und der pH-Wert der Mutterlauge wurde mit Natriumhydroxyd auf 6,5 eingestellt. Der abgeschiedene Niederschlag wurde abfiltriert. So wurde α-Chloralose mit einem Schmelzpunkt von 182 bis 183 °C erhalten.

Beispiel 34
β-Chloralose

Es wurde einem Gemisch von 4,5 g Glucose und 19 g Chloral unter Rühren beziehungsweise Schütteln 0,05 g 4-Methylbenzolsulfonsäure und 20 $cm^3$ Chloroform zugesetzt. Das Gemisch wurde auf die Rückflusstemperatur erwärmt und das entstehende Wasser wurde mit einem Dean-Stark-Gerät abgetrennt. Das Lösungsmittel wurde abdestilliert und dann 20 Minuten lang auf einer Temperatur von 60 °C gehalten. Der abgeschiedene Stoff wurde abfiltriert. So wurde β-Chloralose mit einem Schmelzpunkt von 231 bis 232 °C erhalten.

Beispiel 35
2-(Dibrommethyl)-1,3-dioxolan

Es wurde ein Gemisch von 5,3 g Dibromacetaldehyddiäthylacetal, 7 g Äthylenglykol und 0,05 g 4-Methylbenzolsulfonsäure 7 Stunden lang auf einem Wasserbad erwärmt und dann unter Vakuum destilliert. So wurde 2-(Dibrommethyl)-dioxolan mit einem Siedepunkt von 102 bis 103 °C/9 mm Hg erhalten.

Beispiel 36
2-(Dichlormethyl)-2-methyl-1,3-dioxolan

Es wurde ein Gemisch von 4,5 g 1,1-Dichloraceton, 3,7 $cm^3$ Äthylenglykol, 0,1 g 4-Methylbenzolsulfonsäure und 30 $cm^3$ Benzol 9 Stunden lang auf der Rückflusstemperatur gehalten, wobei ständig Wasser abgetrennt wurde. Das Reaktionsgemisch wurde mit einer Natriumbicarbonatlösung gewaschen, über Magnesiumsulfat getrocknet und destilliert. So wurde 2-(Dichlormethyl)-2-methyl-1,3-dioxolan mit einem Siedepunkt von 92 bis 94 °C/27 mm Hg erhalten.

Beispiel 37
2-(Chlormethyl)-1,3-dioxan

Es wurde ein Gemisch von 4,7 g Chloracetaldehyddiäthylacetal, 9 g Propylenglykol und 0,05 g 4-Methylbenzolsulfonsäure 9 Stunden lang auf einem Wasserbad erwärmt und dann unter Vakuum destilliert. So wurde 2-(Chlormethyl)-1,3-dioxan mit einem Siedepunkt von 71 bis 74 °C/14 mm Hg erhalten.

Beispiel 38
2-(Dichlormethyl)-2-phenyl-1,3-dioxolan

Es wurde ein Gemisch von 6 g 1,1-Dichloracetophenon, 3,8 $cm^3$ Äthylenglykol, 0,1 g 4-Methylbenzolsulfonsäure und 40 $cm^3$ Benzol 11 Stunden lang auf der Rückflusstemperatur gehalten und das entstehende Wasser wurde mit Hilfe eines Dean-Stark-Gerätes laufend abgetrennt. Das Reaktionsgemisch wurde mit einer Natriumbicarbonatlösung gewaschen, über Magnesiumsulfat getrocknet und eingedampft. Der erhaltene feste Stoff wurde aus n-Hexan umkristallisiert. So wurde 2-(Dichlormethyl)-2-phenyl-1,3-dioxolan mit einem Schmelzpunkt von 59 bis 60 °C erhalten.

Beispiel 39
2-(Brommethyl)-1,3-dioxolan

Es wurden 4,3 g Bromacetaldehyddiäthylacetal in Gegenwart von 10 g Äthylenglykol und 0,05 g 4-Methylbenzolsulfonsäure 8 Stunden lang auf einem Wasserbad erwärmt und dann wurde das Reaktionsgemisch destilliert. So wurde 2-(Brommethyl)-1,3-dioxolan mit einem Siedepunkt von 173 bis 174 °C erhalten.

Beispiel 40
Bromacetaldehyddiallylacetal

Es wurden 80 $cm^3$ einer mit salzhaltigem Eis gekühlten Lösung von 43 g Vinylacetat in Tetrachlorkohlenstoff 60 $cm^3$ einer Lösung von 80 g Brom in Tetrachlorkohlenstoff so zugetropft, dass die Temperatur des Reaktionsgemisches + 10 °C nicht überstieg. Dann wurde das Reaktionsgemisch 3,8 Mol mit Eis gekühltem Allylalkohol bei einer Temperatur unter + 10 °C zugetropft. Nach 2 Tage langem Stehen wurde die Tetrachlorkohlenstoffphase mit Wasser gewaschen und über wasserfreiem Natriumsulfat getrocknet und nach dem Eindampfen des Lösungsmittels wurde der so gewonnene Rückstand fraktioniert destilliert. So wurde Bromacetaldehyddiallylacetal mit einem Siedepunkt von 80 bis 82 °C/1 mm Hg beziehungsweise 61 °C/0,5 mm Hg in einer Ausbeute von 72,1% der Theorie erhalten.

Beispiel 41
2-(Dichlormethyl)-2-methyl-1,3-dioxolan

Es wurden 8,0 g 1,1-Dichloraceton mit 6,7 $cm^3$ Äthylenglykol in Gegenwart von p-Toluolsulfonsäure als Katalysator in einem Wasserabscheidesystem oder unter Verwendung eines Molekularsiebes (Å = 4) als wasserbindenden Mittels umgesetzt und dann in einer Vigreux-Kolonne destilliert. So wurde 2-(Dichlormethyl)-2-methyl-1,3-dioxolan mit einem Siedepunkt von 93 bis 94 °C/2 mm Hg und einem $n_D^{20}$-Wert von 1,4671 erhalten.

Das als Ausgangsstoff verwendete 1,1-Di(chlor)-aceton ist wie folgt beschrieben hergestellt worden.

Es wurden 14,5 g Aceton, die in einen mit einem Tropftrichter, einem Rührer und einem mit einem Calciumchloridrohr verbundenen Rückflusskühler versehenen Rundkolben eingewogen wurden, unter Kühlen 67,5 g Sulfurylchlorid mit einer solchen Geschwindigkeit zugetropft, dass die Temperatur des Systemes 30 bis 40 °C nicht überstieg. Nach einer Nachreaktion von 3 bis 5 Stunden wurde das überschüssige Reagens durch Destillation entfernt. So wurde 1,1-Di-(chlor)-aceton mit einem Siedepunkt von 117 bis 118 °C erhalten.

Beispiel 42

2-(Dichlormethyl)-3-phenyl-1,3-oxazolidin

Es wurden in einem mit einem Wasserabscheideaufsatz versehenen Kolben 6,85 g N-(Phenyl)-äthanolamin in einem benzolhaltigen Medium mit 5,6 g Dichloracetaldehyd bis zum Abscheiden der theoretischen Wassermenge umgesetzt. Zum Binden des Wassers konnte auch ein Molekularsieb verwendet werden. Das Benzol wurde mit einem sich drehenden Vakuumverdampfer entfernt und der Rückstand wurde mittels Säulenchromatographie oder Destillation gereinigt. Im Falle der Säulenchromatographie wurde das Reaktionsgemisch an einer Säulenfüllung von Kieselgel (0,2 bis 0,5 mm) mit einem Gemisch von Benzol und Äthylacetat im Volumverhältnis von 12:5 eluiert. So wurde 2-(Dichlormethyl)-3-phenyl-1,3-oxazolidin mit einem Siedepunkt von 132 °C/0,4 mm Hg und einem $n_D^{20}$-Wert von 1,5890 erhalten.

Das als Ausgangsverbindung verwendete N-(Phenyl)-äthanolamin ist wie folgt beschrieben hergestellt worden:

Es wurden 4,39 g Anilin mit 3,2 $cm^3$ Äthylenchlorhydrin 3 bis 4 Stunden lang in Gegenwart von 4,61 g Natriumcarbonat als säurebindendem Mittel beim Siedepunkt von Äthylenchlorhydrin umgesetzt. Der Niederschlag wurde abfiltriert und mit Benzol gewaschen und nach dem Eindampfen des Lösungsmittels wurde der Rückstand unter Vakuum destilliert. So wurde N-(Phenyl)-äthanolamin mit einem Siedepunkt von 182 bis 188 °C/30 mm Hg erhalten.

Beispiel 43

2-(Dichlormethyl)-3-allyl-1,3-oxazolidin

Es wurde wie im Beispiel 42 beschrieben vorgegangen, jedoch mit N-(Allyl)-äthanolamin statt des N-(Phenyl)-äthanolamines als Ausgangsverbindung und unter Verwendung eines Molekularsiebes als wasserbindenden Mittels. So wurde 2-(Dichlormethyl)-3-allyl-1,3-oxazolidin mit einem Siedepunkt von 82 bis 84 °C/0,6 mm Hg erhalten.

Beispiel 44

2-Brompropionaldehyddiäthylacetal

Es wurden 13,22 g Propionaldehyddiäthylacetal in 15 $cm^3$ Tetrachlorkohlenstoff gelöst. Unter kräftigem Rühren beziehungsweise Schütteln wurden bei −10 °C 20 $cm^3$ einer Lösung von 17,58 g Brom in Tetrachlorkohlenstoff zugetropft. Nach dem Erwärmen auf Raumtemperatur wurde das Gemisch unter Vakuum destilliert. So wurde 2-Brompropionaldehyddiäthylacetal mit einem Siedepunkt von 79 bis 80 °C/20 mm Hg erhalten.

Beispiel 45

2,2,2-Trichlor-1-(methylmercapto)-äthanol

Es wurden zu einem auf −10 °C gekühlten Gemisch von 30 $cm^3$ Diäthyläther und 4 g Methylmercaptan 13 g Chloral zugetropft. Der feste Rückstand wurde nach dem Entfernen des Äthers aus Petroläther umkristallisiert. So wurde 2,2,2-Trichlor-1-(methylmercapto)-äthanol mit einem Schmelzpunkt von 55 bis 56 °C erhalten.

Beispiel 46

2-(Dichlormethyl)-4-(acetoxymethyl)-1,3-dioxolan

Es wurden 5 g wie im Beispiel 13 beschrieben erhaltenes 2-(Dichlormethyl)-4-(hydroxymethyl)-dioxolan, 20 $cm^3$ Essigsäureanhydrid und 1 $cm^3$ konzentrierte Schwefelsäure 1 Stunde lang auf einem Wasserbad erhitzt und dann unter Vakuum destilliert. So wurde 2-(Dichlormethyl)-4-(acetoxymethyl)-1,3-dioxolan mit einem Siedepunkt von 181 bis 183 °C/19 mm Hg erhalten.

Nach den in den obigen Beispielen ausführlich beschriebenen Verfahrensweisen wurden aus entsprechenden Ausgangsstoffen zahlreiche weitere Verbindungen der allgemeinen Formel I, die in den erfindungsgemässen Erzeugnissen als Antidota gut wirksam sind, hergestellt. Diese Verbindungen und die von obigen Beispielen sind unter Angabe der Symbole X, Y, $R_1$, $R_2$, $R_3$ und $R_4$ von der allgemeinen Formel I sowie von Kenndaten in der folgenden Tabelle I zusammengestellt. Dabei ist jeder Verbindung eine laufende Nummer zugeordnet, die auch im danach folgenden Teil der Beschreibung verwendet wird.

Die Wirkung der in den erfindungsgemässen Erzeugnissen enthaltenen Antidota beziehungsweise erfindungsgemässen Verbindungen wurde durch eine Samenbehandlungsverfahrensweise und eine Bodenbehandlungsverfahrensweise wie folgt untersucht. Bei beiden Verfahrensweisen wurden in den in Kunststoffgefässe mit Abmessungen von 10 cm × 10 cm × 10 cm gefüllten, je nach den genannten 2 Arten der Behandlung vorbereiteten Boden jeweils 6 Nutzpflanzensamen und jeweils 15 Unkrautsamen eingebracht und die Wirksamkeit der Antidota wurde bei mehreren Herbiziden beziehungsweise Nutz- und Unkrautpflanzen untersucht, wobei die Luftfeuchtigkeit, das Licht und die Temperatur geregelt waren.

In jedem der Samenbehandlungs- beziehungsweise Bodenbehandlungsversuche wurde der Herbizidwirkstoff einerseits allein und andererseits in Kombination mit einem Antidotum der erfindungsgemässen Erzeugnisse beziehungsweise einer erfindungsgemässen Verbindung sowie zur Feststellung der Phytotoxizität das jeweilige Antidotum beziehungsweise die jeweilige erfindungsgemässe Verbindung allein eingesetzt.

A) Untersuchungen durch Samenbehandlung

Es wurden 10 g Samen mit 50 mg des jeweiligen Antidotums der erfindungsgemässen Erzeugnisse

Tabelle I

| Verbindung Laufende Nummer der Verbindung | Beispiel Nr. | Symbole –X– | –Y– | $R_1$– | $R_2$– | $R_3$– | $R_4$– | Kenndaten Siedepunkt | Schmelzpunkt | Brechungsindex |
|---|---|---|---|---|---|---|---|---|---|---|
| 1 | | –O– | –O– | Dichlormethyl | H– | n–Pentyl | n–Pentyl | 98 bis 100°C/ 30 mm Hg | | |
| 2 | | –O– | –O– | Dichlormethyl | H– | Cyclopentyl | Cyclopentyl | 105 bis 110°C/ 1 mm Hg | | |
| 3 | 41 | –O– | –O– | Dichlormethyl | Methyl | $-\underset{H_2}{C}-\underset{H_2}{C}-$ | | 93 bis 94°C/ 27 mm Hg | | $n_D^{25}$ = 1,4671 |
| 4 | | –O– | –O– | Dichlormethyl | H– | 2-Bromäthyl | 2-Bromäthyl | 129 bis 130°C/ 5 mm Hg | | |
| 5 | | –O– | –O– | Dibrommethyl | H– | Äthyl | Äthyl | 195 bis 200°C | | |
| 6 | | –O– | –O– | Dichlormethyl | H– | n-Hexyl | n-Hexyl | 120 bis 122°C/ 38 mm Hg | | |
| 7 | | –O– | –O– | Dichlormethyl | H– | 2-[2'-(Methoxy)-äthoxy]-äthyl | 2-[2'-(Methoxy)-äthoxy]-äthyl | | | $n_D^{25}$ = 1,4427 |
| 8 | 23 | –O– | –O– | Dichlormethyl | H– | n-Dodecyl | n-Dodecyl | | | $n_D^{20}$ = 1,4783 |
| 9 | | –O– | –O– | 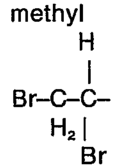 | H– | Äthyl | Äthyl | 107 bis 108°C/ 11 mm Hg | | |
| 10 | | –O– | –O– | Dichlormethyl | H– | Isopropyl | Isopropyl | 212 bis 214°C | | |
| 11 | | –O– | –O– | Furyl | H– | Äthyl | Äthyl | 189 bis 191°C | | |
| 12 | | –O– | –O– | Dichlormethyl | H– | 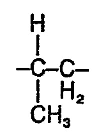 | | 90 bis 93°C/ 10 mm Hg | | |
| 13 | | –O– | –O– | Dichlormethyl | H– | 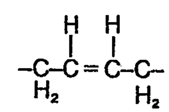 | | 110°C/10 mm Hg | | |
| 14 | | –O– | –O– | 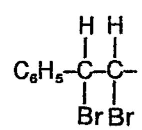 | H– | Äthyl | Äthyl | 152 bis 154°C/ 12 mm Hg | | |

Fortsetzung der Tabelle I

| Verbindung Laufende Nummer der Verbindung | Beispiel Nr. | Symbole –X– | –Y– | $R_1$– | $R_2$– | $R_3$– | $R_4$– | Kenndaten Siedepunkt | Schmelzpunkt | Brechungsindex |
|---|---|---|---|---|---|---|---|---|---|---|
| 15 | | –O– | N–n-Propyl | Dichlormethyl | H– | $-\underset{H_2}{C}-\underset{H_2}{C}-$ | | 126 bis 127°C/ 0,4 mm Hg | | |
| 16 | | –O– | –O– | Trichlormethyl | H– | Methyl | Äthyl | 193 bis 194°C | | |
| 17 | | –O– | –O– | Dichlormethyl | H– | 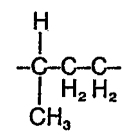 | | 82 bis 84°C/ 15 mm Hg | | |
| 18 | 5 | –O– | –O– | Dichlormethyl | H– | Methyl | Methyl | 167 bis 168°C | | |
| 19 | | –O– | –O– | Dichlormethyl | H– | Benzyl | Benzyl | 176 bis 178°C/ 5 mm Hg | | |
| 20 | 17 | –O– | –O– | Cyanmethyl | H– | Äthyl | Äthyl | 97 bis 98°C/ 10 mm Hg | | |
| 21 | | –O– | –O– | Dichlormethyl | H– | Isobutyl | Isobutyl | 88 bis 90°C/ 30 mm Hg | | |
| 22 | | –O– | –O– | Dichlormethyl | H– | 2-(Methyl)-n-butyl | 2-(Methyl)-n-butyl | 137 bis 139°C/ 38 mm Hg | | |
| 23 | | –O– | –O– | Dichlormethyl | H– | 2-(Methoxy)-äthyl | 2-(Methoxy)-äthyl | 126 bis 128°C/ 28 mm Hg | | |
| 24 | | –O– | –O– | 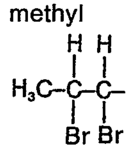 | H– | Äthyl | Äthyl | 113 bis 114°C/ 13 mm Hg | | |
| 25 | 20 | –O– | –O– | Dichlormethyl | H– | $-\underset{H_2}{C}-\underset{H_2}{C}-$ | | 69 bis 72°C/ 9 mm Hg | | |
| 26 | | –O– | –O– | Dichlormethyl | H– | Crotyl | Crotyl | | | $n_D^{20}$ = 1,4981 |
| 27 | | –O– | 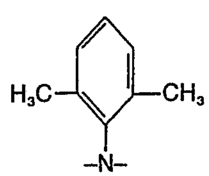 | Dichlormethyl | H– | $-\underset{H_2}{C}-\underset{H_2}{C}-$ | | | | $n_D^{20}$ = 1,5640 |
| 28 | | –O– | –O– | Vinyl | H– | Äthyl | Äthyl | 89 bis 90°C | | |

| | | | | | | | | | |
|---|---|---|---|---|---|---|---|---|---|
| 29 | | -O- | 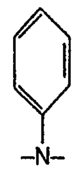 | Dichlor-methyl | H- | 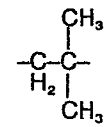 | | 138 bis 140°C/ 1 mm Hg | |
| 30 | | -O- | -O- | 4-Methyl-phenyl | H- | Äthyl | Äthyl | 116 bis 119°C/ 12 mm Hg | |
| 31 | | -O- | -O- | Dichlor-methyl | H- | 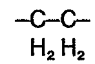 | | 106 bis 108°C/ 7 mm Hg | |
| 32 | | -O- | -O- | Dichlor-methyl | H- | Allyl | Allyl | 61 bis 63°C/ 1,5 mm Hg | |
| 33 | 29 | -O- | -O- | Dichlor-methyl | H- | H- | Äthyl | 109 bis 110°C | |
| 34 | | -O- | -O- | Dichlor-methyl | H- | 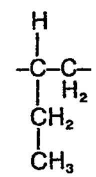 | | 63 bis 65°C/ 2 mm Hg | |
| 35 | | -O- | -O- | Dichlor-methyl | H- | sek.-Isobutyl | sek.-Isobutyl | 84 bis 86°C/ 30 mm Hg | |
| 36 | | -O- | -O- | Dichlor-methyl | H- | Progargyl | Propargyl | | $n_D^{20}$ = 1,4860 |
| 37 | 15 | -O- | -O- | Brommethyl | H- | Äthyl | Äthyl | 59 bis 61°C/ 19 mm Hg | |
| 38 | | -O- | -O- | Dichlor-methyl | H- | Furfuryl | Furfuryl | | $n_D^{20}$ = 1,5372 |
| 39 | 19 | -O- | -O- | Trichlor-methyl | H- | Äthyl | Äthyl | 84 bis 85°C/ 10 mm Hg | |
| 40 | | -O- | -O- | Dichlor-methyl | H- | n-Butyl | n-Butyl | 57 bis 60°C/ 1 mm Hg | |
| 41 | | -O- | -O- | Dichlor-methyl | H- | 1-(Methyl)-n-heptyl | 1-(Methyl)-n-heptyl | | $n_D^{20}$ = 1,4621 |
| 42 | | -O- | -O- | Dichlor-methyl | H- | 2-Chloräthyl | 2-Chloräthyl | | $n_D^{20}$ = 1,4795 |
| 43 | | -O- | 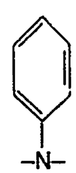 | Dichlor-methyl | H- | 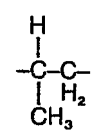 | | 148°C/1 mm Hg | |

Fortsetzung der Tabelle I

| Verbindung Laufende Nummer der Verbindung | Beispiel Nr. | Symbole –X– | –Y– | $R_1$– | $R_2$– | $R_3$– | $R_4$– | Kenndaten Siedepunkt | Schmelzpunkt | Brechungsindex |
|---|---|---|---|---|---|---|---|---|---|---|
| 44 | | 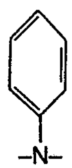 | 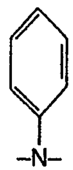 | Dichlormethyl | H– | $-\underset{H_2}{C}-\underset{H_2}{C}-$ | | 161 bis 163°C/ 10 mm Hg | | |
| 45 | 42 | –O– | 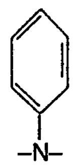 | Dichlormethyl | H– | $-\underset{H_2}{C}-\underset{H_2}{C}-$ | | 132°C/ 0,4 mm Hg | | $n_D^{20}$ = 1,5890 |
| 46 | | –O– | –O– | Dichlormethyl | H– | Dimethylaminoäthyl | Diäthylaminoäthyl | | | $n_D^{20}$ = 1,4996 |
| 47 | | –O– | –O– | 4-Methoxyphenyl | H– | Äthyl | Äthyl | 263 bis 264°C | | |
| 48 | | 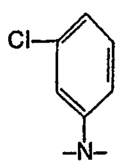 | –O– | Dichlormethyl | H– | $-\underset{H_2}{C}-\underset{H_2}{C}-$ | | | | $n_D^{20}$ = 1,5970 |
| 49 | 38 | –O– | –O– | Dichlormethyl | Phenyl | $-\underset{H_2}{C}-\underset{H_2}{C}-$ | | 115 bis 120°C/ 0,05 mm Hg | 59 bis 61°C | |
| 50 | | –O– | –O– | $H_3C-\overset{H}{C}=\overset{H}{C}-$ | H– | Äthyl | Äthyl | 48 bis 49°C/ 21 mm Hg | | |
| 51 | 1 | –O– | –O– | Dichlormethyl | H– | Äthyl | Äthyl | 107 bis 108°C/ 38 mm Hg | | |
| 52 | | –O– | –O– | Dichlormethyl | H– | 2-[2'-(Äthoxy)-äthoxy]-äthyl | 2-[2'-(Äthoxy)-äthoxy]-äthyl | | | $n_D^{20}$ = 1,4953 |
| 53 | 40 | –O– | –O– | Brommethyl | H– | Allyl | Allyl | 61°C/0,5 mm Hg | | |
| 54 | | –O– | –O– | Dichlormethyl | H– | 2-(Äthoxy)-äthyl | 2-(Äthoxy)-äthyl | | | $n_D^{20}$ = 1,4752 |
| 55 | 18 | –O– | –O– | Dichlormethyl | H– | n-Propyl | n-Propyl | 210 bis 211°C | | |

| | | | | | | | | | | |
|---|---|---|---|---|---|---|---|---|---|---|
| 56 | | –O– | –O– | Dichlor-methyl | H– | n-Octadecyl | n-Octadecyl | 210°C/5 mm Hg | | |
| 57 | | –O– | –O– | Dichlor-methyl | H– | 3-(Methyl)-n-butyl | 3-(Methyl)-n-butyl | 62°C/1 mm Hg | | |
| 58 | | –O– | –O– | Dichlor-methyl | H– | Cyclohexyl | Cyclohexyl | 120°C/1 mm Hg | | |
| 59 | 43 | –O– | $CH_2{=}C(H){-}CH_2{-}N<$ | Dichlor-methyl | H– | $-CH_2-CH_2-$ | | 82 bis 84°C/ 0,6 mm Hg | | |
| 60 | | –O– | –O– | Trichlor-methyl | H– | H– | Äthyl | 183,2°C | | |
| 61 | | –O– | –O– | Dichlor-methyl | H– | Cinnamyl | Cinnamyl | | | $n_D^{20}$ = 1,6492 |
| 62 | 12 | –O– | –O– | Dichlor-methyl | H– | $-CH_2-CH_2-CH_2-$ | | 78 bis 79°C/ 15 mm Hg | | |
| 63 | | $CH_2{=}C(H){-}CH_2{-}N<$ | $CH_2{=}C(H){-}CH_2{-}N<$ | Dichlor-methyl | H– | $-CH_2-CH_2-CH_2-$ | | 152 bis 156°C/ 0,4 mm Hg | | |
| 64 | | –O– | –O– | Dichlor-methyl | H– | Diäthylami-noäthyl | Diäthylami-noäthyl | | | $n_D^{20}$ = 1,5112 |
| 65 | | –O– | –O– | Dichlor-methyl | H– | 1-(Methyl)-n-butyl | 1-(Methyl)-n-butyl | 58°C/1 mm Hg | | |
| 66 | | –O– | –O– | Dichlor-methyl | H– | $-CH_2-CH_2-CH_2-CH_2-$ | | | | $n_D^{20}$ = 1,4850 |
| 67 | | –O– | –O– | Dichlor-methyl | H– | 2-Cyanäthyl | 2-Cyanäthyl | | | $n_D^{20}$ = 1,4749 |
| 68 | 25 | –O– | –O– | Dichlor-methyl | H– | n-Octyl | n-Octyl | | | $n_D^{20}$ = 1,4627 |
| 69 | | –N= | $H{-}N<$ | zweite Bindung | Dichlor-methyl | Phen-1,2-ylen | | | 159°C | |
| 70 | | –O– | –O– | Trichlor-methyl | H– | $-CH_2-CH_2-$ | | 198 bis 200°C/ 740 mm Hg | | |
| 71 | 27 | –O– | –O– | Dichlor-methyl | H– | $-CH_2-CH(CH_2OH)-$ | | 156 bis 157°C/ 19 mm Hg | | |

Fortsetzung der Tabelle I

| Verbindung Laufende Nummer der Verbindung | Beispiel Nr. | Symbole –X– | –Y– | $R_1$– | $R_2$– | $R_3$– | $R_4$– | Kenndaten Siedepunkt | Schmelz-punkt | Brechungs-index |
|---|---|---|---|---|---|---|---|---|---|---|
| 72 | 35 | –O– | –O– | Dibrom-methyl | H– | $-\underset{H_2}{C}-\underset{H_2}{C}-$ | | 102 bis 103°C/ 9 mm Hg | | |
| 73 | 7 | –O– | –O– | Trichlor-methyl | H– | n-Heptyl | n-Heptyl | | | $n_D^{20}$ = 1,4573 |
| 74 | | –O– | –O– | Dichor-methyl | H– | $-\underset{H_2}{C}-\overset{H}{C}=\overset{H}{C}-\underset{H_2}{C}-$ | | 110°C/ 10 mm Hg | | |
| 75 | 33 | –O– | –O– | Trichlor-methyl | H– | α-D-Glucofuranosylen | | 182 bis 183°C | | |
| 76 | 11 | –O– | –O– | Trichlor-methyl | H– | n-Butyl | n-Butyl | 104 bis 105°C/ 3 mm Hg | | |
| 77 | | –O– | –O– | Brommethyl | H– | Äthyl | 1-(Methyl)-äthyl | 110 bis 115°C/ 12 mm Hg | | |
| 78 | 22 | 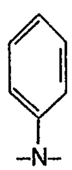 | 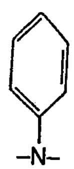 | Trichlor-methyl | H– | $-\underset{H_2}{C}-\underset{H_2}{C}-$ | | | 136 bis 137°C | |
| 79 | 31 | –O– | –O– | Trichlor-methyl | H– | H– | Allyl | | 116°C | |
| 80 | 39 | –O– | –O– | Brommethyl | H– | $-\underset{H_2}{C}-\underset{H_2}{C}-$ | | 173 bis 174°C | | |
| 81 | 4 | –O– | –O– | Trichlor-methyl | H– | n-Pentyl | n-Pentyl | | | $n_D^{20}$ = 1,4549 |
| 82 | 34 | –O– | –O– | Trichlor-methyl | H– | β-D-Glucofuranosylen | | | 231 bis 232°C | |
| 83 | 14 | –O– | –O– | Trichlor-methyl | H– | Methyl | n-Butyl | 77 bis 78°C/ 4 mm Hg | | |
| 84 | | –O– | –O– | Chlormethyl | H– | $-\underset{H_2}{C}-\underset{H_2}{C}-$ | | 154 bis 155°C/ 700 mm Hg | | |
| 85 | 8 | –O– | –O– | Trichlor-methyl | H– | $-\underset{H_2}{C}-\underset{H_2}{C}-\underset{H_2}{C}-$ | | 98°C/9 mm Hg | | |
| 86 | 16 | –O– | –O– | Trichlor-methyl | H– | n-Hexyl | n-Hexyl | | | $n_D^{20}$ = 1,4562 |
| 87 | 45 | –S– | –O– | Trichlor-methyl | H– | Methyl | H– | | 55 bis 56°C | |

| | | | | | | | | | | |
|---|---|---|---|---|---|---|---|---|---|---|
| 88 | 32 | –O– | –O– | Chlormethyl | H– | Methyl | Methyl | 53 bis 54°C/ 16 mm Hg | | |
| 89 | 6 | 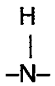 | –O– | Trichlor-methyl | H– | 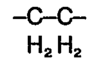 | | | 74 bis 75°C | |
| 90 | 46 | –O– | –O– | Dichlor-methyl | H– | 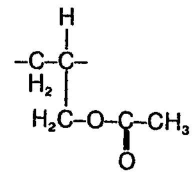 | | 181 bis 183°C/ 19 mm Hg | | |
| 91 | 26 | –S– | –O– | Trichlor-methyl | H– | n-Butyl | H | | 56 bis 57°C | |
| 92 | 28 | –O– | –O– | Trichlor-methyl | H– | 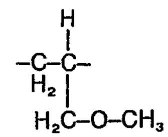 | | 153 bis 155°C/ 18 mm Hg | | |
| 93 | 10 | –O– | –O– | Trichlor-methyl | H– | Methyl | Methyl | 175 bis 176°C | | |
| 94 | | –O– | –O– | Brommethyl | H– | Isopropyl | Isopropyl | 53 bis 55°C/ 3 mm Hg | | |
| 95 | 21 | –O– | –S– | Trichlor-methyl | H– | 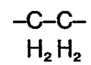 | | | 67 bis 68°C | |
| 96 | 9 | –O– | –O– | Trichlor-methyl | H– | Isopentyl | Isopentyl | | | $n_D^{20}$ = 1,4519 |
| 97 | 13 | –O– | –O– | Trichlor-methyl | H– | 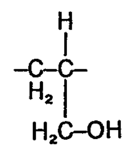 | | 162 bis 164°C/ 2 mm Hg | | |
| 98 | | –O– | –O– | Dichlor-methyl | H– | 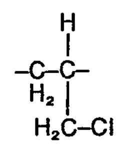 | | 66°C/2 mm Hg | | |

in einem gut schliessenden Schliffgefäss geschüttelt, bis die Samen vom Antidotum gleichmässig bedeckt waren. Das Aufbringen der Antidota auf die Samen konnte gegebenenfalls durch Zugabe von Aceton gefördert werden. Die so vorbehandelten Samen wurden in den in den obigen genannten Versuchsgefässen befindlichen Boden eingebracht, in den zuvor der beziehungsweise die Herbizidwirkstoff(e) gleichmässig in der Weise eingemischt worden war(en), dass in einer geeigneten Mischeinrichtung dem Boden von einer entsprechend eingestellten Stammlösung der beziehungsweise des Herbizide[s] eine bestimmte Menge des beziehungsweise der Herbizidwirkstoffe[s] zugesetzt wurde. Bei Blindversuchen blieb wie bereits erwähnt das Antidotum oder der Herbizidwirkstoff weg.

Die Samen wurden 2,5 cm tief in den vorbereiteten Boden gelegt und die Gefässe wurden so gegossen, dass das günstige Pflanzenwachstum entsprechend sichergestellt war.

Die Ergebnisse sind in der folgenden Tabelle II zusammengestellt.

Tabelle II
Schädigung von Mais durch Herbizide (in %) bei Samen, die mit 0,5 Gew.-% Antidotum behandelt oder nicht behandelt wurden

| Erzeugnis | | | | Schädigung der Pflanzen nach 3 Wochen in % | |
|---|---|---|---|---|---|
| Antidotum Laufende Nr. von der Tabelle I | Beispiel Nr. | Herbizid Bezeichnung | Menge in kg/ha | bei Behandlung mit einem Antidotum | ohne Behandlung mit einem Antidotum |
| 19 | | S-Äthyl-N,N-diisobutylthiocarbamat | 8,1 | 5 | 48 |
| 8 | 23 | S-n-Propyl-N-n-butyl-N-äthylthiocarbamat | 5,7 | 5 | 47 |
| 14 | | S-Äthyl-N,N-di-n-propylthiocarbamat | 6,6 | 13 | 52 |
| 26 | | S-Äthyl-N,N-di-n-propylthiocarbamat | 6,6 | 18 | 52 |
| 24 | | O-{4-(Chlor)-n-but-2-inyl}-N-(3-chlorphenyl)-carbamat | 0,7 | 0 | 41 |
| 68 | 25 | S-Äthyl-N,N-di-n-propylthiocarbamat | 6,6 | 3 | 54 |
| 7 | | S-Äthyl-N,N-di-n-propylthiocarbamat | 6,6 | 12 | 43 |
| 22 | | S-Äthyl-N,N-hexamethylenthiocarbamat | 5,3 | 0 | 52 |
| 51 | 1 | S-Äthyl-N,N-di-n-propyl-thiocarbamat | 6,6 | 0 | 38 |
| 35 | | S-Äthyl-N,N-di-n-protylthiocarbamat | 6,6 | 2 | 41 |
| 2 | | S-n-Propyl-N,N-di-n-propylthiocarbamat | 2,8 | 10 | 61 |
| 56 | | S-Äthyl-N,N-di-n-propylthiocarbamat | 6,6 | 5 | 47 |
| 11 | | S-Äthyl-N,N-di-n-propylthiocarbamat | 6,6 | 38 | 47 |
| 67 | | S-Äthyl-N,N-di-n-propylthiocarbamat | 6,6 | 0 | 41 |
| 41 | | S-(2,3-Dichlorallyl)-N,N-diisopropyl-thio-carbamat | 5,7 | 13 | 48 |
| 3 | 41 | 2′,6′-Diäthyl-N-(methoxymethyl)-2-chloracetanilid | 4,5 | 17 | 51 |
| 58 | | S-Äthyl-N-äthyl-N-cyclohexylthiocarbamat | 6,3 | 28 | 53 |
| 64 | | S-Äthyl-N,N-di-n-propylthiocarbamat | 6,6 | 0 | 38 |

Fortsetzung der Tabelle II
Schädigung von Mais durch Herbizide (in %) bei Samen, die mit 0,5 Gew.-% Antidotum behandelt oder nicht behandelt wurden

| Erzeugnis | | | | Schädigung der Pflanzen nach 3 Wochen in % | |
|---|---|---|---|---|---|
| Antidotum Laufende Nr. von der Tabelle I | Beispiel Nr. | Herbizid Bezeichnung | Menge in kg/ha | bei Behandlung mit einem Antidotum | ohne Behandlung mit einem Antidotum |
| 29 | | S-Äthyl-N,N-di-n-propylthiocarbamat | 6,6 | 0 | 42 |
| 59 | 43 | S-(4-Chlorbenzyl)-N,N-diäthylthioharnstoff [Benthiocarb] | 12,7 | 17 | 48 |
| 61 | | S-Äthyl-N,N-di-n-propylthiocarbamat | 6,6 | 8 | 62 |
| 36 | | 3-(3′,4′-Dichlorphenyl)-1-methoxy-1-methylharnstoff | 2,2 | 30 | 54 |
| 45 | 42 | S-Äthyl-N,N-di-n-propylthiocarbamat | 6,6 | 0 | 57 |
| 54 | | S-Äthyl-N,N-di-n-propylthiocarbamat | 6,6 | 1 | 57 |
| 40 | | S-Äthyl-N,N-di-n-propylthiocarbamat | 6,6 | 0 | 43 |
| 53 | 40 | S-Äthyl-N,N-di-n-propylthiocarbamat | 6,6 | 3 | 60 |
| 81 | 4 | S-n-Propyl-N-n-butyl-N-äthylthiocarbamat | 5,7 | 17 | 56 |
| 73 | 7 | S-Äthyl-N,N-di-n-propylthiocarbamat | 6,6 | 12 | 49 |
| 96 | 9 | S-Äthyl-N,N-diisobutylthiocarbamat | 8,1 | 16 | 61 |
| 97 | 13 | S-Äthyl-N,N-hexamethylenthiocarbamat | 5,3 | 13 | 53 |
| 86 | 16 | S-n-Propyl-N,N-di-n-propylthiocarbamat | 2,8 | 21 | 62 |
| 78 | 22 | S-Äthyl-N,N-di-n-propylthiocarbamat | 6,6 | 20 | 55 |
| 87 | 45 | S-Äthyl-N,N-di-n-propylthiocarbamat | 6,6 | 28 | 42 |
| 91 | 26 | S-Äthyl-N,N-di-n-propylthiocarbamat | 6,6 | 26 | 51 |
| 71 | 27 | S-Äthyl-N,N-di-n-propylthiocarbamat | 6,6 | 3 | 50 |
| 94 | | S-Äthyl-N,N-di-n-propylthiocarbamat | 6,6 | 18 | 39 |
| 79 | 31 | S-Äthyl-N,N-di-n-propylthiocarbamat | 6,6 | 17 | 46 |
| 75 | 33 | S-Äthyl-N,N-di-n-propylthiocarbamat | 6,6 | 9 | 37 |
| 82 | 34 | S-Äthyl-N,N-di-n-propylthiocarbamat | 6,6 | 12 | 46 |
| 72 | 35 | S-Äthyl-N,N-di-n-propylthiocarbamat | 6,6 | 8 | 37 |
| 98 | | S-Äthyl-N,N-di-n-propylthiocarbamat | 6,6 | 0 | 59 |

B) Untersuchungen durch Bodenbehandlung

Bei diesen Versuchen wurde das jeweilige Erzeugnis, das Herbizidwirkstoff und Antidotum enthielt, gleichmässig in der Weise mit dem Boden vermischt, dass dem Boden in einem Mischgefäss entsprechende Mengen von einer entsprechend eingestellten Stammlösung der beziehungsweise des Herbizide(s) und des zu untersuchenden Antidotums zugesetzt wurden. Bei Blindversuchen blieb wie bereits erwähnt wiederum das Antidotum oder der Herbizidwirkstoff weg.

In den so vorbereiteten und in die Versuchsgefässe gefüllten Boden wurden die Samen der untersuchten grasartigen und breitblättrigen Nutzpflanzen 2,5 cm tief gelegt. Die Gefässe wurden so gegossen, dass die günstige Entwicklung der Pflanzen sichergestellt war.

Die Ergebnisse sind in der folgenden Tabelle III zusammengestellt.

Tabelle III
Prozentuale Schutzwirkung von Antidota gegenüber der Schädigung von Nutzpflanzen durch Herbizide

| Erzeugnis | | | | | Nutzpflanze | Schutzwirkung in % |
|---|---|---|---|---|---|---|
| Antidotum Laufende Nummer von der Tabelle I | Beispiel Nr. | Menge in kg/ha | Herbizid Bezeichnung | Menge in kg/ha | | |
| 12 | | 1,3 | S-Äthyl-N,N-di-n-propylthiocarbamat | 6,3 | Mais | 82 |
| 32 | | 0,4 | S-Äthyl-N,N-di-n-propylthiocarbamat | 6,3 | Mais | 100 |
| 60 | | 0,35 | S-Äthyl-N,N-di-n-propylthiocarbamat | 6,3 | Mais | 85 |
| 15 | | 0,9 | S-Äthyl-N,N-di-n-propylthiocarbamat | 6,3 | Mais | 100 |
| 23 | | 1,3 | S-Äthyl-N,N-di-n-propylthiocarbamat | 6,3 | Mais | 99 |
| 5 | | 1,1 | S-Äthyl-N,N-di-n-propylthiocarbamat | 6,3 | Mais | 82 |
| 49 | 38 | 1,1 | S-Äthyl-N,N-di-n-propylthiocarbamat | 6,3 | Mais | 78 |
| 66 | | 0,9 | S-Äthyl-N,N-di-n-propylthiocarbamat | 6,3 | Mais | 100 |
| 6 | | 0,7 | S-(2,3,3-Trichlorallyl)-N,N-diisopropylthiocarbamat [Triallat] | 3,0 | Mohrenhirse | 89 |
| 21 | | 1,1 | S-Äthyl-N,N-di-n-propylthiocarbamat | 6,3 | Mais | 100 |
| 65 | | 0,8 | S-Äthyl-N,N-di-n-propylthiocarbamat | 6,3 | Mais | 100 |
| 13 | | 0,8 | S-Äthyl-N,N-di-n-propylthiocarbamat | 6,3 | Mais | 90 |
| 38 | | 1,1 | S-Äthyl-N,N-di-n-propylthiocarbamat | 6,3 | Mais | 96 |
| 69 | | 1,4 | S-Äthyl-N,N-di-n-propylthiocarbamat | 6,3 | Mais | 92 |
| 1 | | 0,9 | S-Äthyl-N,N-di-n-propylthiocarbamat | 6,3 | Mais | 93 |
| 17 | | 0,45 | S-Äthyl-N,N-diisobutylthiocarbamat | 8,9 | Mais | 97 |
| 95 | 21 | 0,55 | S-Äthyl-N,N-di-n-propylthiocarbamat | 6,3 | Mais | 78 |
| 76 | 11 | 0,35 | S-Äthyl-N,N-di-n-propylthiocarbamat | 6,3 | Mais | 95 |
| 62 | 12 | 0,65 | S-Äthyl-N,N-di-n-propylthiocarbamat | 6,3 | Mais | 98 |
| 46 | | 0,45 | S-Äthyl-N,N-di-n-propylthiocarbamat | 6,3 | Mais | 97 |
| 16 | | 0,4 | S-Äthyl-N,N-di-n-propylthiocarbamat | 6,3 | Mais | 65 |
| 4 | | 1,4 | S-Äthyl-N,N-di-n-propylthiocarbamat | 6,3 | Mais | 91 |
| 18 | 5 | 0,3 | S-Äthyl-N,N-di-n-propylthiocarbamat | 6,3 | Mais | 96 |
| 44 | | 1,3 | S-Äthyl-N,N-di-n-propylthiocarbamat | 6,3 | Mais | 89 |
| 31 | | 0,45 | S-Äthyl-N,N-di-n-propylthiocarbamat | 6,3 | Mais | 93 |
| 78 | 22 | 0,25 | S-Äthyl-N-äthyl-N-cyclohexylthiocarbamat | 6,4 | Mais | 42 |
| 90 | 46 | 1,1 | S-Äthyl-N,N-di-n-propylthiocarbamat | 6,3 | Mais | 89 |
| 63 | | 0,75 | S-Äthyl-N,N-di-n-propylthiocarbamat | 6,3 | Mais | 88 |
| 33 | 29 | 0,4 | S-Äthyl-N,N-di-n-propylthiocarbamat | 6,3 | Mais | 92 |
| 9 | | 0,3 | S-Äthyl-N,N-di-n-propylthiocarbamat | 6,3 | Mais | 88 |
| 25 | 20 | 0,5 | S-(2,3,3-Trichlorallyl)-N,N-diisopropylthiocarbamat [Triallat] | 8,4 | Mais | 79 |
| 57 | | 1,2 | S-Äthyl-N,N-di-n-propylthiocarbamat | 6,3 | Mais | 100 |
| 10 | | 0,9 | S-Äthyl-N,N-di-n-propylthiocarbamat | 6,3 | Mais | 73 |
| 77 | | 0,7 | S-Äthyl-N,N-di-n-propylthiocarbamat | 6,3 | Mais | 72 |
| 55 | 18 | 0,8 | S-Äthyl-N,N-di-n-propylthiocarbamat | 6,3 | Mais | 100 |
| 88 | 32 | 0,9 | S-Äthyl-N,N-di-n-propylthiocarbamat | 6,3 | Mais | 90 |
| 39 | 19 | 0,45 | S-Äthyl-N,N-di-n-propylthiocarbamat | 6,3 | Mais | 68 |
| 27 | | 0,35 | S-Äthyl-N,N-di-n-propylthiocarbamat | 6,3 | Mais | 95 |
| 43 | | 0,3 | N-Isopropyl-α-chloracetanilid [Propachlor] | 3,1 | Mohrenhirse | 60 |
| 92 | 28 | 0,75 | S-Äthyl-N,N-di-n-propylthiocarbamat | 6,3 | Mais | 86 |
| 42 | | 0,6 | S-Äthyl-N,N-di-n-propylthiocarbamat | 6,3 | Mais | 98 |
| 93 | 10 | 0,75 | S-Äthyl-N,N-di-n-propylthiocarbamat | 6,3 | Mais | 83 |
| 80 | 39 | 1,2 | S-Äthyl-N,N-di-n-propylthiocarbamat | 6,3 | Mais | 83 |

Fortsetzung der Tabelle III
Prozentuale Schutzwirkung von Antidota gegenüber der Schädigung von Nutzpflanzen durch Herbizide

| Erzeugnis | | | | | Nutz-pflanze | Schutz-wirkung in % |
|---|---|---|---|---|---|---|
| Antidotum Laufende Nummer von der Tabelle I | Beispiel Nr. | Menge in kg/ha | Herbizid Bezeichnung | Menge in kg/ha | | |
| 52 | | 1,4 | S-n-Propyl-N,N-di-n-propylthiocarbamat | 6,5 | Mais | 93 |
| 84 | | 3,0 | S-Äthyl-N,N-di-n-propylthiocarbamat | 6,5 | Mais | 63 |
| 30 | | 3,0 | S-Äthyl-N,N-di-n-propylthiocarbamat | 6,5 | Mais | 54 |
| 34 | | 1,6 | S-Äthyl-N,N-di-n-propylthiocarbamat | 6,5 | Mais | 95 |
| 89 | 6 | 0,9 | S-Äthyl-N,N-di-n-propylthiocarbamat | 6,3 | Mais | 79 |
| 70 | | 0,25 | S-Äthyl-N,N-di-n-propylthiocarbamat | 6,3 | Mais | 81 |
| 37 | 15 | 0,95 | S-Äthyl-N,N-di-n-propylthiocarbamat | 6,3 | Mais | 100 |
| 85 | 8 | 0,8 | S-Äthyl-N,N-di-n-propylthiocarbamat | 6,3 | Mais | 87 |
| 74 | | 0,45 | S-Äthyl-N,N-di-n-propylthiocarbamat | 6,3 | Mais | 77 |
| 48 | | 1,1 | S-Äthyl-N,N-di-n-propylthiocarbamat | 6,3 | Mais | 98 |
| 20 | 17 | 1,2 | S-Äthyl-N,N-di-n-propylthiocarbamat | 6,3 | Mais | 87 |
| 83 | 14 | 0,7 | S-Äthyl-N,N-di-n-propylthiocarbamat | 6,3 | Mais | 81 |

Aus den obigen Tabellen II und III geht hervor, dass durch die Antidota der erfindungsgemässen Erzeugnisse beziehungsweise erfindungsgemässen Verbindungen eine beträchtliche Verminderung bis völlige Beseitigung der schädlichen Wirkungen der Herbizide auf die Nutzpflanzen erzielt wurde.

Die in den obigen Tabellen II und III aufgeführten Antidota senken neben dem Schutz der Nutzpflanzen gegen schädliche Einflüsse der Herbizide die unkrautvernichtende Wirkung der Herbizidwirkstoffe nicht.

Ferner wird die Herstellung von gebrauchsfertigen Pflanzenschutzmittelpräparaten mit einem Gehalt an erfindungsgemäss festgelegten Antidota an Hand der folgenden Beispiele näher erläutert.

Beispiel 47
Wirkstoffkonzentrat

Es wurden 90 Gew.-Teile eines der obigen Herbizidwirkstoffe, 8 Gew.-Teile eines der obigen Antidota und 5 Gew.-Teile des Emulgiermittels Polyoxyäthylensorbitanmonooleat [Tween 80®] miteinander vermischt. Aus dem so gewonnenen Konzentrat konnte durch Verdünnen mit einer entsprechenden Menge Lösungsmittel, zum Beispiel Toluol, und Wasser eine stabile versprühbare Emulsion bereitet werden.

Beispiel 48
Emulgierbares Konzentrat

Es wurden 10 Gew.-Teile eines der obigen Herbizidwirkstoffe in 25 Gew.-Teilen Xylol gelöst und dann wurden 2 Gew.-Teile eines der obigen Antidota und 4 Gew.-Teile des Emulgiermittels Polyoxyäthylensorbitanmonooleat [Tween 80®] zugesetzt. Das so gewonnene Konzentrat lieferte beim Verdünnen mit Wasser eine stabile versprühbare Emulsion.

Beispiel 49
Benetzbares Pulver

Es wurden 15 Gew.-Teile eines der obigen Herbizidwirkstoffe, 2 Gew.-Teile eines der obigen Antidota, 50 Gew.-Teile Kaolin und 6 Gew.-Teile Trimethylcetylammoniumbromid miteinander vermischt und in einer Kugelmühle gemahlen. Das so bereitete benetzbare Pulver konnte in Wasser suspendiert werden und die Suspension war versprühbar.

**Patentansprüche für die Vertragsstaaten: BE, CH, DE, FR, GB, IT, LI, LU, NL, SE**

1. Erzeugnisse, enthaltend mindestens 1 Antidotum der allgemeinen Formel

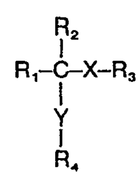
I ,

worin

X und Y unabhängig voneinander für Sauerstoff, Schwefel, einen Rest der allgemeinen Formel

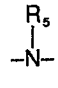
II ,

in welch letzterer

$R_5$ Wasserstoff, einen, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierten, Phenylrest, einen Alkylrest mit 1 bis 4 Kohlenstoffatom(en) oder einen Alkenylrest mit 3 oder 4 Kohlenstoffatomen bedeutet,
beziehungsweise einen Rest der allgemeinen Formel

$$\begin{array}{c} R_6 \\ | \\ -N- \end{array} \qquad III ,$$

in welch letzterer

$R_6$ Wasserstoff, einen, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierten, Phenylrest, einen Alkylrest mit 1 bis 4 Kohlenstoffatom(en) oder einen Alkenylrest mit 3 oder 4 Kohlenstoffatomen bedeutet,
stehen,

$R_1$ und $R_2$ unabhängig voneinander Wasserstoff, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierte, Phenylrest(e), Furylrest(e), gegebenenfalls durch 1 oder mehr Halogenatom(e) substituierte, Phenylalkylreste mit 1 bis 4 Kohlenstoffatom(en) im Alkylteil, geradkettige oder verzweigte Alkyl-, Mono- beziehungsweise Polyhalogenalkyl- beziehungsweise Mono- beziehungsweise Polycyanalkylreste mit 1 bis 4 Kohlenstoffatom(en) beziehungsweise geradkettige oder verzweigte Alkenylreste mit 2 bis 4 Kohlenstoffatomen bedeuten und von diesen Symbolen wahlweise,
falls X und/oder Y für einen Rest beziehungsweise Reste der allgemeinen Formel(n) II und/oder III steht beziehungsweise stehen,

$R_1$ auch eine das Kohlenstoffatom, an welches es gebunden ist, mit X oder Y verbindende zweite Bindung darstellen kann, und

$R_3$ und $R_4$ unabhängig voneinander für Wasserstoff, geradkettige oder verzweigte Alkylreste mit 1 bis 18 Kohlenstoffatom(en), Mono- beziehungsweise Polyhalogenalkyl- beziehungsweise Mono- beziehungsweise Polycyanalkylreste jeweils mit 1 bis 4 Kohlenstoffatom(en), geradkettige oder verzweigte Alkoxyalkyl- beziehungsweise Alkoxyalkoxyalkylreste mit 1 bis 4 Kohlenstoffatom(en) im Alkylteil und 1 bis 4 Kohlenstoffatom(en) im Alkoxyteil beziehungsweise in den Alkoxyteilen, Cycloalkylreste mit 5 oder 6 Kohlenstoffatomen, Phenylalkylreste mit 1 oder 2 Kohlenstoffatom(en) im Alkylteil, Alkenylreste mit 3 oder 4 Kohlenstoffatomen, Alkinylreste mit 3 oder 4 Kohlenstoffatomen, Phenylalkenylreste mit 3 oder 4 Kohlenstoffatomen im Alkenylteil, Dialkylaminoalkylreste mit 1 bis 4 Kohlenstoffatom(en) in jedem Alkylteil, Hydroxyalkylreste mit 1 bis 4 Kohlenstoffatom(en), Furfurylreste beziehungsweise Tetrahydrofurfurylreste stehen oder

$R_3$ und $R_4$ zusammen einen X und Y unter Bildung eines 5- bis 7-gliedrigen Ringes verbindenden, gegebenenfalls durch 1 oder mehr Hydroxygruppe(n), Methoxyrest(e), Halogenatom(e) und/oder Acetoxyrest(e) substituierten, Alkylenrest mit 2 bis 4 Kohlenstoffatom(en), Alkenylenrest mit 2 bis 4 Kohlenstoffatomen oder Phenylenrest oder zusammen einen X und Y verbindenden Glucofuranosylenrest darstellen,
mit den weiteren Massgaben, dass

a) im Falle dass mindestens 1 von $R_1$ und $R_2$ für einen Methyl-, Monochlormethyl- oder Monochlorphenylrest steht, mindestens 1 von $R_3$ und $R_4$ von einem nicht substituierten Alkylrest mit 2 bis 18 Kohlenstoffatomen verschieden ist, und,

b) im Falle dass $R_2$ für Wasserstoff steht und $R_1$ einen nicht substituierten Phenylrest bedeutet, mindestens 1 von $R_3$ und $R_4$ von einem Methylrest verschieden ist,

und 1 oder mehr Thiocarbamat-, Carbamat-, Säureamid-, Harnstoffderivat- und/oder Thioharnstoffderivatherbizidwirkstoff(e) als Kombinationspräparat zur gleichzeitigen, getrennten oder zeitlich abgestuften Anwendung im Pflanzenschutz zur Unkrautbekämpfung, zweckmässig zusammen mit 1 oder mehr üblichen Träger- und/oder Hilfsstoff(en).

2. Erzeugnisse nach Anspruch 1, dadurch gekennzeichnet, dass der Alkyl-, Mono- oder Polyhalogenalkylrest, für den $R_1$ stehen kann, und/oder der/die Alkylrest(e) für den/die $R_2$ und/oder $R_5$ und/oder $R_6$ stehen kann/können, ein solcher/solche mit 1 oder 2 Kohlenstoffatom(en) ist/sind.

3. Erzeugnisse nach Anspruch 1 oder 2, dadurch gekennzeichnet, dass das Halogenatom des Mono- oder Polyhalogenalkylrestes, für den $R_1$ stehen kann, und/oder das/die Halogenatom(e), durch welches der Phenylalkylrest, für den $R_1$ stehen kann, und/oder der/die Phenylrest(e), für den/die $R_5$ und/oder $R_6$ stehen kann/können, substituiert sein kann/können, und/oder das Halogenatom, durch welches der Alkylenrest, den $R_3$ und $R_4$ zusammen darstellen können, substituiert sein kann, Chlor und/oder Brom ist beziehungsweise sind.

4. Erzeugnisse nach Anspruch 1, dadurch gekennzeichnet, dass der Mono- oder Polyhalogenalkylrest, für den $R_1$ stehen kann, ein Chlormethyl-, Dichlormethyl, Trichlormethyl-, Brommethyl-, Dichloräthyl-, Dibromäthyl- oder Dibrom-n-propylrest ist.

5. Erzeugnisse nach Anspruch 1, dadurch gekennzeichnet, dass der Phenylalkylrest, für den $R_1$ stehen kann, ein solcher mit 1 oder 2 Kohlenstoffatom(en) im Alkylteil ist.

6. Erzeugnisse nach Anspruch 1, dadurch gekennzeichnet, dass der Alkyl- oder Alkoxyrest, durch welchen der Phenylrest, für den $R_1$ stehen kann, substituiert sein kann, ein solcher mit 1 oder 2 Kohlenstoffatom(en) ist.

7. Erzeugnisse nach Anspruch 1, dadurch gekennzeichnet, dass der/die Alkylrest(e), durch welchen/welche der/die Phenylrest(e), für den/die $R_5$ und/oder $R_6$ stehen kann/können, substituiert sein kann/können, ein solcher/solche mit 1 oder 2 Kohlenstoffatom(en) ist/sind.

8. Erzeugnisse nach Anspruch 1, dadurch gekennzeichnet, dass der Alkenylrest, für den $R_1$ stehen kann, ein solcher mit 3 oder 4 Kohlenstoffatomen ist.

9. Erzeugnisse nach Anspruch 1, dadurch gekennzeichnet, dass der/die Alkylrest(e), für den/

die $R_3$ und/oder $R_4$ stehen kann/können, ein solcher/solche mit 1 bis 12, insbesondere 1 bis 7, Kohlenstoffatom(en) ist/sind.

10. Erzeugnisse nach Anspruch 1, dadurch gekennzeichnet, dass der/die Alkoxyalkyl- und/oder Alkoxyalkoxyalkylrest(e), für den/die $R_3$ und/oder $R_4$ stehen kann/können, ein solcher/solche mit 1 oder 2 Kohlenstoffatom(en) im Alkoxyteil beziehungsweise in den Alkoxyteilen ist/sind.

11. Erzeugnisse nach Anspruch 1, dadurch gekennzeichnet, dass der/die Alkenylrest(e), für den/die $R_3$ und/oder $R_4$ und/oder $R_5$ und/oder $R_6$ stehen kann/können, [ein] Allyl- und/oder Crotylrest(e) ist/sind.

12. Erzeugnisse nach Anspruch 1, dadurch gekennzeichnet, dass der/die Alkinylrest(e), für den/die $R_3$ und/oder $R_4$ stehen kann/können, [ein] Propargylrest(e) ist/sind.

13. Erzeugnisse nach Anspruch 1, dadurch gekennzeichnet, dass der/die Dialkylaminoalkylrest(e), für den/die $R_3$ und/oder $R_4$ stehen kann/können, ein solcher/solche mit 1 oder 2 Kohlenstoffatom(en) in jedem Alkylteil ist/sind.

14. Erzeugnisse nach Anspruch 1, dadurch gekennzeichnet, dass der Ring, welcher durch die gegebenenfalls erfolgende Verbindung von X und Y durch den Rest, den $R_3$ und $R_4$ zusammen darstellen können, gebildet sein kann, 5- oder 6-gliedrig ist.

15. Erzeugnisse nach Anspruch 1, dadurch gekennzeichnet, dass der Alkylenrest, den $R_3$ und $R_4$ zusammen darstellen können, ein solcher mit 2 oder 3 Kohlenstoffatomen, ganz besonders ein Äthylen-, Prop-1,3-ylen-, Prop-1,2-ylen- oder But-1,2-ylenrest, ist.

16. Erzeugnisse nach Anspruch 1, dadurch gekennzeichnet, dass der Alkenylenrest, den $R_3$ und $R_4$ zusammen darstellen können, ein solcher mit 2 bis 4 Kohlenstoffatomen, insbesondere ein But-2-en-1,2-ylen- oder Propenylenrest, ist.

17. Erzeugnisse nach Anspruch 1, dadurch gekennzeichnet, dass

X und Y für Sauerstoff stehen,

$R_1$ einen, gegebenenfalls durch einen Alkyl- oder Alkoxyrest mit 1 bis 4 Kohlenstoffatom(en) substituierten, Phenylrest, einen Furylrest, einen, gegebenenfalls durch 1 oder mehr Halogenatom(e) substituierten, Phenylalkylrest mit 1 bis 4 Kohlenstoffatom(en) im Alkylteil, einen geradkettigen Alkyl-, Mono- oder Polyhalogenalkyl- oder Monocyanalkylrest mit 1 bis 4 Kohlenstoffatom(en) oder einen geradkettigen Alkenylrest mit 2 bis 4 Kohlenstoffatomen bedeutet,

$R_2$ Wasserstoff bedeutet und

$R_3$ und $R_4$ unabhängig voneinander für geradkettige oder verzweigte Alkylreste mit 1 bis 18 Kohlenstoffatom(en), Monohalogen- beziehungsweise Monocyanalkylreste mit 1 bis 4 Kohlenstoffatom(en), geradkettige Alkoxyalkyl- beziehungsweise Alkoxyalkoxyalkylreste mit 1 bis 4 Kohlenstoffatom(en) im Alkylteil und 1 bis 4 Kohlenstoffatom(en) im Alkoxyteil beziehungsweise in den Alkoxyteilen, Cycloalkylreste mit 5 oder 6 Kohlenstoffatomen, Phenylalkylreste mit 1 oder 2 Kohlenstoffatom(en) im Alkylteil, Alkenylreste mit 3 oder 4 Kohlenstoffatomen, einen Alkinylrest mit 3 oder 4 Kohlenstoffatomen, Phenylalkenylreste mit 3 oder 4 Kohlenstoffatomen im Alkenylteil, Dialkylaminoalkylreste mit 1 bis 4 Kohlenstoffatom(en) in jedem Alkylteil beziehungsweise Furfurylreste stehen und

$R_3$ zusätzlich wahlweise Wasserstoff bedeuten kann oder

$R_3$ und $R_4$ zusammen einen X und Y unter Bildung eines 5- bis 7-gliedrigen Ringes verbindenden, gegebenenfalls durch 1 Hydroxygruppe, Methoxyrest, Halogenatom und/oder Acetoxyrest substituierten, Alkylenrest mit 2 bis 4 Kohlenstoffatomen, Alkenylenrest mit 2 bis 4 Kohlenstoffatomen oder zusammen einen X und Y verbindenden Glucofuranosylenrest darstellen.

18. Erzeugnisse nach Anspruch 1, dadurch gekennzeichnet, dass

X und Y für Sauerstoff stehen,

$R_1$ einen geradkettigen oder verzweigten Alkyl-, Mono- oder Polyhalogenalkyl- oder Monocyanalkylrest jeweils mit 1 bis 4 Kohlenstoffatom(en) oder einen Alkenylrest mit 2 bis 4 Kohlenstoffatomen bedeutet,

$R_2$ einen Alkylrest mit 1 bis 4 Kohlenstoffatom(en) oder einen Phenylrest darstellt und

$R_3$ und $R_4$ die im Anspruch 17 angegebenen Bedeutungen haben.

19. Erzeugnisse nach Anspruch 1, dadurch gekennzeichnet, dass

X für Sauerstoff steht,

Y einen Rest der allgemeinen Formel

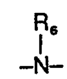 III ,

in welch letzterer

$R_6$ für einen Phenyl- oder Allylrest steht,

bedeutet,

$R_1$ einen Dichlormethylrest darstellt,

$R_2$ für Wasserstoff steht und

$R_3$ und $R_4$ zusammen einen X und Y unter Bildung eines Oxazolidinringes verbindenden Äthylenrest darstellen.

20. Erzeugnisse nach Anspruch 1, dadurch gekennzeichnet, dass

X für Sauerstoff steht,

Y Schwefel bedeutet,

$R_1$ einen geradkettigen oder verzweigten Alkyl-, Mono- oder Polyhalogenalkyl- oder Monocyanalkylrest jeweils mit 1 bis 4 Kohlenstoffatom(en) oder einen Alkenylrest mit 2 bis 4 Kohlenstoffatomen darstellt,

$R_2$ für Wasserstoff steht und

$R_3$ und $R_4$ die im Anspruch 17 angegebenen Bedeutungen haben.

21. Erzeugnisse nach Anspruch 1, dadurch gekennzeichnet, dass

X für Sauerstoff oder einen Rest der allgemeinen Formel

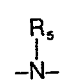 II ,

in welch letzterer

$R_5$ einen Phenylrest, einen Alkylrest mit 1 bis 4 Kohlenstoffatom(en) oder einen Alkenylrest mit 3 oder 4 Kohlenstoffatomen bedeutet,

steht,

Y einen Rest der allgemeinen Formel

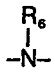 III ,

in welch letzterer

$R_6$ für Wasserstoff, einen Phenylrest, einen Alkylrest mit 1 bis 4 Kohlenstoffatom(en) oder einen Alkenylrest mit 3 oder 4 Kohlenstoffatomen steht, bedeutet,

$R_1$ die im Anspruch 17 angegebenen Bedeutungen hat,

$R_2$ Wasserstoff bedeutet und

$R_3$ und $R_4$ zusammen einen X und Y unter Bildung eines 5- bis 7-gliedrigen Ringes verbindenden Alkylenrest mit 2 bis 4 Kohlenstoffatomen oder Alkenylenrest mit 2 bis 4 Kohlenstoffatomen darstellen.

22. Erzeugnisse nach Anspruch 1, dadurch gekennzeichnet, dass

X für einen Rest der Formel $-N=$ steht,

Y einen Rest der Formel

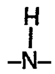 bedeutet,

$R_1$ eine das Kohlenstoffatom, an welches es gebunden ist, mit X oder Y verbindende zweite Bindung darstellt,

$R_2$ für einen Dichlormethylrest steht und

$R_3$ und $R_4$ zusammen einen Phen-1,2-ylenrest darstellen.

23. Erzeugnisse nach Anspruch 1 bis 22, dadurch gekennzeichnet, dass sie das Antidotum beziehungsweise die Antidota der allgemeinen Formel I in einer Menge von 0,01 bis 15-Gew.-%, bezogen auf das Gewicht des/der Herbizidwirkstoffe[s], enthalten und die Gesamtmenge des Antidotums beziehungsweise der Antidota und des/der Herbizidwirkstoffe[s], bezogen auf die Gesamtmenge des Erzeugnisses, 0,1 bis 95 Gew.-% beträgt, während der Rest 1 oder mehr feste[r] und/oder flüssige[r] neutrale[r] Träger und/oder anionenaktive[r], kationenaktive[r] und/oder nicht-ionogene[r] oberflächenaktive[r] Stoff(e) ist/sind.

24. 2-(Dichlormethyl)-3-phenyl-1,3-oxazolidin.

25. 2-(Dichlormethyl)-3-allyl-1,3-oxazolidin.

26. Verfahren zur Herstellung der Verbindungen nach Anspruch 24 oder 25, dadurch gekennzeichnet, dass man in an sich bekannter Weise Dichloracetaldehyd mit N-(Phenyl)-äthanolamin oder N-(Allyl)-äthanolamin in aromatischen Kohlenwasserstoffen, insbesondere Benzol, in etwa stöchiometrischen Mengenverhältnissen vermischt und mehrere Stunden unter Rückfluss zum Sieden erhitzt.

**Patentansprüche für den Vertragsstaat: AT**

1. Erzeugnisse, enthaltend mindestens 1 Antidotum der allgemeinen Formel

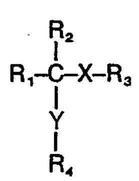 I ,

worin

X und Y unabhängig voneinander für Sauerstoff, Schwefel, einen Rest der allgemeinen Formel

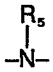 II ,

in welch letzterer

$R_5$ Wasserstoff, einen, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierten, Phenylrest, einen Alkylrest mit 1 bis 4 Kohlenstoffatom(en) oder einen Alkenylrest mit 3 oder 4 Kohlenstoffatomen bedeutet,

beziehungsweise einen Rest der allgemeinen Formel

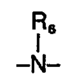 III ,

in welch letzterer

$R_6$ Wasserstoff, einen, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierten, Phenylrest, einen Alkylrest mit 1 bis 4 Kohlenstoffatom(en) oder einen Alkenylrest mit 3 oder 4 Kohlenstoffatomen bedeutet,

stehen,

$R_1$ und $R_2$ unabhängig voneinander Wasserstoff, gegebenenfalls durch 1 oder mehr Alkyl- und/oder Mono- und/oder Polyhalogenalkylrest(e) mit 1 bis 4 Kohlenstoffatom(en), Alkoxyrest(e) mit 1 bis 4 Kohlenstoffatom(en) und/oder Halogenatom(e) substituierte, Phenylrest(e), Furylrest(e), gegebenenfalls durch 1 oder mehr Halogenatom(e) substituierte, Phenylalkylreste mit 1 bis 4 Kohlenstoffatom(en) im Alkylteil, geradkettige oder verzweigte Alkyl-, Mono- beziehungsweise Polyhalogenalkyl- beziehungsweise Mono- beziehungsweise Polycyanalkylreste mit 1 bis 4 Kohlenstoffatom(en) beziehungsweise geradkettige oder verzweigte Alkenylreste mit 2 bis 4 Kohlenstoffatomen bedeuten und von diesen Symbolen wahlweise,

fass X und/oder Y für einen Rest beziehungsweise Reste der allgemeinen Formel(n) II und/oder III steht beziehungsweise stehen,

$R_1$ auch eine das Kohlenstoffatom, an welches es gebunden ist, mit X oder Y verbindende zweite Bindung darstellen kann, und

$R_3$ und $R_4$ unabhängig voneinander für Wasserstoff, geradkettige oder verzweigte Alkylreste mit 1 bis 18 Kohlenstoffatom(en), Mono- beziehungsweise Polyhalogenalkyl- beziehungsweise Mono- beziehungsweise Polycyanalkylreste jeweils mit 1 bis 4 Kohlenstoffatom(en), geradkettige oder verzweigte Alkoxyalkyl- beziehungsweise Alkoxyalkoxyalkylreste mit 1 bis 4 Kohlenstoffatom(en) im Alkylteil und 1 bis 4 Kohlenstoffatom(en) im Alkoxyteil beziehungsweise in den Alkoxyteilen, Cycloalkylreste mit 5 oder 6 Kohlenstoffatomen, Phenylalkylreste mit 1 oder 2 Kohlenstoffatom(en) im Alkylteil, Alkenylreste mit 3 oder 4 Kohlenstoffatomen, Alkinylreste mit 3 oder 4 Kohlenstoffatomen, Phenylalkenylreste mit 3 oder 4 Kohlenstoffatomen im Alkenylteil, Dialkylaminoalkylreste mit 1

bis 4 Kohlenstoffatom(en) in jedem Alkylteil, Hydroxyalkylreste mit 1 bis 4 Kohlenstoffatom(en), Furfurylreste beziehungsweise Tetrahydrofurfurylreste stehen oder

$R_3$ und $R_4$ zusammen einen X und Y unter Bildung eines 5- bis 7-gliedrigen Ringes verbindenden, gegebenenfalls durch 1 oder mehr Hydroxygruppe(n), Methoxyrest(e), Halogenatom(e) und/oder Acetoxyrest(e) substituierten, Alkylenrest mit 2 bis 4 Kohlenstoffatom(en), Alkenylenrest mit 2 bis 4 Kohlenstoffatomen oder Phenylenrest oder zusammen einen X und Y verbindenden Glucofuranosylenrest darstellen,

mit den weiteren Massgaben, dass

a) im Falle dass mindestens 1 von $R_1$ und $R_2$ für einen Methyl-, Monochlormethyl- oder Monochlorphenylrest steht, mindestens 1 von $R_3$ und $R_4$ von einem nicht substituierten Alkylrest mit 2 bis 18 Kohlenstoffatomen verschieden ist, und,

b) im Falle dass $R_2$ für Wasserstoff steht und $R_1$ einen nicht substituierten Phenylrest bedeutet, mindestens 1 von $R_3$ und $R_4$ von einem Methylrest verschieden ist,

und 1 oder mehr Thiocarbamat-, Carbamat-, Säureamid-, Harnstoffderivat- und/oder Thioharnstoffderivatherbizidwirkstoff(e) als Kombinationspräparat zur gleichzeitigen, getrennten oder zeitlich abgestuften Anwendung im Pflanzenschutz zur Unkrautbekämpfung, zweckmässig zusammen mit 1 oder mehr üblichen Träger- und/oder Hilfsstoff(en).

2. Erzeugnisse nach Anspruch 1, dadurch gekennzeichnet, dass der Alkyl-, Mono- oder Polyhalogenalkylrest, für den $R_1$ stehen kann, und/oder der/die Alkylrest(e) für den/die $R_2$ und/oder $R_5$ und/oder $R_6$ stehen kann/können, ein solcher/solche mit 1 oder 2 Kohlenstoffatom(en) ist/sind.

3. Erzeugnisse nach Anspruch 1 oder 2, dadurch gekennzeichnet, dass das Halogenatom des Mono- oder Polyhalogenalkylrestes, für den $R_1$ stehen kann, und/oder das/die Halogenatom(e), durch welches der Phenylalkylrest, für den $R_1$ stehen kann, und/oder der/die Phenylrest(e), für den/die $R_5$ und/oder $R_6$ stehen kann/können, substituiert sein kann/können, und/oder das Halogenatom, durch welches der Alkylenrest, den $R_3$ und $R_4$ zusammen darstellen können, substituiert sein kann, Chlor und/oder Brom ist beziehungsweise sind.

4. Erzeugnisse nach Anspruch 1, dadurch gekennzeichnet, dass der Mono- oder Polyhalogenalkylrest, für den $R_1$ stehen kann, ein Chlormethyl-, Dichlormethyl-, Trichlormethyl-, Brommethyl-, Dichloräthyl-, Dibromäthyl- oder Dibrom-n-propylrest ist.

5. Erzeugnisse nach Anspruch 1, dadurch gekennzeichnet, dass der Phenylalkylrest, für den $R_1$ stehen kann, ein solcher mit 1 oder 2 Kohlenstoffatom(en) im Alkylteil ist.

6. Erzeugnisse nach Anspruch 1, dadurch gekennzeichnet, dass der Alkyl- oder Alkoxyrest, durch welchen der Phenylrest, für den $R_1$ stehen kann, substituiert sein kann, ein solcher mit 1 oder 2 Kohlenstoffatom(en) ist.

7. Erzeugnisse nach Anspruch 1, dadurch gekennzeichnet, dass der/die Alkylrest(e), durch welchen/welche der/die Phenylrest(e) für den/die $R_5$ und/oder $R_6$ stehen kann/können, substituiert sein kann/können, ein solcher/solche mit 1 oder 2 Kohlenstoffatom(en) ist/sind.

8. Erzeugnisse nach Anspruch 1, dadurch gekennzeichnet, dass der Alkenylrest, für den $R_1$ stehen kann, ein solcher mit 3 oder 4 Kohlenstoffatomen ist.

9. Erzeugnisse nach Anspruch 1, dadurch gekennzeichnet, dass der/die Alkylrest(e), für den/die $R_3$ und/oder $R_4$ stehen kann/können, ein solcher/solche mit 1 bis 12, insbesondere 1 bis 7, Kohlenstoffatom(en) ist/sind.

10. Erzeugnisse nach Anspruch 1, dadurch gekennzeichnet, dass der/die Alkoxyalkyl- und/oder Alkoxyalkoxyalkylrest(e), für den/die $R_3$ und/oder $R_4$ stehen kann/können, ein solcher/solche mit 1 oder 2 Kohlenstoffatom(en) im Alkoxyteil beziehungsweise in den Alkoxyteilen ist/sind.

11. Erzeugnisse nach Anspruch 1, dadurch gekennzeichnet, dass der/die Alkenylrest(e), für den/die $R_3$ und/oder $R_4$ und/oder $R_5$ und/oder $R_6$ stehen kann/können, [ein] Allyl- und/oder Crotylrest(e) ist/sind.

12. Erzeugnisse nach Anspruch 1, dadurch gekennzeichnet, dass der/die Alkinylrest(e), für den/die $R_3$ und/oder $R_4$ stehen kann/können, [ein] Propargylrest(e) ist/sind.

13. Erzeugnisse nach Anspruch 1, dadurch gekennzeichnet, dass der/die Dialkylaminoalkylrest(e), für den/die $R_3$ und/oder $R_4$ stehen kann/können, ein solcher/solche mit 1 oder 2 Kohlenstoffatom(en) in jedem Alkylteil ist/sind.

14. Erzeugnisse nach Anspruch 1, dadurch gekennzeichnet, dass der Ring, welcher durch die gegebenenfalls erfolgende Verbindung von X und Y durch den Rest, den $R_3$ und $R_4$ zusammen darstellen können, gebildet sein kann, 5- oder 6-gliedrig ist.

15. Erzeugnisse nach Anspruch 1, dadurch gekennzeichnet, dass der Alkylenrest, den $R_3$ und $R_4$ zusammen darstellen können, ein solcher mit 2 oder 3 Kohlenstoffatomen, ganz besonders ein Äthylen-, Prop-1,3-ylen-, Prop-1,2-ylen- oder But-1,2-ylenrest, ist.

16. Erzeugnisse nach Anspruch 1, dadurch gekennzeichnet, dass der Alkenylenrest, den $R_3$ und $R_4$ zusammen darstellen können, ein solcher mit 2 bis 4 Kohlenstoffatomen, insbesondere ein But-2-en-1,2-ylen- oder Propenylenrest, ist.

17. Erzeugnisse nach Anspruch 1, dadurch gekennzeichnet, dass

X und Y für Sauerstoff stehen,

$R_1$ einen, gegebenenfalls durch einen Alkyl- oder Alkoxyrest mit 1 bis 4 Kohlenstoffatom(en) substituierten, Phenylrest, einen Furylrest, einen, gegebenenfalls durch 1 oder mehr Halogenatom(e) substituierten, Phenylalkylrest mit 1 bis 4 Kohlenstoffatom(en) im Alkylteil, einen geradkettigen Alkyl-, Mono- oder Polyhalogenalkyl- oder Monocyanalkylrest mit 1 bis 4 Kohlenstoffatom(en) oder einen geradkettigen Alkenylrest mit 2 bis 4 Kohlenstoffatomen bedeutet,

$R_2$ Wasserstoff bedeutet und

$R_3$ und $R_4$ unabhängig voneinander für geradkettige oder verzweigte Alkylreste mit 1 bis 18 Kohlenstoffatom(en), Monohalogen- beziehungsweise Monocyanalkylreste mit 1 bis 4 Kohlenstoffatom(en), geradkettige Alkoxyalkyl- beziehungsweise Alkoxyalkoxyalkylreste mit 1 bis 4 Kohlenstoffatom(en) im Alkylteil und 1 bis 4 Kohlenstoffatom(en) im Alkoxyteil beziehungsweise in den Alkoxyteilen, Cycloalkylreste mit 5 oder 6 Kohlenstoffatomen, Phenylalkylreste mit 1 oder 2 Kohlenstoffatom(en) im Alkylteil, Alkenylreste mit 3 oder 4 Kohlenstoffatomen, einen Alkinylrest mit 3 oder 4 Kohlenstoffatomen, Phenylalkenylreste mit 3 oder 4 Kohlenstoffatomen im Alkenylteil, Dialkylaminoalkylreste mit 1 bis 4 Kohlenstoffatom(en) in jedem Alkylteil beziehungsweise Furfurylreste stehen und

$R_3$ zusätzlich wahlweise Wasserstoff bedeuten kann oder

$R_3$ und $R_4$ zusammen einen X und Y unter Bildung eines 5- bis 7-gliedrigen Ringes verbindenden, gegebenenfalls durch 1 Hydroxygruppe, Methoxyrest, Halogenatom und/oder Acetoxyrest substituierten, Alkylenrest mit 2 bis 4 Kohlenstoffatomen, Alkenylenrest mit 2 bis 4 Kohlenstoffatomen oder zusammen einen X und Y verbindenden Glucofuranosylenrest darstellen.

18. Erzeugnisse nach Anspruch 1, dadurch gekennzeichnet, dass

X und Y für Sauerstoff stehen,

$R_1$ einen geradkettigen oder verzweigten Alkyl-, Mono- oder Polyhalogenalkyl- oder Monocyanalkylrest jeweils mit 1 bis 4 Kohlenstoffatom(en) oder einen Alkenylrest mit 2 bis 4 Kohlenstoffatomen bedeutet,

$R_2$ einen Alkylrest mit 1 bis 4 Kohlenstoffatom(en) oder einen Phenylrest darstellt und

$R_3$ und $R_4$ die im Anspruch 17 angegebenen Bedeutungen haben.

19. Erzeugnisse nach Anspruch 1, dadurch gekennzeichnet, dass

X für Sauerstoff steht,

Y einen Rest der allgemeinen Formel

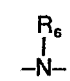 III ,

in welch letzterer

$R_6$ für einen Phenyl- oder Allylrest steht, bedeutet,

$R_1$ einen Dichlormethylrest darstellt,

$R_2$ für Wasserstoff steht und

$R_3$ und $R_4$ zusammen einen X und Y unter Bildung eines Oxazolidinringes verbindenden Äthylenrest darstellen.

20. Erzeugnisse nach Anspruch 1, dadurch gekennzeichnet, dass

X für Sauerstoff steht,

Y Schwefel bedeutet,

$R_1$ einen geradkettigen oder verzweigten Alkyl-, Mono- oder Polyhalogenalkyl- oder Monocyanalkylrest jeweils mit 1 bis 4 Kohlenstoffatom(en) oder einen Alkenylrest mit 2 bis 4 Kohlenstoffatomen darstellt,

$R_2$ für Wasserstoff steht und

$R_3$ und $R_4$ die im Anspruch 17 angegebenen Bedeutungen haben.

21. Erzeugnisse nach Anspruch 1, dadurch gekennzeichnet, dass

X für Sauerstoff oder einen Rest der allgemeinen Formel

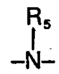 II ,

in welch letzterer

$R_5$ einen Phenylrest, einen Alkylrest mit 1 bis 4 Kohlenstoffatom(en) oder einen Alkenylrest mit 3 oder 4 Kohlenstoffatomen bedeutet,

steht,

Y einen Rest der allgemeinen Formel

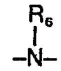 III ,

in welch letzterer

$R_6$ für Wasserstoff, einen Phenylrest, einen Alkylrest mit 1 bis 4 Kohlenstoffatom(en) oder einen Alkenylrest mit 3 bis 4 Kohlenstoffatomen steht, bedeutet,

$R_1$ die im Anspruch 17 angegebenen Bedeutungen hat,

$R_2$ Wasserstoff bedeutet und

$R_3$ und $R_4$ zusammen einen X und Y unter Bildung eines 5- bis 7-gliedrigen Ringes verbindenden Alkylenrest mit 2 bis 4 Kohlenstoffatomen oder Alkenylenrest mit 2 bis 4 Kohlenstoffatomen darstellen.

22. Erzeugnisse nach Anspruch 1, dadurch gekennzeichnet, dass

X für einen Rest der Formel –N = steht,

Y einen Rest der Formel

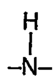 bedeutet,

$R_1$ eine das Kohlenstoffatom, an welches es gebunden ist, mit X oder Y verbindende zweite Bindung darstellt,

$R_2$ für einen Dichlormethylrest steht und

$R_3$ und $R_4$ zusammen einen Phen-1,2-ylenrest darstellen.

23. Erzeugnisse nach Anspruch 1 bis 22, dadurch gekennzeichnet, dass sie das Antidotum beziehungsweise die Antidota der allgemeinen Formel I in einer Menge von 0,01 bis 15 Gew.-%, bezogen auf das Gewicht des/der Herbizidwirkstoffe[s], enthalten und die Gesamtmenge des Antidotums beziehungsweise der Antidota und des/der Herbizidwirkstoffe[s], bezogen auf die Gesamtmenge des Erzeugnisses, 0,1 bis 95 Gew.-% beträgt, während der Rest 1 oder mehr feste[r] und/oder flüssige[r] neutrale[r] Träger und/oder anionenaktive[r], kationenaktive[r] und/oder nicht-ionogene[r] oberflächenaktive[r] Stoff(e) ist/sind.

24. Verfahren zur Herstellung von 2-(Dichlormethyl)-3-phenyl-1,3-oxazolidin bzw. 2-(Dichlormethyl)-3-allyl-1,3-oxazolidin, dadurch gekennzeichnet, dass man in an sich bekannter Weise Dichloracetaldehyd mit N-(Phenyl)-äthanolamin oder N-(Allyl)-äthanolamin in aromatischen Kohlenwasserstoffen, insbesondere Benzol, in etwa stöchiometrischen Mengenverhältnissen vermischt und mehrere Stunden unter Rückfluss zum Sieden erhitzt.

**Claims for the Contracting States BE, CH, DE, FR, GB, IT, LI, LU, NL, SE**

1. Products containing at least 1 antidot of the generic formula

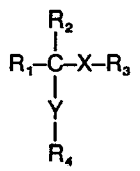

I ,

wherein

X and Y are standing independently from each other for oxygen, sulfur, a radical of the generic formula

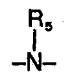

II ,

in which

$R_5$ is hydrogen, a phenyl radical optionally substituted by 1 or more alkyl- and/or mono- and/or poly haloalkyl radical(s) having 1 to 4 carbon atom(s), alkoxy radical(s) having 1 to 4 carbon atom(s) and/or halogen atom(s), an alkyl radical having 1 to 4 carbon atom(s) or an alkenyl radical having 3 or 4 carbon atoms,

and a radical of the generic formula

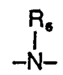

III ,

respectively, in which

$R_6$ is hydrogen, a phenyl radical optionally substituted by 1 or more alkyl- and/or mono- and/or polyhaloalkyl radical(s) having 1 to 4 carbon atom(s), alkoxy radical(s) having 1 to 4 carbon atom(s) and/or halogen atom(s), an alkyl radical having 1 to 4 carbon atom(s) or an alkenyl radical having 3 or 4 carbon atoms,

$R_1$ and $R_2$ independently from each other are hydrogen, phenyl radical(s) optionally substituted by 1 or more alkyl- and/or mono- and/or polyhaloalkyl radical(s) having 1 to 4 carbon atom(s), alkoxy radical(s) having 1 to 4 carbon atom(s) and/or halogen atom(s), furyl radical(s), phenyl alkyl radicals having 1 to 4 carbon atom(s) in the alkyl moiety, optionally substituted by 1 or more halogen atom(s), straight or branched alkyl-, mono- and polyhaloalkyl-, respectively, and mono- and polycyanoalkyl radicals, respectively, having 1 to 4 carbon atom(s) and straight or branched alkenyl radicals having 2 to 4 carbon atoms, respectively, whereby among these symbols alternatively,

if X and/or Y is standing (are standing) for a radical and radicals, respectively, of the generic formula (formulae) II or III,

$R_1$ also can be a second bond connecting the carbon atom to which it is attached with X or Y, and

$R_3$ and $R_4$ independently from each other are standing for hydrogen, straight or branched alkyl radicals having 1 to 18 carbon atom(s), mono- and polyhaloalkyl-, respectively, and mono- and polycyanoalkyl radicals, respectively, having each 1 to 4 carbon atom(s), straight or branched alkoxy alkyl- and alkoxy alkoxy alkyl radicals, respectively, having 1 to 4 carbon atom(s) in the alkyl moiety and 1 to 4 carbon atom(s) in the alkoxy moiety, and the alkoxy moieties, respectively, cycloalkyl radicals having 5 or 6 carbon atoms, phenyl alkyl radicals having 1 or 2 carbon atom(s) in the alkyl moiety, alkenyl radicals having 3 or 4 carbon atoms, alkynyl radicals having 3 or 4 carbon atoms, phenyl alkenyl radicals having 3 or 4 carbon atoms in the alkenyl moiety, dialkylamino alkyl radicals having 1 to 4 carbon atom(s) in each alkyl moiety, hydroxy alkyl radicals having 1 to 4 carbon atom(s), furfuryl radicals and tetrahydrofurfuryl radicals, respectively, or

$R_3$ and $R_4$ are together an alkylene radical having 2 to 4 carbon atoms, alkenyl radical having 2 to 4 carbon atoms or a phenyl radical, optionally substituted by 1 or more hydroxy group(s), methoxy radical(s), halogen atom(s) and/or acetoxy radical(s), connecting X and Y under formation of a 5 to 7 membered ring, or are together an X and Y connecting glucofuranosyl radical, with the additional proviso that

a) in the case that at least 1 of $R_1$ and $R_2$ is standing for a methyl-, monochlormethyl- or monochlorphenyl radical, at least 1 of $R_3$ and $R_4$ is different from a non-substituted alkyl radical having 2 to 18 carbon atoms, and

b) in the case that $R_2$ is standing for hydrogen and $R_1$ is meaning a non-substituted phenyl radical at least 1 of $R_3$ and $R_4$ is different from a methyl radical,

and 1 or more herbicidally active thiocarbamate-, carbamate-, acid amide-, urea derivative- and/or thiourea derivative ingredient(s) as combination composition for the simultaneous, separate or timed use in the field of plant protection or controlling weeds, optionally together with 1 or more conventional carrier(s) and/or adjuvant(s).

2. Products according to claim 1, characterized in that the alkyl-, mono- or polyhaloalkyl radical for which $R_1$ can stand, and/or the alkyl radical(s) for which $R_2$ and/or $R_5$ and/or $R_6$ can stand is a such one (are those ones) having 1 or 2 carbon atom(s).

3. Products according to claim 1 or 2, characterized in that the halogen atom of the mono- or polyhaloalkyl radical for which $R_1$ can stand, and/or the halogen atom(s) by which the phenyl alkyl radical for which $R_1$ can stand, and/or the phenyl radical(s) for which $R_5$ and/or $R_6$ can stand, can be substituted and/or the halogen atom by which the alkylene radical, which $R_3$ and $R_4$ together can form, can be substituted, is (are) chlorine and/or bromine.

4. Products according to claim 1, characterized in that the mono- or polyhaloalkyl radical for which $R_1$ can stand, is a chloromethyl-, dichloromethyl-, trichloromethyl-, bromomethyl-, dichloroethyl-, dibromoethyl- or dibromo-n-propyl radical.

5. Products according to claim 1, characterized in that the phenyl alkyl radical for which $R_1$ can stand is a such one having 1 or 2 carbon atom(s) in the alkyl moiety.

6. Products according to claim 1, characterized in that the alkyl- or alkoxy radical by which the phenyl radical for which $R_1$ can stand can be substituted is a such one having 1 or 2 carbon atom(s).

7. Products according to claim 1, characterized

in that the alkyl radical(s) by which the phenyl radical(s) for which $R_5$ and/or $R_6$ can stand, can be substituted is a such one (are those ones) having 1 or 2 carbon atom(s).

8. Products according to claim 1, characterized in that the alkenyl radical for which $R_1$ can stand is a such one having 3 or 4 carbon atoms.

9. Products according to claim 1, characterized in that the alkyl radical(s) for which $R_3$ and/or $R_4$ can stand is a such one (are those ones) having 1 to 12, especially 1 to 7 carbon atom(s).

10. Products according to claim 1, characterized in that the alkoxy alkyl- and/or alkoxyalkoxyalkyl radical(s) for which $R_3$ and/or $R_4$ can stand is a such one (are those ones) having 1 or 2 carbon atom(s) in the alkoxy moiety and in the alkoxy moieties, respectively.

11. Products according to claim 1, characterized in that the alkenyl radical(s) for which $R_3$ and/or $R_4$ and/or $R_5$ and/or $R_6$ can stand is (are) allyl- and/or crotyl radical(s).

12. Products according to claim 1, characterized in that the alkynyl radical(s) for which $R_3$ and/or $R_4$ can stand is (are) propargyl radical(s).

13. Products according to claim 1, characterized in that the dialkylaminoalkyl radical(s) for which $R_3$ and/or $R_4$ can stand, is a such one (are those ones) having 1 or 2 carbon atom(s) in each alkyl moiety.

14. Products according to claim 1, characterized in that the ring which can be formed by the optional connection of X and Y through the radical which $R_3$ and $R_4$ together can form, is a 5- or 6-membered one.

15. Products according to claim 1, characterized in that the alkylene radical which $R_3$ and $R_4$ together can form is a such one having 2 or 3 carbons, especially an ethylene-, prop-1,3-ylene-, prop-1,2-ylene- or but-1,2-ylene radical.

16. Products according to claim 1, characterized in that the alkenyl radical which $R_3$ and $R_4$ together can form is a such one having 2 to 4 carbon atoms, especially a but-2-ene-1,2-ylene- or propenylene radical.

17. Products according to claim 1, characterized in that

X and Y are oxygen,

$R_1$ is a phenyl radical, optionally substituted by an alkyl- or alkoxy radical having 1 to 4 carbon atom(s), a furyl radical, a phenyl alkyl radical having 1 to 4 carbon atom(s) in the alkyl moiety, optionally substituted by 1 or more halogen atom(s), a straight alkyl-, mono- or polyhaloalkyl- or monocyanoalkyl radical having 1 to 4 carbon atom(s), or a straight alkenyl radical having 2 to 4 carbon atoms,

$R_2$ is hydrogen and

$R_3$ and $R_4$ independently from each other are straight or branched alkyl radicals having 1 to 18 carbon atom(s), monohalo- and monocyanoalkyl radicals, respectively having 1 to 4 carbon atom(s), straight alkoxy alkyl- and alkoxyalkoxyalkyl radicals, respectively and having 1 to 4 carbon atom(s) in the alkyl moiety and 1 to 4 carbon atom(s) in the alkoxy moiety and in the alkoxy moieties, respectively, cycloalkyl radicals having 5 or 6 carbon atoms, phenyl alkyl radicals having 1 or 2 carbon atom(s) in the alkyl moiety, alkenyl radicals having 3 or 4 carbon atoms, an alkynyl radical having 3 or 4 carbon atoms, phenyl alkenyl radicals having 3 or 4 carbon atoms in the alkenyl moiety, dialkyl amino alkyl radicals having 1 to 4 carbon atom(s) in each alkyl moiety and furfuryl radicals, respectively, and

$R_3$ additionally can mean hydrogen, or

$R_3$ and $R_4$ together are a X or Y under formation of a 5- to 7-membered ring connecting alkylene radical having 2 to 4 carbon atoms, alkenyl radical having 2 to 4 carbon atoms, optionally substituted by 1 hydroxy group, methoxy radical, halogen atom and/or acetoxy radical, or are together an X and Y connecting glucofuranosylene radical.

18. Products according to claim 1, characterized in that

X and Y are standing for oxygen,

$R_1$ is a straight or branched alkyl-, mono- or polyhaloalkyl- or monocyanoalkyl radical having each 1 to 4 carbon atom(s), or an alkenyl radical having 2 to 4 carbon atoms,

$R_2$ is an alkyl radical having 1 to 4 carbon atom(s) or a phenyl radical, and

$R_3$ and $R_4$ have the meanings as quoted in claim 17.

19. Products according to claim 1, characterized in that

X is oxygen,

Y is a radical of the generic formula

$$\begin{array}{c} R_6 \\ | \\ -N- \end{array} \qquad III,$$

in which

$R_6$ is a phenyl- or allyl radical,

$R_1$ is a dichloromethyl radical,

$R_2$ is hydrogen, and

$R_3$ and $R_4$ are together a X and Y under formation of an oxazolidine ring connecting ethylen radical.

20. Products according to claim 1, characterized in that

X is oxygen,

Y is sulfur,

$R_1$ is a straight or branched alkyl-, mono- or polyhaloalkyl- or monocyanoalkyl radical having each 1 to 4 carbon atom(s), or an alkenyl radical having 2 to 4 carbon atoms,

$R_2$ is hydrogen and

$R_3$ and $R_4$ have the meaning as quoted in claim 17.

21. Products according to claim 1, characterized in that

X is oxygen or a radical of the generic formula

$$\begin{array}{c} R_5 \\ | \\ -N- \end{array} \qquad II,$$

in which

$R_5$ is a phenyl radical, an alkyl radical having 1 to 4 carbon atom(s) or an alkenyl radical having 3 or 4 carbon atoms,

Y is a radical of the generic formula

$$\begin{array}{c} R_6 \\ | \\ -N- \end{array} \qquad III,$$

in which

$R_6$ is hydrogen, a phenyl radical, an alkyl radical having 1 to 4 carbon atom(s) or an alkenyl radical having 3 or 4 carbon atoms,

$R_1$ has the meaning as quoted in claim 17,

$R_2$ is hydrogen and

$R_3$ and $R_4$ are together a X and Y under formation of a 5- to 7-membered ring connecting alkylene radical having 2 to 4 carbon atoms, or alkenyl radical having 2 to 4 carbon atoms.

22. Products according to claim 1, characterized in that

X is a radical of the formula $-N=$,

Y is a radical of the formula

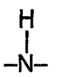

$R_1$ is a second bond connecting the carbon atom with which it is attached with X or Y,

$R_2$ is a dichloromethyl radical and

$R_3$ and $R_4$ are together a phen-1,2-ylene radical.

23. Products according to claim 1 to 22, characterized in that they contain the antidot and the antidots, respectively of the generic formula I in an amount of 0,01 to 15% by weight, based on the weight of the herbicidally active ingredient(s), and the total amount of the antidot and the antidots, respectively and of the herbicidally active ingredient(s), based on the total amount of the product, is 0,1 to 95% by weight, while the remainder is 1 or more solid and/or liquid neutral carrier(s) and/or anion active, cation active and/or non-ionic surfactant(s).

24. 2-(dichloromethyl)-3-phenyl-1,3-oxazolidine.

25. 2-(dichloromethyl)-3-allyl-1,3-oxazolidine.

26. A process for preparing the compounds according to claim 24 or 25, characterized in that in a manner known per se dichloroacetaldehyde is mixed with N-(phenyl)-ethanolamine or N-(allyl)-ethanolamine in aromatic hydrocarbons, especially benzene, in approximately stoichiometric ratios, and is heated to boiling under reflux for several hours.

**Claims for the Contracting State AT**

1. Products containing at least 1 antidot of the generic formula

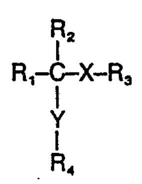

I ,

wherein

X and Y are standing independently from each other for oxygen, sulfur, a radical of the generic formula

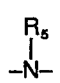

II ,

in which

$R_5$ is hydrogen, a phenyl radical optionally substituted by 1 or more alkyl- and/or mono- and/or poly haloalkyl radical(s) having 1 to 4 carbon atom(s), alkoxy radical(s) having 1 to 4 carbon atom(s) and/or halogen atom(s), an alkyl radical having 1 to 4 carbon atom(s) or an alkenyl radical having 3 or 4 carbon atoms,

and a radical of the generic formula

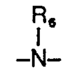

III ,

respectively, in which

$R_6$ is hydrogen, a phenyl radical optionally substituted by 1 or more alkyl- and/or mono- and/or polyhaloalkyl radical(s) having 1 to 4 carbon atom(s), alkoxy radical(s) having 1 to 4 carbon atom(s) and/or halogen atom(s), an alkyl radical having 1 to 4 carbon atom(s) or an alkenyl radical having 3 or 4 carbon atoms,

$R_1$ and $R_2$ independently from each other are hydrogen, phenyl radical(s) optionally substituted by 1 or more alkyl- and/or mono- and/or polyhaloalkyl radical(s) having 1 to 4 carbon atom(s), alkoxy radical(s) having 1 to 4 carbon atom(s) and/or halogen atom(s), furyl radical(s), phenyl alkyl radicals having 1 to 4 carbon atom(s) in the alkyl moiety, optionally substituted by 1 or more halogen atom(s), straight or branched alkyl-, mono- and polyhaloalkyl-, respectively, and mono- and polycyanoalkyl radicals, respectively, having 1 to 4 carbon atom(s) and straight or branched alkenyl radicals having 2 to 4 carbon atoms, respectively, whereby among these symbols alternatively,

if X and/or Y is standing (are standing) for a radical and radicals, respectively, of the generic formula (formulae) II or III,

$R_1$ also can be a second bond connecting the carbon atom to which it is attached with X or Y, and

$R_3$ and $R_4$ independently from each other are standing for hydrogen, straight or branched alkyl radicals having 1 to 18 carbon atom(s), mono- and polyhaloalkyl-, respectively, and mono- and polycyanoalkyl radicals, respectively, having each 1 to 4 carbon atom(s), straight or branched alkoxy alkyl- and alkoxy alkoxy alkyl radicals, respectively, having 1 to 4 carbon atom(s) in the alkyl moiety and 1 to 4 carbon atom(s) in the alkoxy moiety, and the alkoxy moieties, respectively, cycloalkyl radicals having 5 or 6 carbon atoms, phenyl alkyl radicals having 1 or 2 carbon atom(s) in the alkyl moiety, alkenyl radicals having 3 or 4 carbon atoms, alkynyl radicals having 3 or 4 carbon atoms, phenyl alkenyl radicals having 3 or 4 carbon atoms in the alkenyl moiety, dialkylamino alkyl radicals having 1 to 4 carbon atom(s) in each alkyl moiety, hydroxy alkyl radicals having 1 to 4 carbon atom(s), furfuryl radicals and tetrahydrofurfuryl radicals, respectively, or

$R_3$ and $R_4$ are together an alkylene radical having 2 to 4 carbon atoms, alkenyl radical having 2 to 4 carbon atoms or a phenyl radical, optionally substituted by 1 or more hydroxy group(s), methoxy radical(s), halogen atom(s) and/or acetoxy radical(s), connecting X and Y under formation of a 5 to 7 membered ring, or are together an X and Y connecting glucofuranosyl radical, with the additional proviso that

a) in the case that at least 1 of $R_1$ and $R_2$ is standing for a methyl-, monochlormethyl- or monochlorphenyl radical, at least 1 of $R_3$ and $R_4$

is different from a non-substituted alkyl radical having 2 to 18 carbon atoms, and

b) in the case that $R_2$ is standing for hydrogen and $R_1$ is meaning a non-substituted phenyl radical at least 1 of $R_3$ and $R_4$ is different from a methyl radical,

and 1 or more herbicidally active thiocarbamate-, carbamate-, acid amide-, urea derivative- and/or thiourea derivative ingredient(s) as combination composition for the simultaneous, separate or timed use in the field of plant protection or controlling weeds, optionally together with 1 or more conventional carrier(s) and/or adjuvant(s).

2. Products according to claim 1, characterized in that the alkyl-, mono- or polyhaloalkyl radical for which $R_1$ can stand, and/or the alkyl radical(s) for which $R_2$ and/or $R_5$ and/or $R_6$ can stand is a such one (are those ones) having 1 or 2 carbon atom(s).

3. Products according to claim 1 or 2, characterized in that the halogen atom of the mono- or polyhaloalkyl radical for which $R_1$ can stand, and/or the halogen atom(s) by which the phenyl alkyl radical for which $R_1$ can stand, and/or the phenyl radical(s) for which $R_5$ and/or $R_6$ can stand, can be substituted and/or the halogen atom by which the alkylene radical, which $R_3$ and $R_4$ together can form, can be substituted, is (are) chlorine and/or bromine.

4. Products according to claim 1, characterized in that the mono- or polyhaloalkyl radical for which $R_1$ can stand, is a chloromethyl-, dichloromethyl-, trichloromethyl-, bromomethyl-, dichloroethyl-, dibromoethyl- or dibromo-n-propyl radical.

5. Products according to claim 1, characterized in that the phenyl alkyl radical for which $R_1$ can stand is a such one having 1 or 2 carbon atom(s) in the alkyl moiety.

6. Products according to claim 1, characterized in that the alkyl- or alkoxy radical by which the phenyl radical for which $R_1$ can stand can be substituted is a such one having 1 or 2 carbon atom(s).

7. Products according to claim 1, characterized in that the alkyl radical(s) by which the phenyl radical(s) for which $R_5$ and/or $R_6$ can stand, can be substituted is a such one (are those ones) having 1 or 2 carbon atom(s).

8. Products according to claim 1, characterized in that the alkenyl radical for which $R_1$ can stand ist a such one having 3 or 4 carbon atoms.

9. Products according to claim 1, characterized in that the alkyl radical(s) for which $R_3$ and/or $R_4$ can stand is a such one (are those ones) having 1 to 12, especially 1 to 7 carbon atom(s).

10. Products according to claim 1, characterized in that the alkoxy alkyl- and/or alkoxyalkoxyalkyl radical(s) for which $R_3$ and/or $R_4$ can stand is a such one (are those ones) having 1 or 2 carbon atom(s) in the alkoxy moiety and in the alkoxy moieties, respectively.

11. Products according to claim 1, characterized in that the alkenyl radical(s) for which $R_3$ and/or $R_4$ and/or $R_5$ and/or $R_6$ can stand is (are) allyl- and/or crotyl radical(s).

12. Products according to claim 1, characterized in that the alkynyl radical(s) for which $R_3$ and/or $R_4$ can stand is (are) propargyl radical(s).

13. Products according to claim 1, characterized in that the dialkylaminoalkyl radical(s) for which $R_3$ and/or $R_4$ can stand, is a such one (are those ones) having 1 or 2 carbon atom(s) in each alkyl moiety.

14. Products according to claim 1, characterized in that the ring which can be formed by the optional connection of X and Y through the radical which $R_3$ and $R_4$ together can form, is a 5- or 6-membered one.

15. Products according to claim 1, characterized in that the alkylene radical which $R_3$ and $R_4$ together can form is a such one having 2 or 3 carbons, especially an ethylene-, prop-1,3-ylene-, prop-1,2-ylene or but-1,2-ylene radical.

16. Products according to claim 1, characterized in that the alkenyl radical which $R_3$ and $R_4$ together can form is a such one having 2 to 4 carbon atoms, especially a but-2-ene-1,2-ylene- or propenylene radical.

17. Products according to claim 1, characterized in that

X and Y are oxygen,

$R_1$ is a phenyl radical, optionally substituted by an alkyl- or alkoxy radical having 1 to 4 carbon atom(s), a furyl radical, a phenyl alkyl radical having 1 to 4 carbon atom(s) in the alkyl moiety, optionally substituted by 1 or more halogen atom(s), a straight alkyl-, mono- or polyhaloalkyl- or monocyanoalkyl radical having 1 to 4 carbon atom(s), or a straight alkenyl radical having 2 to 4 carbon atoms,

$R_2$ is hydrogen and

$R_3$ and $R_4$ independently from each other are straight or branched alkyl radicals having 1 to 18 carbon atom(s), monohalo- and monocyanoalkyl radicals, respectively having 1 to 4 carbon atom(s), straight alkoxy alkyl- and alkoxyalkoxyalkyl radicals, respectively and having 1 to 4 carbon atom(s) in the alkyl moiety and 1 to 4 carbon atom(s) in the alkoxy moiety and in the alkoxy moieties, respectively, cycloalkyl radicals having 5 or 6 carbon atoms, phenyl alkyl radicals having 1 or 2 carbon atom(s) in the alkyl moiety, alkenyl radicals having 3 or 4 carbon atoms, an alkynyl radical having 3 or 4 carbon atoms, phenyl alkenyl radicals having 3 or 4 carbon atoms in the alkenyl moiety, dialkyl amino alkyl radicals having 1 to 4 carbon atom(s) in each alkyl moiety and furfuryl radicals, respectively, and

$R_3$ additionally can mean hydrogen, or

$R_3$ and $R_4$ together are a X or Y under formation of a 5- to 7-membered ring connecting alkylene radical having 2 to 4 carbon atoms, alkenyl radical having 2 to 4 carbon atoms, optionally substituted by 1 hydroxy group, methoxy radical, halogen atom and/or acetoxy radical, or are together an X and Y connecting glucofuranosylene radical.

18. Products according to claim 1, characterized in that

X and Y are standing for oxygen,

$R_1$ is a straight or branched alkyl-, mono- or polyhaloalkyl- or monocyanoalkyl radical having

each 1 to 4 carbon atom(s), or an alkenyl radical having 2 to 4 carbon atoms,

$R_2$ is an alkyl radical having 1 to 4 carbon atom(s) or a phenyl radical, and

$R_3$ and $R_4$ have the meanings as quoted in claim 17.

19. Products according to claim 1, characterized in that

X is oxygen,

Y is a radical of the generic formula

$R_6$
|
–N– III ,

in which

$R_6$ is a phenyl- or allyl radical,

$R_1$ is a dichloromethyl radical,

$R_2$ is hydrogen, and

$R_3$ and $R_4$ are together a X and Y under formation of an oxazolidine ring connecting ethylene radical.

20. Products according to claim 1, characterized in that

X is oxygen,

Y is sulfur,

$R_1$ is a straight or branched alkyl-, mono- or polyhaloalkyl- or monocyanoalkyl radical having each 1 to 4 carbon atom(s), or an alkenyl radical having 2 to 4 carbon atoms,

$R_2$ is hydrogen and

$R_3$ and $R_4$ have the meaning as quoted in claim 17.

21. Products according to claim 1, characterized in that

X is oxygen or a radical of the generic formula

$R_5$
|
–N– II ,

in which

$R_5$ is a phenyl radical, an alkyl radical having 1 to 4 carbon atom(s) or an alkenyl radical having 3 or 4 carbon atoms,

Y is a radical of the generic formula

$R_6$
|
–N– III ,

in which

$R_6$ is hydrogen, a phenyl radical, an alkyl radical having 1 to 4 carbon atom(s) or an alkenyl radical having 3 or 4 carbon atoms,

$R_1$ has the meaning as quoted in claim 17,

$R_2$ is hydrogen and

$R_3$ and $R_4$ are together a X and Y under formation of a 5- to 7-membered ring connecting alkylene radical having 2 to 4 carbon atoms, or alkenyl radical having 2 to 4 carbon atoms.

22. Products according to claim 1, characterized in that

X is a radical of the formula –N=,

Y is a radical of the formula

H
|
–N–

$R_1$ is a second bond connecting the carbon atom with which it is attached with X or Y,

$R_2$ is a dichloromethyl radical and

$R_3$ and $R_4$ are together a phen-1,2-ylene radical.

23. Products according to claim 1 to 22, characterized in that they contain the antidot and the antidots, respectively of the generic formula I in an amount of 0,01 to 15% by weight, based on the weight of the herbicidally active ingredient(s), and the total amount of the antidot and the antidots, respectively and of the herbicidally active ingredient(s), based on the total amount of the product, is 0,1 to 95% by weight, while the remainder is 1 or more solid and/or liquid neutral carrier(s) and/or anion active, cation active and/or non-ionic surfactant(s).

24. A process for preparing 2-(dichloromethyl)-3-phenyl-1,3-oxazolidine and 2-(dichloromethyl)-3-allyl-1,3-oxazolidine, respectively, characterized in that in a manner known per se dichloroacetaldehyde is mixed with N-(phenyl)-ethanolamine or N-(allyl)-methanolamine in aromatic hydrocarbons, especially benzene, in approximately stoichiometric amounts, and is heated to boiling under reflux for several hours.

**Revendications pour les Etats contractants BE, CH, DE, FR, GB, IT, LI, LU, NL, SE**

1. Produit renfermant au moins 1 antidote répondant à la formule générale

$R_2$
|
$R_1$–C–X–$R_3$ I ,
|
Y
|
$R_4$

dans laquelle:

X et Y représentent, indépendamment l'un de l'autre, l'oxygène, le soufre, un reste répondant à la formule générale

$R_5$
|
–N– II ,

dans laquelle

$R_5$ représente l'hydrogène, un reste phényle éventuellement substitué par un ou plusieurs reste(s) alcoyle et/ou mono- et/ou poly-halo-alcoyle comportant de 1 à 4 atome(s) de carbone, un ou plusieurs reste(s) alkoxy comportant de 1 à 4 atome(s) de carbone et/ou un ou plusieurs atome(s) d'halogène, un reste alcoyle comportant 1 à 4 atome(s) de carbone ou un reste alcényle comportant 3 ou 4 atomes de carbone,

et/ou un reste répondant à la formule générale

$R_6$
|
–N– III ,

dans laquelle

$R_6$ représente l'hydrogène, un reste phényle éventuellement substitué par un ou plusieurs reste(s) alcoyle et/ou mono- et/ou poly-haloalcoyle comportant de 1 à 4 atome(s) de carbone, un ou plusieurs reste(s) alkoxy comportant de 1 à 4 atome(s) de carbone et/ou un ou plusieurs atome(s) d'halogène, un reste alcoyle comportant de 1 à 4 atome(s) de carbone ou un reste alcényle comportant 3 ou 4 atomes de carbone,

$R_1$ et $R_2$ représentent, indépendamment l'un de l'autre, l'hydrogène, un ou plusieurs reste(s) phényle, éventuellement substitué(s) par un ou plusieurs reste(s) alcoyle et/ou mono- et/ou polyhalo-alcoyle comportant de 1 à 4 atome(s) de carbone, un ou plusieurs reste(s) alkoxy comportant de 1 à 4 atome(s) de carbone et/ou un ou plusieurs atome(s) d'halogène, un ou plusieurs reste(s) furyle, des restes phénylalcoyle dont la portion alcoyle comporte de 1 à 4 atome(s) de carbone, éventuellement substitués par un ou plusieurs atome(s) d'halogène, des restes alcoyle, mono- ou poly-halo-alcoyle, ou mono- ou polycyanalcoyle linéaires ou ramifiés, comportant de 1 à 4 atome(s) de carbone, et/ou bien des restes alcényle linéaires ou ramifiés comportant de 2 à 4 atomes de carbone et, parmi ces symboles, au choix, dans le cas où X et/ou Y représente(nt) un reste ou des restes répondant à la ou aux formule(s) générale(s) II et/ou III,

$R_1$ pouvant aussi représenter une seconde liaison liant l'atome de carbone auquel il est fixé à X ou Y, et

$R_3$ et $R_4$ représentent, indépendamment l'un de l'autre, l'hydrogène, des restes alcoyle linéaires ou ramifiés comportant 1 à 18 atome(s) de carbone, des restes mono- ou poly-halo-alcoyle ou bien mono- ou polycyanalcoyle comportant, dans chaque cas, de 1 à 4 atome(s) de carbone, des restes alkoxyalcoyle ou alkoxyalkoxyalcoyle comportant de 1 à 4 atome(s) de carbone dans la portion alcoyle et 1 à 4 atome(s) de carbone dans la portion alkoxy ou dans les portions alkoxy, des restes cycloalcoyle comportant 5 ou 6 atomes de carbone, des restes phénylalcoyle comportant 1 ou 2 atome(s) de carbone dans la portion alcoyle, des restes alcényle comportant 3 ou 4 atomes de carbone, des restes alcynyle comportant 3 ou 4 atomes de carbone, des restes phénylalcényle comportant 3 ou 4 atomes de carbone dans la portion alcényle, des restes dialcoylaminoalcoyle comportant 1 à 4 atome(s) de carbone dans chaque portion alcoyle, des restes hydroxyalcoyle comportant 1 à 4 atome(s) de carbone, des restes furfuryle ou des restes tétrahydrofurfuryle, ou bien

$R_3$ et $R_4$ représentent ensemble un reste alkylène comportant de 2 à 4 atomes de carbone, éventuellement substitué par un ou plusieurs groupe(s) hydroxy, un ou plusieurs reste(s) méthoxy, un ou plusieurs atome(s) d'halogène et/ou un ou plusieurs reste(s) acétoxy, un reste alkylène comportant de 2 à 4 atomes de carbone, un reste alcénylène comportant de 2 à 4 atomes de carbone ou un reste phénylène, liant X et Y avec formation d'un noyau (anneau) à cinq à sept maillons, ou bien représentent ensemble un reste glucofuranosylène liant X et Y, avec, pour autres mesures, que

a) dans le cas où l'un au moins des symboles $R_1$ et $R_2$ représente un reste méthyle, monochlorométhyle ou monochlorophényle, l'un au moins des symboles $R_3$ et $R_4$ est différent d'un reste alcoyle non substitué comportant de 2 à 18 atomes de carbone, et

b) dans le cas où $R_2$ représente l'hydrogène et $R_1$ représente un reste phényle non substitué, l'un au moins des symboles $R_3$ et $R_4$ est différent d'un reste méthyle,

et une ou plusieurs substance(s) active(s) herbicides à base de dérivés de thiocarbamates, de carbamates, d'amides d'acide et d'urée, et/ou de dérivés de thiourée, pour constituer en combinaison un produit à appliquer simultanément, séparément ou de façon étagée dans le temps pour protéger les plantes ou combattre les mauvaises herbes, de façon appropriée en même temps qu'une ou plusieurs substance(s) de support et/ou auxiliaire(s) usuelles.

2. Produit selon la revendication 1, caractérisé en ce que le reste alcoyle, mono- ou polyhaloalcoyle, que peut représenter $R_1$, et/ou le/les reste(s) alcoyle que peut/peuvent représenter $R_2$ et/ou $R_5$ et/ou $R_6$, est/sont un/des reste(s) comportant 1 ou 2 atome(s) de carbone.

3. Produit selon la revendication 1 ou 2, caractérisé en ce que l'atome d'halogène du reste mono- ou polyhalo-alcoyle que peut représenter $R_1$, et/ou l'atome ou les atomes d'halogène pouvant être un ou des substituant(s) du reste phénylalcoyle que peut représenter $R_1$ et/ou le/les reste(s) phényle que peut/peuvent représenter $R_5$ et/ou $R_6$ et/ou l'atome d'halogène pouvant être un ou des substituant(s) du reste alkylène que peuvent représenter ensemble $R_3$ et $R_4$ est ou sont le chlore et/ou le brome.

4. Produit selon la revendication 1, caractérisé en ce que le reste mono- ou polyhalo-alcoyle que peut représenter $R_1$ est un reste chlorométhyle, dichlorométhyle, trichlorométhyle, bromométhyle, dichloroéthyle, dibromoéthyle ou dibromo-n-propyle.

5. Produit selon la revendication 1, caractérisé en ce que le reste phénylalcoyle que peut représenter $R_1$ est un reste dont la portion alcoyle comporte 1 ou 2 atome(s) de carbone.

6. Produit selon la revendication 1, caractérisé en ce que le reste alcoyle ou alkoxy pouvant être un substituant du reste phényle que peut représenter $R_1$ est un reste comportant 1 ou 2 atome(s) de carbone.

7. Produit selon la revendication 1, caractérisé en ce que le ou les reste(s) alcoyle pouvant être un ou des substituant(s) du/des reste(s) phényle que peut/peuvent représenter $R_5$ et/ou $R_6$, est/sont un reste/des restes comportant 1 ou 2 atome(s) de carbone.

8. Produit selon la revendication 1, caractérisé en ce que le reste alcényle que peut représenter $R_1$ est un reste alcényle comportant 3 ou 4 atomes de carbone.

9. Produit selon la revendication 1, caractérisé en ce que le/les reste(s) alcoyle que peut/peuvent représenter $R_3$ et/ou $R_4$ est/sont un reste/des restes comportant 1 à 12, en particulier 1 à 7 atome(s) de carbone.

10. Produit selon la revendication 1, caractérisé en ce que le/les reste(s) alkoxyalcoyle et/ou alkoxyalkoxyalcoyle que peut/peuvent représenter $R_3$ et/ou $R_4$ sont un/des reste(s) comportant 1 ou 2 atome(s) de carbone dans la portion alkoxy ou les portions alkoxy.

11. Produit selon la revendication 1, caractérisé en ce que le/les reste(s) alcényle que peut/peuvent représenter $R_3$ et/ou $R_4$ et/ou $R_5$ et/ou $R_6$, est/sont un ou des reste(s) allyle et/ou crotyle.

12. Produit selon la revendication 1, caractérisé en ce que le ou les reste(s) alcynyle que peut ou peuvent représenter $R_3$ et/ou $R_4$ est/sont un ou des reste(s) propargyle.

13. Produit selon la revendication 1, caractérisé en ce que le/les reste(s) dialcoylaminoalcoyle que peut/peuvent représenter $R_3$ et/ou $R_4$ sont un ou des reste(s) comportant 1 ou 2 atome(s) de carbone dans chaque portion alcoyle.

14. Produit selon la revendication 1, caractérisé en ce que le noyau (ou anneau) pouvant être formé par la liaison éventuelle de X et Y par le reste que $R_3$ et $R_4$ peuvent représenter ensemble, comporte 5 ou 6 maillons.

15. Produit selon la revendication 1, caractérisé en ce que le reste alkylène que peuvent représenter ensemble $R_3$ et $R_4$ est un reste comportant 2 ou 3 atomes de carbone, tout particulièrement un reste éthylène, prop-1,3-ylène, prop-1,2-ylène ou but-1,2-ylène.

16. Produit selon la revendication 1, caractérisé en ce que le reste alcényle que peuvent représenter ensemble $R_3$ et $R_4$ est un reste comportant 2 à 4 atomes de carbone, en particulier un reste but-2-ène, but-1,2-ylène ou propénylène.

17. Produit selon la revendication 1, caractérisé en ce que

X et Y représentent l'oxygène,

$R_1$ représente un reste phényle éventuellement substitué par un reste alcoyle ou alkoxy comportant 1 à 4 atome(s) de carbone, un reste furyle, un reste phénylalcoyle comportant 1 à 4 atome(s) de carbone dans la portion alcoyle,
éventuellement substitué par un ou plusieurs atome(s) d'halogène, un reste alcoyle, mono- ou poly-halo-alcoyle ou monocyanalcoyle à chaîne linéaire comportant 1 à 4 atome(s) de carbone ou un reste alcényle à chaîne linéaire comportant 2 à 4 atomes de carbone,

$R_2$ représente l'hydrogène,

$R_3$ et $R_4$ représentent, indépendamment l'un de l'autre, des restes alcoyle à chaîne linéaire ou ramifiée comportant 1 à 18 atome(s) de carbone, des restes monohalo- ou monocyanalcoyle comportant 1 à 4 atome(s) de carbone, des restes alkoxyalcoyle ou alkoxyalkoxyalcoyle à chaîne linéaire comportant 1 à 4 atome(s) de carbone dans la portion alcoyle et 1 à 4 atome(s) de carbone dans la portion alkoxy ou dans les portions alkoxy, des restes cycloalcoyle comportant 5 à 6 atomes de carbone, des restes phénylalcoyle comportant 1 ou 2 atome(s) de carbone dans la portion alcoyle, des restes alcényle comportant 3 ou 4 atomes de carbone, un reste alcynyle comportant 3 ou 4 atomes de carbone, des restes phénylalcényle comportant 3 ou 4 atomes de carbone dans la portion alcényle, des restes dialcoylaminoalcoyle comportant 1 à 4 atome(s) de carbone dans chaque portion alcoyle, ou des restes furfuryles, et

$R_3$ peut représenter, en plus, à volonté, de l'hydrogène,

$R_3$ et $R_4$ peuvent représenter ensemble un reste alkylène comportant 2 à 4 atomes de carbone, éventuellement substitué par un groupe hydroxy, un reste méthoxy, un atome d'halogène et/ou un reste acétoxy, un reste alcényle comportant 2 à 4 atomes de carbone, liant X et Y avec formation d'un noyau (anneau) à 5 à 7 maillons, ou bien, ensemble, un reste glucofuranosylène liant X et Y.

18. Produit selon la revendication 1, caractérisé en ce que

X et Y représentent l'oxygène,

$R_1$ représente un reste alcoyle, mono- ou poly-halo-alcoyle, ou un reste monocyanalcoyle, linéaire ou ramifié, comportant dans chaque cas de 1 à 4 atome(s) de carbone, ou bien un reste alcényle comportant de 2 à 4 atomes de carbone,

$R_2$ représente un reste alcoyle comportant de 1 à 4 atome(s) de carbone ou un reste phényle, et

$R_3$ et $R_4$ ont la signification indiquée dans la revendication 17.

19. Produit selon la revendication 1, caractérisé en ce que

X représente l'oxygène,

Y représente un reste répondant à la formule générale

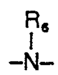 III ,

dans laquelle

$R_6$ représente un reste phényle ou allyle,

$R_1$ représente un reste dichlorométhyle,

$R_2$ représente l'hydrogène, et

$R_3$ et $R_4$ représentent ensemble un reste éthylénique liant X et Y avec formation d'un noyau oxazolidine.

20. Produit selon la revendication 1, caractérisé en ce que

X représente l'oxygène,

Y représente le soufre,

$R_1$ représente un reste alcoyle, mono- ou poly-halo-alcoyle ou monocyanalcoyle à chaîne linéaire ou ramifiée comportant, dans chaque cas, de 1 à 4 atome(s) de carbone, ou bien
un reste alcényle comportant de 2 à 4 atomes de carbone,

$R_2$ représente l'hydrogène, et

$R_3$ et $R_4$ ont la signification indiquée dans la revendication 17.

21. Produit selon la revendication 1, caractérisé en ce que

X représente l'oxygène ou un reste répondant à la formule générale

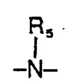 II ,

dans laquelle

$R_5$ représente un reste phényle, un reste alcoyle comportant de 1 à 4 atome(s) de carbone ou un reste alcényle comportant 3 ou 4 atomes de carbone,

Y représente un reste répondant à la formule générale

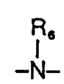 III ,

dans laquelle

$R_6$ représente l'hydrogène, un reste phényle, un reste alcoyle comportant de 1 à 4 atome(s) de

carbone ou un reste alcényle comportant 3 ou 4 atomes de carbone,

$R_1$ a les significations indiquées dans la revendication 17,

$R_2$ représente l'hydrogène, et

$R_3$ et $R_4$ représentent ensemble un reste alkylène comportant 2 à 4 atomes de carbone ou un reste alcénylène comportant 2 à 4 atomes de carbone, liant X et Y avec formation d'un noyau à 5 à 7 maillons.

22. Produit selon la revendication 1, caractérisé en ce que

X représente un reste répondant à la formule –N=,

Y représente un reste répondant à la formule

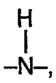

,

$R_1$ représente une seconde liaison liant l'atome de carbone auquel il est fixé à X ou Y,

$R_2$ représente un reste dichlorométhyle, et

$R_3$ et $R_4$ représentent ensemble un reste phén-1,2-ylène.

23. Produit selon les revendications 1 à 22, caractérisé en ce qu'il renferme l'antidote ou les antidotes répondant à la formule générale I dans une proportion de 0,01 à 15% en poids, rapportée au poids de la ou des substance(s) active(s) herbicides, et la proportion total de l'antidote ou des antidotes et de la ou des substance(s) active(s) herbicide(s), rapportée à la quantité totale du produit, est de 0,1 à 95% en poids, tandis que le reste consiste en un ou plusieurs support(s) neutre(s) solide(s) et/ou liquide(s) et/ou une ou plusieurs substance(s) tensio-active(s) anionique(s), cathionique(s) et/ou non-ionique(s).

24. 2-(dichlorométhyl)-3-phényl-1,3-oxazolidine.

25. 2-(dichlorométhyl)-3-allyl-1,3-oxazolidine.

26. Procédé de préparation de composé selon la revendication 24 ou 25, caractérisé en ce que, de façon connue en soi, on mélange du dichloracétaldéhyde avec de la N-(phényl)-éthanolamine ou de la N-(allyl)-éthanolamine dans des hydrocarbures aromatiques, en particulier du benzène, dans des proportions à peu près stoéchiométriques, et l'on chauffe au reflux à l'ebullition plusieurs heures.

**Revendications pour l'Etat contractant AT**

1. Produit renfermant au moins 1 antidote répondant à la formule générale

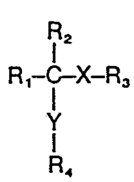

I ,

dans laquelle:

X et Y représentent, indépendamment l'un de l'autre, l'oxygène, le soufre, un reste répondant à la formule générale

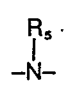

II ,

dans laquelle

$R_5$ représente l'hydrogène, un reste phényle éventuellement substitué par un ou plusieurs reste(s) alcoyle et/ou mono- et/ou poly-halo-alcoyle comportant de 1 à 4 atome(s) de carbone, un ou plusieurs reste(s) alkoxy comportant de 1 à 4 atome(s) de carbone et/ou un ou plusieurs atome(s) d'halogène, un reste alcoyle comportant 1 à 4 atome(s) de carbone ou un reste alcényle comportant 3 ou 4 atomes de carbone,

et/ou un reste répondant à la formule generale

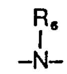

III ,

dans laquelle

$R_6$ représente l'hydrogène, un reste phényle éventuellement substitué par un ou plusieurs reste(s) alcoyle et/ou mono- et/ou poly-haloalcoyle comportant de 1 à 4 atome(s) de carbone, un ou plusieurs reste(s) alkoxy comportant de 1 à 4 atome(s) de carbone et/ou un ou plusieurs atome(s) d'halogène, un reste alcoyle comportant de 1 à 4 atome(s) de carbone ou un reste alcényle comportant 3 ou 4 atomes de carbone,

$R_1$ et $R_2$ représentent, indépendamment l'un de l'autre, l'hydrogène, un ou plusieurs reste(s) phényle, éventuellement substitué(s) par un ou plusieurs reste(s) alcoyle et/ou mono- et/ou poly-halo-alcoyle comportant de 1 à 4 atome(s) de carbone, un ou plusieurs reste(s) alkoxy comportant de 1 à 4 atome(s) de carbone et/ou un ou plusieurs atome(s) d'halogène, un ou plusieurs reste(s) furyle, des restes phénylalcoyle dont la portion alcoyle comporte de 1 à 4 atome(s) de carbone, éventuellement substitués par un ou plusieurs atome(s) d'halogène, des restes alcoyle, mono- ou poly-halo-alcoyle, ou mono- ou polycyanalcoyle linéaires ou ramifiés, comportant de 1 à 4 atome(s) de carbone, et/ou bien des restes alcényle linéaires ou ramifiés comportant de 2 à 4 atomes de carbone et, parmi ces symboles, au choix, dans le cas où X et/ou Y représente(nt) un reste ou des restes répondant à la ou aux formule(s) générale(s) II et/ou III,

$R_1$ pouvant aussi représenter une seconde liaison liant l'atome de carbone auquel il est fixé à X ou Y, et

$R_3$ et $R_4$ représentent, indépendamment l'un de l'autre, l'hydrogène, des restes alcoyle linéaires ou ramifiés comportant 1 à 18 atome(s) de carbone, des restes mono- ou poly-halo-alcoyle ou bien mono- ou polycyanalcoyle comportant, dans chaque cas, de 1 à 4 atome(s) de carbone, des restes alkoxyalcoyle ou alkoxyalkoxyalcoyle comportant de 1 à 4 atome(s) de carbone dans la portion alcoyle et 1 à 4 atome(s) de carbone dans la portion alkoxy ou dans les portions alkoxy, des restes cycloalcoyle comportant 5 ou 6 atomes de carbone, des restes phénylalcoyle comportant 1 ou 2 atome(s) de carbone dans la portion alcoyle, des restes alcényle comportant 3 ou 4 atomes de carbone, des restes alcynyle comportant 3 ou 4 atomes de carbone, des restes phénylalcényle comportant 3 ou 4 atomes de carbone dans la portion alcényle, des restes dialcoylaminoalcoyle

comportant 1 à 4 atome(s) de carbone dans chaque portion alcoyle, des restes hydroxyalcoyle comportant 1 à 4 atome(s) de carbone, des restes furfuryle ou des restes tétrahydrofurfuryle, ou bien

$R_3$ et $R_4$ représentent ensemble un reste alkylène comportant de 2 à 4 atomes de carbone, éventuellement substitué par un ou plusieurs groupe(s) hydroxy, un ou plusieurs reste(s) méthoxy, un ou plusieurs atome(s) d'halogène et/ou un ou plusieurs reste(s) acétoxy, un reste alkylène comportant de 2 à 4 atomes de carbone, un reste alcénylène comportant de 2 à 4 atomes de carbone ou un reste phénylène, liant X et Y avec formation d'un noyau (anneau) à cinq à sept maillons, ou bien représentent ensemble un reste glucofuranosylène liant X et Y, avec, pour autres mesures, que

a) dans le cas où l'un au moins des symboles $R_1$ et $R_2$ représente un reste méthyle, monochlorométhyle ou monochlorophényle,
l'un au moins des symboles $R_3$ et $R_4$ est différent d'un reste alcoyle non substitué comportant de 2 à 18 atomes de carbone, et

b) dans le cas où $R_2$ représente l'hydrogène et $R_1$ représente un reste phényle non substitué, l'un au moins des symboles $R_3$ et $R_4$ est différent d'un reste méthyle,
et une ou plusieurs substance(s) active(s) herbicides à base de dérivés de thiocarbamates, de carbamates, d'amides d'acide et d'urée, et/ou de dérivés de thiourée, pour constituer en combinaison un produit à appliquer simultanément, séparément ou de façon étagée dans le temps pour protéger les plantes ou combattre les mauvaises herbes, de façon appropriée en même temps qu'une ou plusieurs substance(s) de support et/ou auxiliaire(s) usuelles.

2. Produit selon la revendication 1, caractérisé en ce que le reste alcoyle, mono- ou polyhaloalcoyle, que peut représenter $R_1$, et/ou le/les reste(s) alcoyle que peut/peuvent représenter $R_2$ et/ou $R_5$ et/ou $R_6$, est/sont un/des reste(s) comportant 1 ou 2 atome(s) de carbone.

3. Produit selon la revendication 1 ou 2, caractérisé en ce que l'atome d'halogène du reste mono- ou polyhalo-alcoyle que peut représenter $R_1$, et/ou l'atome ou les atomes d'halogène pouvant être un ou des substituant(s) du reste phénylalcoyle que peut représenter $R_1$ et/ou le/les reste(s) phényle que peut/peuvent représenter $R_5$ et/ou $R_6$ et/ou l'atome d'halogène pouvant être un ou des substituant(s) du reste alkylène que peuvent représenter ensemble $R_3$ et $R_4$ est ou sont le chlore et/ou le brome.

4. Produit selon la revendication 1, caractérisé en ce que le reste mono- ou polyhalo-alcoyle que peut représenter $R_1$ est un reste chlorométhyle, dichlorométhyle, trichlorométhyle, bromométhyle, dichloroéthyle, dibromoéthyle ou dibromo-n-propyle.

5. Produit selon la revendication 1, caractérisé en ce que le reste phénylalcoyle que peut représenter $R_1$ est un reste dont la portion alcoyle comporte 1 ou 2 atome(s) de carbone.

6. Produit selon la revendication 1, caractérisé en ce que le reste alcoyle ou alkoxy pouvant être un substituant du reste phényle que peut représenter $R_1$ est un reste comportant 1 ou 2 atome(s) de carbone.

7. Produit selon la revendication 1, caractérisé en ce que le ou les reste(s) alcoyle pouvant être un ou des substituant(s) du/des reste(s) phényle que peut/peuvent représenter $R_5$ et/ou $R_6$, est/sont un reste/des restes comportant 1 ou 2 atome(s) de carbone.

8. Produit selon la revendication 1, caractérisé en ce que le reste alcényle que peut représenter $R_1$ est un reste alcényle comportant 3 ou 4 atomes de carbone.

9. Produit selon la revendication 1, caractérisé en ce que le/les reste(s) alcoyle que peut/peuvent représenter $R_3$ et/ou $R_4$ est/sont un reste/des restes comportant 1 à 12, en particulier 1 à 7 atome(s) de carbone.

10. Produit selon la revendication 1, caractérisé en ce que le/les reste(s) alkoxyalcoyle et/ou alkoxyalkoxyalcoyle que peut/peuvent représenter $R_3$ et/ou $R_4$ sont un/des reste(s) comportant 1 ou 2 atome(s) de carbone dans la portion alkoxy ou les portions alkoxy.

11. Produit selon la revendication 1, caractérisé en ce que le/les reste(s) alcényle que peut/peuvent représenter $R_3$ et/ou $R_4$ et/ou $R_5$ et/ou $R_6$, est/sont un ou des reste(s) allyle et/ou crotyle.

12. Produit selon la revendication 1, caractérisé en ce que le ou les reste(s) alcynyle que peut ou peuvent représenter $R_3$ et/ou $R_4$ est/sont un ou des reste(s) propargyle.

13. Produit selon la revendication 1, caractérisé en ce que le/les reste(s) dialcoylaminoalcoyle que peut/peuvent représenter $R_3$ et/ou $R_4$ sont un ou des reste(s) comportant 1 ou 2 atome(s) de carbone dans chaque portion alcoyle.

14. Produit selon la revendication 1, caractérisé en ce que le noyau (ou anneau) pouvant être formé par la liaison éventuelle de X et Y par le reste que $R_3$ et $R_4$ peuvent représenter ensemble, comporte 5 ou 6 maillons.

15. Produit selon la revendication 1, caractérisé en ce que le reste alkylène que peuvent représenter ensemble $R_3$ et $R_4$ est un reste comportant 2 ou 3 atomes de carbone, tout particulièrement un reste éthylène, prop-1,3-ylène, prop-1,2-ylène ou but-1,2-ylène.

16. Produit selon la revendication 1, caractérisé en ce que le reste alcényle que peuvent représenter ensemble $R_3$ et $R_4$ est un reste comportant 2 à 4 atomes de carbone, en particulier un reste but-2-ène, but-1,2-ylène ou propénylène.

17. Produit selon la revendication 1, caractérisé en ce que

X et Y représentent l'oxygène,

$R_1$ représente un reste phényle éventuellement substitué par un reste alcoyle ou alkoxy comportant 1 à 4 atome(s) de carbone, un reste furyle, un reste phénylalcoyle comportant 1 à 4 atome(s) de carbone dans la portion alcoyle,
éventuellement substitué par un ou plusieurs atome(s) d'halogène, un reste alcoyle, mono- ou poly-halo-alcoyle ou monocyanalcoyle à chaîne linéaire comportant 1 à 4 atome(s) de carbone ou un reste alcényle à chaîne linéaire comportant 2 à 4 atomes de carbone,

$R_2$ représente l'hydrogène,

$R_3$ et $R_4$ représentent, indépendamment l'un de l'autre, des restes alcoyle à chaîne linéaire ou ramifiée comportant 1 à 18 atome(s) de carbone, des restes monohalo- ou monocyanalcoyle comportant 1 à 4 atome(s) de carbone, des restes alkoxyalcoyle ou alkoxyalkoxyalcoyle à chaîne linéaire comportant 1 à 4 atome(s) de carbone dans la portion alcoyle et 1 à 4 atome(s) de carbone dans la portion alkoxy ou dans les portions alkoxy, des restes cycloalcoyle comportant 5 à 6 atomes de carbone, des restes phénylalcoyle comportant 1 ou 2 atome(s) de carbone dans la portion alcoyle, des restes alcényle comportant 3 ou 4 atomes de carbone, un reste alcynyle comportant 3 ou 4 atomes de carbone, des restes phénylalcényle comportant 3 ou 4 atomes de carbone dans la portion alcényle, des restes dialcoylaminoalcoyle comportant 1 à 4 atome(s) de carbone dans chaque portion alcoyle, ou des restes furfuryles, et

$R_3$ peut représenter, en plus, à volonté, de l'hydrogène, .

$R_3$ et $R_4$ peuvent représenter ensemble un reste alkylène comportant 2 à 4 atomes de carbone, éventuellement substitué par un groupe hydroxy, un reste méthoxy, un atome d'halogène et/ou un reste acétoxy, un reste alcényle comportant 2 à 4 atomes de carbone, liant X et Y avec formation d'un noyau (anneau) à 5 à 7 maillons, ou bien, ensemble, un reste glucofuranosylène liant X et Y.

18. Produit selon la revendication 1, caractérisé en ce que

X et Y représentent l'oxygène,

$R_1$ représente un reste alcoyle, mono- ou polyhalo-alcoyle, ou un reste monocyanalcoyle, linéaire ou ramifié, comportant dans chaque cas de 1 à 4 atome(s) de carbone, ou bien un reste alcényle comportant de 2 à 4 atomes de carbone,

$R_2$ représente un reste alcoyle comportant de 1 à 4 atome(s) de carbone ou un reste phényle, et

$R_3$ et $R_4$ ont la signification indiquée dans la revendication 17.

19. Produit selon la revendication 1, caractérisé en ce qu

X représente l'oxygène,

Y représente un reste répondant à la formule générale

$R_6$
|
–N–     III ,

dans laquelle

$R_6$ représente un reste phényle ou allyle,

$R_1$ représente un reste dichlorométhyle,

$R_2$ représente l'hydrogène, et

$R_3$ et $R_4$ représentent ensemble un reste éthylénique liant X et Y avec formation d'un noyau oxazolidine.

20. Produit selon la revendication 1, caractérisé en ce que

X représente l'oxygène,

Y représente le soufre,

$R_1$ représente un reste alcoyle, mono- ou polyhalo-alcoyle ou monocyanalcoyle à chaîne linéaire ou ramifiée comportant, dans chaque cas, de 1 à 4 atome(s) de carbone, ou bien un reste alcényle comportant de 2 à 4 atomes de carbone,

$R_2$ représente l'hydrogène, et

$R_3$ et $R_4$ ont la signification indiquée dans la revendication 17.

21. Produit selon la revendication 1, caractérisé en ce que

X représente l'oxygène ou un reste répondant à la formule générale

$R_5$
|
–N–     II ,

dans laquelle

$R_5$ représente un reste phényle, un reste alcoyle comportant de 1 à 4 atome(s) de carbone ou un reste alcényle comportant 3 ou 4 atomes de carbone,

Y représente un reste répondant à la formule générale

$R_6$
|
–N–     III ,

dans laquelle

$R_6$ représente l'hydrogène, un reste phényle, un reste alcoyle comportant de 1 à 4 atome(s) de carbone ou un reste alcényle comportant 3 ou 4 atomes de carbone,

$R_1$ a les significations indiquées dans la revendication 17,

$R_2$ représente l'hydrogène, et

$R_3$ et $R_4$ représentent ensemble un reste alkylène comportant 2 à 4 atomes de carbone ou un reste alcénylène comportant 2 à 4 atomes de carbone, liant X et Y avec formation d'un noyau à 5 à 7 maillons.

22. Produit selon la revendication 1, caractérisé en ce que

X représente un reste répondant à la formule –N=,

Y représente un reste répondant à la formule

H
|
–N–,

$R_1$ représente une seconde liaison liant l'atome de carbone auquel il est fixé à X ou Y,

$R_2$ représente un reste dichlorométhyle, et

$R_3$ et $R_4$ représentent ensemble un reste phén-1,2-ylène.

23. Produit selon les revendications 1 à 22, caractérisé en ce qu'il renferme l'antidote ou les antidotes répondant à la formule générale I dans une proportion de 0,01 à 15% en poids, rapportée au poids de la ou des substance(s) active(s) herbicides, et la proportion totale de l'antidote ou des antidotes et de la ou des substance(s) active(s) herbicide(s), rapportée à la quantité totale du produit, est de 0,1 à 95% en poids, tandis que le reste consiste en un ou plusieurs support(s) neutre(s) solide(s) et/ou liquide(s) et/ou une ou plusieurs substance(s) tensio-active(s) anionique(s), cathionique(s) et/ou non-ionique(s).

24. Procédé de préparation de 2-(dichlorométhyl)-3-phényl-1,3-oxazolidine respectivement 2-(dichlorométhyl)-3-allyl-1,3-oxazolidine, caractérisé en ce que, de façon connue en soi, on mélange du dichloracétaldéhyde avec de la N-(phényl)-éthanolamine ou de la N-(allyl)-éthanolamine dans des hydrocarbures aromatiques, en particulier du benzène, dans des proportions à peu près stoéchiométriques, et l'on chauffe au reflux à l'ébullition plusieurs heures.